(19) Europäisches Patentamt
European Patent Office
Office européen des brevets

(11) EP 2 272 837 B1

(12) **EUROPEAN PATENT SPECIFICATION**

(45) Date of publication and mention of the grant of the patent:
**17.12.2014 Bulletin 2014/51**

(21) Application number: **09742659.7**

(22) Date of filing: **15.04.2009**

(51) Int Cl.:
***C09K 19/04*** (2006.01) ***C07D 309/06*** (2006.01)
***C07D 309/10*** (2006.01) ***C09K 19/34*** (2006.01)

(86) International application number:
**PCT/JP2009/057585**

(87) International publication number:
**WO 2009/136534 (12.11.2009 Gazette 2009/46)**

(54) **LIQUID CRYSTALLINE COMPOUND WITH NEGATIVE DIELECTRIC ANISOTROPY, LIQUID CRYSTAL COMPOSITION, AND LIQUID CRYSTAL DISPLAY DEVICE**

FLÜSSIGKRISTALLVERBINDUNG MIT NEGATIVER DIELEKTRISCHER ANISOTROPIE, FLÜSSIGKRISTALLZUSAMMENSETZUNG UND FLÜSSIGKRISTALLANZEIGEVORRICHTUNG

COMPOSÉ CRISTAL LIQUIDE AVEC UNE ANISOTROPIE DIÉLECTRIQUE NÉGATIVE, COMPOSITION DE CRISTAUX LIQUIDES ET DISPOSITIF D'AFFICHAGE À CRISTAUX LIQUIDES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **09.05.2008 JP 2008123171**

(43) Date of publication of application:
**12.01.2011 Bulletin 2011/02**

(73) Proprietors:
- **JNC Corporation**
**Chiyoda-ku**
**Tokyo (JP)**
- **JNC Petrochemical Corporation**
**Tokyo (JP)**

(72) Inventor: **MASUKAWA, Tokifumi**
**Ichihara-shi**
**Chiba 290-8551 (JP)**

(74) Representative: **Thurston, Joanna et al**
**Withers & Rogers LLP**
**4 More London Riverside**
**London SE1 2AU (GB)**

(56) References cited:
**EP-A1- 0 967 261** **EP-A1- 2 128 225**
**EP-A1- 2 206 695** **WO-A1-98/27036**
**WO-A1-2009/031437** **JP-A- 2000 008 040**
**JP-A- 2006 037 053** **JP-A- 2006 037 053**
**JP-A- 2008 088 165**

- **REIFFENRATH V ET AL: "New liquid-crystalline compounds with negative dielectric anisotropy", LIQUID CRYSTALS: AN INTERNATIONAL JOURNAL OF SCIENCE AND TECHNOLOGY, TAYLOR & FRANCIS, GB, vol. 5, no. 1, 1 January 1989 (1989-01-01) , pages 159-170, XP008143632, ISSN: 0267-8292**
- **REIFFENRATH, V. ET AL.: 'New liquid-crystalline compounds with negative dielectric anisotropy' LIQUID CRYSTALS vol. 5, no. 1, 1989, pages 159 - 170, XP008143632**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 272 837 B1

## Description

### Field of the Invention

The present invention relates to a novel liquid crystal compound and a liquid crystal composition. More specifically, it relates to a liquid crystal compound having negative dielectric anisotropy (Δε), a liquid crystal composition containing the compound, and a liquid crystal display device containing the liquid crystal composition.

### Related Art

A liquid crystal display device is classified into modes such as DS (dynamic scattering), TN (twisted nematic), GH (guest host), STN (super twisted nematic), IPS (in-plane switching), VA (vertical alignment) and OCB (optically compensated bend) according to the display mode. Desirable characteristics of a liquid crystal composition that is contained in the liquid crystal display devices commonly include the following, (1) to (6), in any display mode, although they may vary according to the display modes.

(1) The composition is stable to external environmental factors, such as water, air, heat and light.
(2) The composition exhibits a liquid crystal phase in a wide temperature range centering around room temperature.
(3) The composition has a small viscosity.
(4) The composition has a low driving voltage when the display device is driven.
(5) The composition has an optimum dielectric anisotropy (Δε).
(6) The composition has optimum refractive index anisotropy (Δn).

However, a liquid crystal compound that satisfies all of the characteristics (1) to (6) has not yet been found. Thus, a liquid crystal composition is generally used in which several kinds or a couple of dozen kinds of liquid crystal compounds are mixed. The liquid crystal compounds used as components of the composition necessarily have an excellent compatibility with each other for this purpose. A liquid crystal display device capable of being used in various environments, such as a very low temperature, has been demanded in recent years, and liquid crystal compounds exhibiting an excellent compatibility at a very low temperature are thus also demanded.

In recent years, modes such as IPS, VA and OCB among the display modes have been receiving attention as a display mode capable of overcoming a narrow viewing angle of a liquid crystal display device, which is the greatest problem of a liquid crystal display device. In particular, a liquid crystal display device having the VA mode and the IPS mode among these modes has been studied earnestly, since it has an excellent responsivity in addition to a wide viewing angle, and is capable of providing high-contrast display. The liquid crystal composition used in the liquid crystal display device having these modes is characterized by having negative dielectric anisotropy (Δε). It has been known that a liquid crystal composition having a negatively large dielectric anisotropy (Δε) can decrease the driving voltage of a liquid crystal display device containing the liquid crystal composition (see the none-patent document No. 1). Accordingly, liquid crystal compounds as the constitutional components of the liquid crystal composition are also demanded to have a negatively larger dielectric anisotropy (Δε).

Various liquid crystal compounds where hydrogen at a lateral position on a benzene ring is replaced by fluorine have been investigated as a component of a liquid crystal composition having negative dielectric anisotropy (Δε), (see the patent documents No. 1 and No. 2) . The following compound (a) had been reported, for example. However, although the compound (a) had negative dielectric anisotropy (Δε), the value was not necessarily large in some cases , and thus the compound (a) was not sufficient in some cases for decreasing the driving voltage of a liquid crystal display device having the VA mode, the IPS mode or the like.

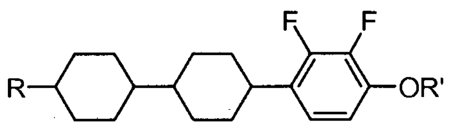

(a)

In formula (a), R and R' are alkyl.

In view of the circumstances, there have been attempts to increase the absolute value of the negative dielectric anisotropy (Δε) of the compound having a 2,3-difluorophenylene moiety. For example, such a compound has been reported that is obtained by introducing a tetrahydropyran-2,5-diyl moiety to the compound having a 2,3-difluorophenylene moiety (see the patent document No. 3). The compound (b) has a negatively larger dielectric anisotropy (Δε) than the compound (a).

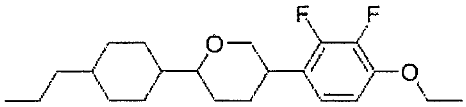
(b)

However, a liquid crystal compound that has a negatively much larger dielectric anisotropy (Δε), a liquid crystal composition containing the compound, and a liquid crystal display device containing the composition have been demanded for decreasing the driving voltage of a liquid crystal display device having the VA mode, the IPS mode or the like.

**PRIOR ART Patent**

**Document**

Patent document No. 1: Japanese Patent No. 2,811,342; the patent document No. 2: JP H02-004725 A (1990); the patent document No. 3, JP 2000-008040 A (2000) ; the patent document No. 4: EP 0967261 A1; the patent document No. 5: JP 2006-037053 A; the patent document No. 6: EP 2206695 A1; and the patent document No. 7: EP 2128225 A1. Non-patent document No. 1: Mol. Cryst. Liq. Cryst., vol. 12, p. 57 (1970) ; and non-patent document No. 2: Liquid Crystals: An International Journal of Science and Technology, vol. 5, p.159-170 (1989). Patent documents 4-7 and non-patent document No. 2 describe liquid crystal compounds with negative dielectric anisotropy and terminal 1,4-phenylene rings substituted with both fluorine atoms and alkoxy groups.

SUMMARY OF THE INVENTION

Subject to be solved

The first aim of the invention is to provide a liquid crystal compound that not only has a negatively large dielectric anisotropy (Δε), but also has at least one of characteristics such as the stability to heat, light and so forth, a high clearing point, a suitable refractive index anisotropy (Δn), a negatively large dielectric anisotropy (Δε) and an excellent compatibility with other liquid crystal compounds.

The second aim of the invention is to provide a liquid crystal composition that contains the compound and has at least one of characteristics such as a low viscosity, a suitable refractive index anisotropy (Δn), a suitable and negative dielectric anisotropy (Δε), a low threshold voltage, a high maximum temperature of a nematic phase (a high transition temperature of the phase from a nematic phase to an isotropic phase) and a low minimum temperature of a nematic phase, or has a suitable balance among at least two of the characteristics.

The third aim of the invention is to provide a liquid crystal display device that contains the composition and has at least one of characteristics such as a short response time, a small electric power consumption, a small driving voltage, a large contrast and a wide temperature range in which the device can be used, or has a suitable balance among at

**Means to solve the subject**

As a result of research on the subject described above, it was found that synergy among the following three factors caused an excellent effect in which the value of the dielectric anisotropy (Δε) was increased negatively and that the subject can be solved by a suitable use of the effect. Thus, the invention has been completed.

(1) a tetrahydropyran ring,
(2) $-CH_2O-$ or $-OCH_2-$ and
(3)

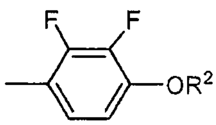

The invention includes features of the following items [1] to [17] :

[1] A compound represented by formula (1-1) :

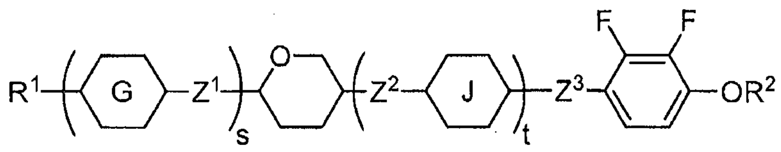

(1-1)

wherein

$R^1$ and $R^2$ are each independently alkyl having 1 to 10 carbons; and in the alkyl arbitrary $-CH_2-$ may be replaced by -O-, -S-, -CO- or $-SiH_2-$, where a plurality of $-CH_2-$ adjacent to each other are not replaced, and arbitrary $-(CH_2)_2-$ may be replaced by -CH=CH- or -C≡C-;

the ring G and the ring J are each independently 1,4-cyclohexylene or 1,4-phenylene; and in the 1,4-cyclohexylene arbitrary $-CH_2-$ may be replaced by -O-, -S-, -CO- or $-SiH_2-$, and arbitrary - $(CH_2)_2-$ may be replaced by -CH=CH-, in the 1,4-phenylene arbitrary -CH= may be replaced by -N=; and in these rings arbitrary hydrogen may be replaced by halogen, $-CF_3$, $-CHF_2$, $-CH_2F$, $-OCF_3$, $-OCHF_2$ or $-OCH_2F$;

$Z^1$ and $Z^2$ are each a single bond and $Z^3$ is $-CH_2O-$; and

s and t are each independently 0, 1, 2 or 3, where the sum of s and t is 1, 2 or 3.

[2] The compound according to item [1], wherein in formula (1-1), $R^1$ is alkyl having 1 to 10 carbons or alkenyl having 2 to 10 carbons; $R^2$ is alkyl having 1 to 10 carbons; and the ring G and the ring J are each independently 1,4-cyclohexylene or 1,4-phenylene.

[3] The compound according to any one of items [1] to [3], wherein the sum of s and t is 1 or 2.

[4] A compound according to claim 1 represented by formula (1-1-1):

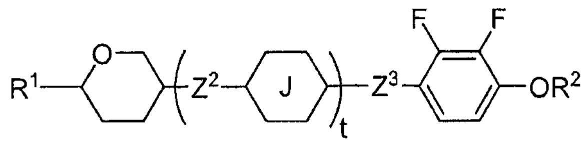

(1-1-1)

wherein $R^1$ is alkyl having 1 to 10 carbons or alkenyl having 2 to 10 carbons; $R^2$ is alkyl having 1 to 10 carbons; the ring J is independently 1,4-cyclohexylene or 1,4-phenylene; $Z^2$ is a single bond and $Z^3$ is $-CH_2O-$; and t is 1, 2 or 3.

[5] A compound according to claim 1 represented by formula (1-1-1-1):

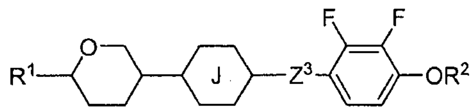

(1-1-1-1)

wherein $R^1$ and $R^2$ are each independently alkyl having 1 to 8 carbons; the ring J is 1,4-cyclohexylene or 1,4-phenylene; and $Z^3$ is $-CH_2O-$.

[6] A liquid crystal composition comprising at least one of compounds according to any one of items [1] to [5], as a component A.

[7] The liquid crystal composition according to item [6], further comprising at least one compound selected from the group of compounds represented by formulae (2), (3) and (4), as a component B:

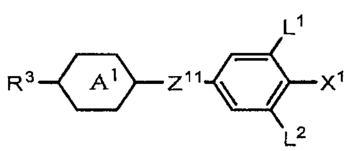

(2)

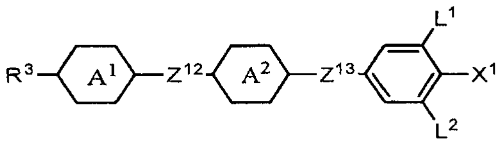

(3)

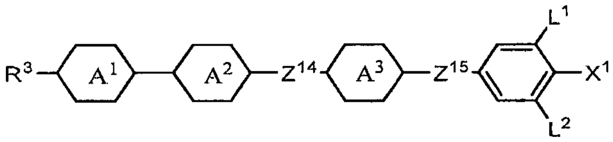

(4)

wherein

$R^3$ is alkyl having 1 to 10 carbons or alkenyl having 2 to 10 carbons, and in the alkyl and the alkenyl, arbitrary hydrogen may be replaced by fluorine and arbitrary $-CH_2-$ may be replaced by -O-;

$X^1$ is fluorine, chlorine, $-OCF_3$, $-OCHF_2$, $-CF_3$, $-CHF_2$, $-CH_2F$, $-OCF_2CHF_2$ or $-OCF_2CHFCF_3$;

the ring $A^1$, the ring $A^2$ and the ring $A^3$ are each independently 1,4-cyclohexylene, 1,3-dioxane-2,5-diyl, pyrimidine-2,5-diyl, 1-tetrahydropyran-2,5-diyl or 1,4-phenylene in which arbitrary hydrogen may be replaced by fluorine;

$Z^{11}$, $Z^{12}$, $Z^{13}$, $Z^{14}$ and $Z^{15}$ are each independently $-(CH_2)_2-$, $-(CH_2)_4-$, -COO-, $-CF_2O-$, $-OCF_2-$, -CH=CH-, -C≡C-, $-CH_2O-$ or a single bond; and

$L^1$ and $L^2$ are each independently hydrogen or fluorine.

[8] The liquid crystal composition according to item [6], further comprising at least one compound selected from the group of compounds represented by formula (5), as a component C:

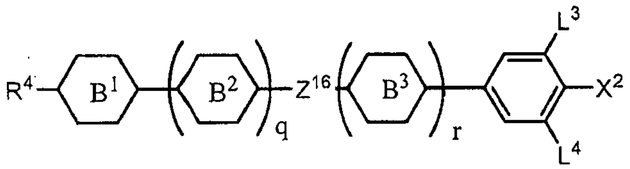

(5)

wherein

$R^4$ is alkyl having 1 to 10 carbons or alkenyl having 2 to 10 carbons, and in the alkyl and the alkenyl, arbitrary hydrogen may be replaced by fluorine and arbitrary $-CH_2-$ may be replaced by -O-;

$X^2$ is -C≡N or -C≡C-C≡N;

the ring $B^1$, the ring $B^2$ and the ring $B^3$ are each independently 1,4-cyclohexylene, 1, 4-phenylene in which arbitrary hydrogen may be replaced by fluorine, 1,3-dioxane-2,5-diyl, 1-tetrahydropyran-2,5-diyl or pyrimidine-2,5-diyl;

$Z^{16}$ is $-(CH_2)_2-$, -COO-, $-CF_2O-$, $-OCF_2-$, -C≡C-, $-CH_2O-$ or a single bond;

$L^3$ and $L^4$ are each independently hydrogen or fluorine; and

q is 0, 1 or 2 and r is 0 or 1.

[9] The liquid crystal composition according to item [6], further comprising at least one compound selected from the group of compounds represented by formulae (6), (7), (8), (9) and (10), as a component D:

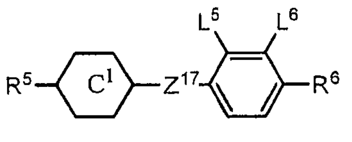

(6)

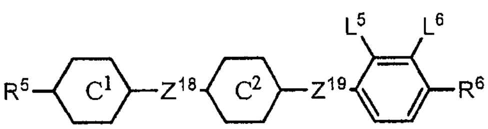

(7)

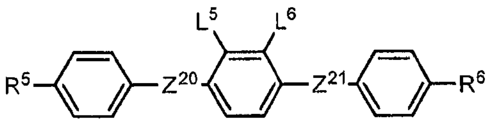

(8)

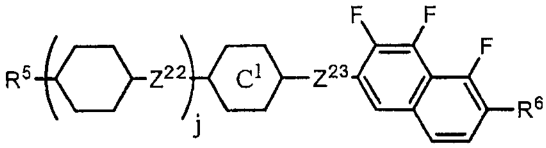

(9)

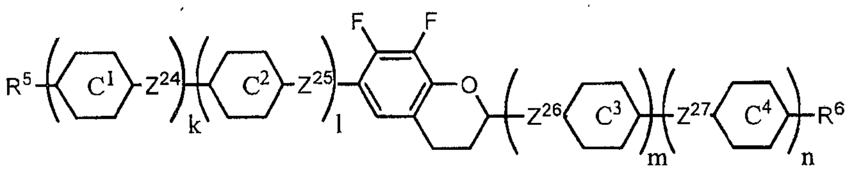

(10)

wherein

$R^5$ and $R^6$ are each independently alkyl having 1 to 10 carbons or alkenyl having 2 to 10 carbons, and in the alkyl and the alkenyl, arbitrary hydrogen may be replaced by fluorine and arbitrary $-CH_2-$ may be replaced by -O-;
the ring $C^1$, the ring $C^2$, the ring $C^3$ and the ring $C^4$ are each independently 1,4-cyclohexylene, 1,4-cyclohexenylene, 1,4-phenylene in which arbitrary hydrogen may be replaced by fluorine or decahydro-2,6-naphthalene;
$Z^{17}$, $Z^{18}$, $Z^{19}$, $Z^{20}$, $Z^{21}$, $Z^{22}$, $Z^{23}$, $Z^{24}$, $Z^{25}$, $Z^{26}$, and $Z^{27}$ are each independently $-(CH_2)_2-$, -COO-, $-CH_2O-$, $-OCF_2-$, $-OCF_2(CH_2)_2-$ or a single bond;
$L^5$ and $L^6$ are each independently chlorine or fluorine; and
j, k, l, m and n are each independently 0 or 1, and the sum of k, 1, m and n is 1 or 2.

[10] The liquid crystal composition according to item [6], further comprising at least one compound selected from the group of compounds represented by formulae (11), (12) and (13), as a component E:

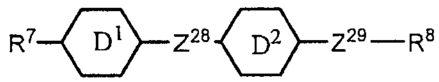

(11)

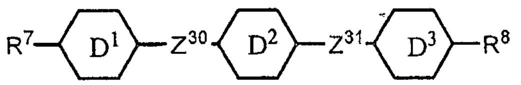

(12)

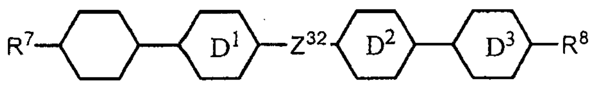

(13)

wherein

$R^7$ and $R^8$ are each independently alkyl having 1 to 10 carbons or alkenyl having 2 to 10 carbons, and in the alkyl and the alkenyl, arbitrary hydrogen may be replaced by fluorine and arbitrary $-CH_2-$ may be replaced by -O-;
the ring $D^1$, the ring $D^2$ and the ring $D^3$ are each independently 1,4-cyclohexylene, pyrimidine-2,5-diyl, 1,4-phenylene, 2-fluoro-1,4-phenylene, 3-fluoro-1,4-phenylene or 2,5-difluoro-1,4-phenylene; and
$Z^{28}$, $Z^{29}$, $Z^{30}$, $Z^{31}$ and $Z^{32}$ are each independently -C≡C-, -COO-, - $(CH_2)_2-$, -CH=CH- or a single bond.

[11] The liquid crystal composition according to item [7], further comprising at least one compound selected from the group of compounds represented by formula (5) according to item [8].

[12] The liquid crystal composition according to item [7], further comprising at least one compound selected from the group of compounds represented by formulae (11), (12) and (13) according to item [10].

[13] The liquid crystal composition according to item [8], further comprising at least one compound selected from the group of compounds represented by formulae (11), (12) and (13) according to item [10].

[14] The liquid crystal composition according to item [9], further comprising at least one compound selected from the group of compounds represented by formulae (11), (12) and (13) according to item [10].

[15] The liquid crystal composition according to any one of items [6] to [14], further comprising at least one optically active compound.

[16] The liquid crystal composition according to any one of items [6] to [15], further comprising at least one antioxidant, or at least one ultraviolet absorbent, or at least each of an antioxidant and an ultraviolet absorbent.

[17] A liquid crystal display device comprising at least one of the liquid crystal compositions according to any one of items [6] to [16] .

## Effect of the Invention

A liquid crystal compound that not only has a negatively large dielectric anisotropy ($\Delta\varepsilon$), but also has at least one of characteristics such as the stability to heat, light and so forth, a high clearing point, a suitable refractive index anisotropy ($\Delta n$) and an excellent compatibility with other liquid crystal compounds is obtained according to the invention. A liquid crystal composition that has at least one of characteristics such as a low viscosity, a suitable refractive index anisotropy ($\Delta n$), a suitable and negative dielectric anisotropy ($\Delta\varepsilon$), a low threshold voltage, a high maximum temperature of a nematic phase and a low minimum temperature of a nematic phase is obtained according to the invention. A liquid crystal display device that has at least one of characteristics such as a short response time, a low electric power consumption, a low driving voltage, a large contrast and a wide temperature range in which the device can be used is obtained according to the invention.

## Aspect to carry out the Invention

The invention will be explained in detail below.

The liquid crystal compound of the invention is represented by formula (1-1). Also illustrated is compound (1-2) (not according to the invention):

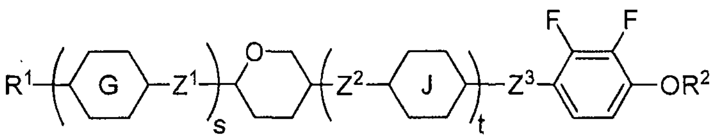

(1-1)

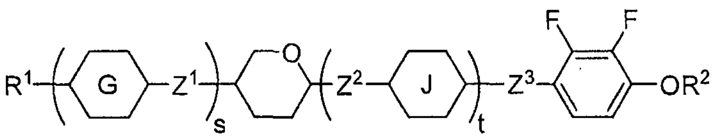

(1-2)

In formulae (1-1) and (1-2) , $R^1$ and $R^2$ are each independently alkyl having 1 to 10 carbons. In the alkyl, arbitrary $-CH_2-$ may be replaced by -O-, -S-, -CO- or $-SiH_2-$, where a plurality of $-CH_2-$ adjacent to each other are not replaced, and arbitrary $-(CH_2)_2-$ may be replaced by -CH=CH- or -C≡C-. In consideration of the stability of the compound, it is not desirable that two oxygens are adjacent to each other.

Examples of $R^1$ include alkyl having 1 to 10 carbons, alkoxy having 1 to 9 carbons, alkoxyalkyl having 2 to 9 carbons, alkoxyalkoxy having 2 to 8 carbons, alkenyl having 2 to 10 carbons, alkenyloxy having 2 to 9 carbons, alkenyloxyalkyl having 3 to 9 carbons and alkoxyalkenyl having 3 to 9 carbons. The alkyl chain in these groups is preferably straight. The temperature range of a liquid crystal phase is increased and the viscosity is decreased when the alkyl chain is straight. The alkenyl preferably has the double bond at an odd number position, and preferably has a trans configuration. When the alkenyl has a plurality of the double bonds, they are preferably not conjugated.

Examples of the alkyl include $-CH_3$, $-C_2H_5$, $-C_3H_7$, $-C_4H_9$, $-C_5H_{11}$, $-C_6H_{13}$, $-C_7H_{15}$, $-C_8H_{17}$, $-C_9H_{19}$ and $-C_{10}H_{21}$.

Examples of the alkoxy include $-OCH_3$, $-OC_2H_5$, $-OC_3H_7$, $-OC_4H_9$, $-OC_5H_{11}$, $-OC_6H_{13}$, $-OC_7H_{15}$, $-OC_8H_{17}$ and $-OC_9H_{19}$.

Examples of the alkoxyalkyl include $-CH_2OCH_3$, $-CH_2OC_2H_5$, $-(CH_2)_2OCH_3$ and $-(CH_2)_2OC_2H_5$.

Examples of alkoxyalkoxy include $-OCH_2OCH_3$, $-OCH_2OC_2H_5$, $-O(CH_2)_2OCH_3$ and $-O(CH_2)_2OC_2H_5$.

Examples of the alkenyl include $-CH=CH_2$, $-CH=CHCH_3$, $-CH=CHC_2H_5$, $-(CH_2)_2CH=CH_2$, $-CH=CHC_3H_7$,

$-(CH_2)_2CH=CHCH_3$, $-(CH_2)_3CH=CH_2$, $-CH=CH(CH_2)_2CH=CH_2$ and $-(CH_2)_2CH=CH(CH_2)_2CH=CH_2$.

Examples of the alkenyloxy include $-OCH_2CH=CH_2$, $-OCH_2CH=CHCH_3$ and $-OCH_2CH=CHC_2H_5$.

Examples of alkenyloxyalkyl include $-CH_2OCH_2CH=CH_2$, $-CH_2OCH_2CH=CHCH_3$, and $-(CH_2)_2O(CH_2)_2CH=CH_3$.

Examples of the alkoxyalkenyl include $-CH=CHCH_2OCH_3$, $-CH=CHCH_2OC_2H_5$ and $-CH_2CH=CHCH_2OCH_3$.

Desirable $R^1$ among these are $-CH_3$, $-C_2H_5$, $-C_3H_7$, $-C_4H_9$, $-C_5H_{11}$, $-C_6H_{13}$, $-C_7H_{15}$, $-C_8H_{17}$, $-C_9H_{19}$, $-C_{10}H_{21}$, $-OCH_3$, $-OC_2H_5$, $-OC_3H_7$, $-OC_4H_9$, $-OC_5H_{10}$, $-OC_6H_{13}$, $-OC_7H_{15}$, $-OC_8H_{17}$, $-OC_9H_{19}$, $-CH=CH_2$, $-CH=CHCH_3$, $-CH=CHC_2H_5$, $-(CH_2)_2CH=CH_2$, $-CH=CHC_3H_7$, $-(CH_2)_2CH=CHCH_3$, $-(CH_2)_3CH=CH_2$, $-CH=CH(CH_2)_2CH=CH_2$ and $-(CH_2)_2CH=CH(CH_2)_2CH=CH_2$.

More desirable $R^1$ are $-CH_3$, $-C_2H_5$, $-C_3H_7$, $-C_4H_9$, $-C_5H_{11}$, $-C_6H_{13}$-$CH=CH_2$, $-CH=CHCH_3$, $-CH=CHC_2H_5$, $-(CH_2)_2CH=CH_2$, $-CH=CHC_3H_7$, $-(CH_2)_2CH=CHCH_3$, $-(CH_2)_3CH=CH_2$, $-CH=CH(CH_2)_2CH=CH_2$ and $-(CH_2)_2CH=CH(CH_2)_2CH=CH_2$.

Examples of $R^2$ include alkyl having 1 to 10 carbons, alkoxyalkyl having 2 to 9 carbons, alkenyl having 2 to 10 carbons, alkenyloxyalkyl having 3 to 9 carbons and alkoxyalkenyl having 3 to 9 carbons. The alkyl chain in these groups is preferably straight. The temperature range of a liquid crystal phase is increased and the viscosity is decreased, when the alkyl chain is straight. The alkenyl preferably has a trans configuration. $R^2$ is preferably not alkoxy in view of the stability of the compound since two oxygens are adjacent to each other.

Examples of the alkyl include $-CH_3$, $-C_2H_5$, $-C_3H_7$, $-C_4H_9$, $-C_5H_{11}$, $-C_6H_{13}$, $-C_7H_{15}$, $-C_8H_{17}$, $-C_9H_{19}$ and $-C_{10}H_{21}$.

Examples of the alkoxyalkyl include $-CH_2OCH_3$, $-CH_2OC_2H_5$, $-(CH_2)_2OCH_3$ and $-(CH_2)_2OC_2H_5$.

Examples of the alkenyl include $-CH=CH_2$, $-CH=CHCH_3$, $-CH_2CH=CH_2$, $-CH=CHC_2H_5$, $-CH_2CH=CHCH_3$, $-(CH_2)_2CH=CH_2$, $-CH=CHC_3H_7$, $CH_2CH=CHC_2H_5$, $-(CH_2)_2CH=CHCH_3$ and $-(CH_2)_3CH=CH_2$.

Examples of alkenyloxyalkyl include $-CH_2OCH_2CH=CH_2$, $-CH_2OCH_2CH=CHCH_3$ and $-(CH_2)_2O(CH_2)_2CH=CH_3$.

Examples of the alkoxyalkenyl include $-CH=CHCH_2OCH_3$, $-CH=CHCH_2OC_2H_5$ and $-CH_2CH=CHCH_2OCH_3$.

Desirable $R^2$ among these are $-CH_3$, $-C_2H_5$, $-C_3H_7$, $-C_4H_9$, $-C_5H_{11}$, $-C_6H_{13}$, $-C_7H_{15}$, $-C_8H_{17}$, $-C_9H_{19}$, $-C_{10}H_{21}$, $-CH_2CH=CH_2$, $-CH_2CH=CHCH_3$, $-(CH_2)_2CH=CH_2$, $-CH_2CH=CHC_2H_5$, $-(CH_2)_2CH=CHCH_3$ and $-(CH_2)_3CH=CH_2$.

More desirable $R^2$ are $-CH_3$, $-C_2H_5$, $-C_3H_7$, $-C_4H_9$, $-C_5H_{11}$ and $-C_6H_{13}$.

The ring G and the ring J in formulae (1-1) and (1-2) are each independently 1,4-cyclohexylene or 1,4-phenylene. In these rings, arbitrary $-CH_2-$ may be replaced by -O-, -S-, -CO- or $-SiH_2-$, and arbitrary $-(CH_2)_2-$ may be replaced by -CH=CH-. In the 1,4-phenylene arbitrary -CH= may be replaced by -N=, and in these rings, arbitrary hydrogen may be replaced by halogen, $-CF_3$, $-CHF_2$, $-CH_2F$, $-OCF_3$, $-OCHF_2$ or $-OCH_2F$.

The refractive index anisotropy ($\Delta n$) is decreased and the viscosity is decreased, when these rings are each 1,4-cyclohexylene, and the maximum temperature of a nematic phase of a liquid crystal composition is increased by the addition of this liquid crystal compound to the composition.

The refractive index anisotropy ($\Delta n$) is relatively increased and the orientational order parameter is increased when the ring is 1,4-phenylene in which hydrogen may be replaced by halogen.

Desirable ring G and ring J among these are 1,4-cyclohexylene, 1,4-cyclohexenylene, 1,3-dioxane-2,5-diyl, 1,4-phenylene, 2-fluoro-1,4-phenylene, 3-fluoro-1,4-phenylene, 2,3-difluoro-1,4-phenylene, 2-fluoro-3-chloro-1,4-phenylene, 2-chloro-3-fluoro-1,4-phenylene, 2,3-dichloro-1,4-phenylene and 1,3-pyrimidine-2,5-yl, and more desirable ring G and ring J are 1,4-cyclohexenylene and 1,4-phenylene.

In formula (1-1), $Z^1$ and $Z^2$ are each a single bond, $Z^3$ is $-CH_2O-$. In formula (1-2), $Z^1$, $Z^2$ and $Z^3$ are each independently a single bond, $-CH_2O-$ or $-OCH_2-$, provided that at least one of $Z^1$, $Z^2$ and $Z^3$ in formula (1-2), is $-CH_2O-$ or $-OCH_2-$. In formula (1-2), $Z^3$ is preferably $-CH_2O-$ for increasing the absolute value of the dielectric anisotropy ($\Delta\varepsilon$), and $Z^1$ and $Z^2$ are each preferably a single bond for increasing the temperature range of a liquid crystal phase and for decreasing the viscosity.

s and t are each independently 0, 1, 2 or 3, and the sum of s and t is 1, 2 or 3. The sum of s and t is preferably 1 or 2, since the compatibility with other liquid crystal compounds is decreased when the number of the rings is increased.

A plurality of the ring G may be the same or different and a plurality of the bonding group $Z^1$ may be the same or different, when s is 2 or 3. A plurality of the ring J may be the same or different and a plurality of bonding group $Z^2$ may be the same or different, when t is 2 or 3.

In the case where t is 0 in formula (1-2), the absolute value of the dielectric anisotropy ($\Delta\varepsilon$) is decreased when $Z^3$ is a single bond. This is because the oxygen in the tetrahydropyran ring is located

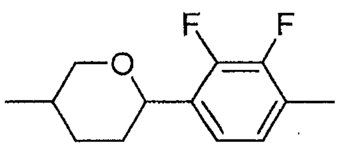

(A)

at the opposite side of the fluorine on 2,3-difluoro-1,4-phenylene for a stable conformation in the structure of Figure (A), and thus the dielectric anisotropy ($\Delta\varepsilon$) is decreased, because two dipole moments operated in reverse. For the above reason, $Z^3$ is preferably $-CH_2O-$ or $-OCH_2-$when t is 0.

In the liquid crystal compound represented by formula (1-1) or (1-2), the terminal groups $R^1$ and $R^2$, the rings G and J, and the bonding groups $Z^1$ $Z^2$ and $Z^3$ can be appropriately selected from the aforementioned definitions, adjusting characteristics such as the refractive index anisotropy ($\Delta n$) and the dielectric anisotropy ($\Delta\varepsilon$) to desired values.

Desirable examples of the liquid crystal compound represented by formula (1-1) or (1-2) include compounds represented by formula (1-1-1) and (1-2-1) :

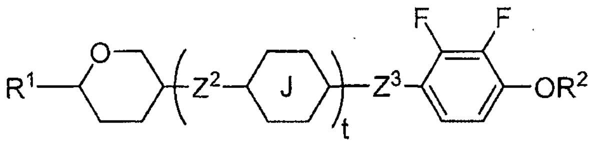

(1-1-1) (according to the invention)

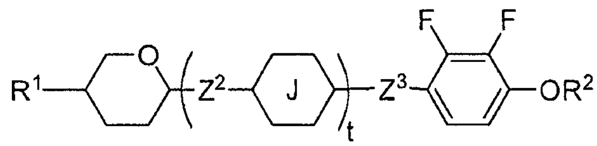

(1-2-1) (not according to the invention)

wherein $R^1$ is alkyl having 1 to 10 carbons or alkenyl having 2 to 10 carbons; $R^2$ is alkyl having 1 to 10 carbons; the ring J is independently 1,4-cyclohexylene or 1,4-phenylene; in formula (1-1-1) $Z^2$ is a single bond and $Z^3$ is $-CH_2O-$. In formula (1-2-1), $Z^2$ and $Z^3$ are each independently a single bond, $-CH_2O-$ or $-OCH_2-$, provided that at least one of $Z^2$ and $Z^3$ is $-CH_2O-$ or $-OCH_2-$. t is 1, 2 or 3.

More desirable examples of the liquid crystal compound represented by formula (1-1) or (1-2) include compounds represented by formula (1-1-1-1) and (1-2-1-1):

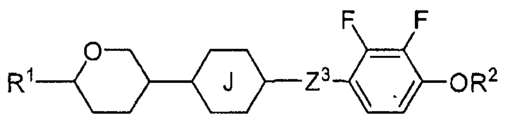

(1-1-1-1) (according to the invention)

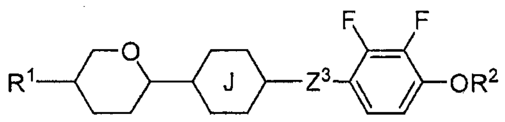

(1-2-1-1) (not according to the invention)

wherein $R^1$ and $R^2$ are each independently alkyl having 1 to 8 carbons; the ring J is 1,4-cyclohexylene or 1, 4-phenylene; and in formula (1-1-1-1) $Z^3$ is $-CH_2O-$, and in formula (1-2-1-1) $Z^3$ is $-CH_2O-$ or $-OCH_2-$.

The liquid crystal compound represented by formula (1-1) of the invention or formula (1-2) as illustrated can be obtained by introducing appropriate groups at the position of $R^1$, $R^2$, the ring G, the ring J, $Z^1$, $Z^2$ and $Z^3$, and the groups can be introduced according to known and general synthetic organic methods. Representative examples of the synthesis include the methods described in "Vol. 14: Synthesis and Reaction of Organic Compounds" (1978) in New Experimental Chemistry Course (Shin Jikken Kagaku Kouza, in Japanese title; Maruzen Co., Ltd.), or "Vol. 19 to Vol. 26: Organic Synthesis I to VIII" (1991) in Experimental Chemistry Course (Jikken Kagaku Kouza, in Japanese title; the fourth edition, Maruzen Co., Ltd.).

An example of a method for forming the bonding groups $Z^1$, $Z^2$ and $Z^3$ will be shown in the schemes, and the schemes will be then explained in the sections (I) and (II). In the schemes, $MSG^1$ and $MSG^2$ are each a monovalent organic group having at least one ring. A plurality of $MSG^1$ (or $MSG^2$) used in the scheme may be the same or different. The compounds (1A) and (1B) in the scheme correspond to the liquid crystal compounds represented by formula (1-1) or (1-2).

$$MSG^1\text{—}B(OH)_2 + Hal\text{—}MSG^2 \xrightarrow{Pd(PPh_3)_4,\ Na_2CO_3aq} MSG^1\text{—}MSG^2$$

(21) (22) Hal = Br, I (1A)

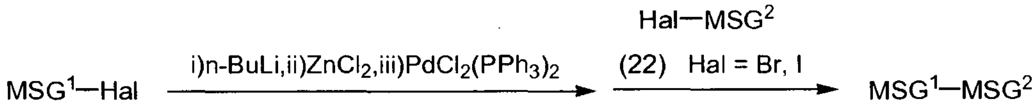


(23) Hal = Br, I

(1A)

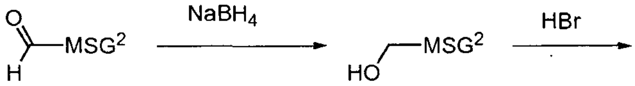


(24)

(25)

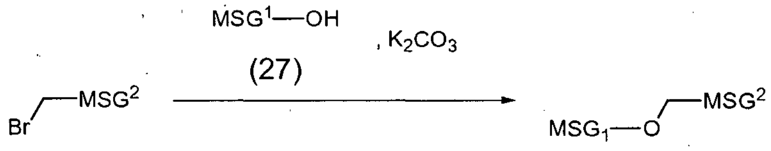


(26)

(1B)

(I) Formation of Single Bond

The compound (1A) is prepared by the reaction of the aryl borate (21) with the compound (22) that is prepared by a known method, in the presence of a catalyst such as an aqueous solution of carbonate and tetrakis (triphenylphosphine) palladium. The compound (1A) can also be prepared by the reaction of the compound (23) that is prepared by a known method with n-butyllithium and then with zinc chloride, and by the reaction of the product with the compound (22) in the presence of a catalyst, such as dichlorobis(triphenylphosphine)palladium.

(II) Formation of $-CH_2O-$ or $-OCH_2-$

The compound (24) is reduced with a reducing agent, such as sodium borohydride, to give the compound (25). The compound (25) is halogenated with hydrobromic acid to give the compound (26). The compound (26) is reacted with the compound (27) in the presence of potassium carbonate to give the compound (1B).

An example of methods for synthesizing the tetrahydropyran compound represented by formula (1-1) will be shown in the schemes. A scheme in which the synthetic intermediate (31) having a lactone moiety is prepared will be explained, and then an example of a method for preparing the tetrahydropyran compound (34) by using the synthetic intermediate (31) as a starting material will be described.

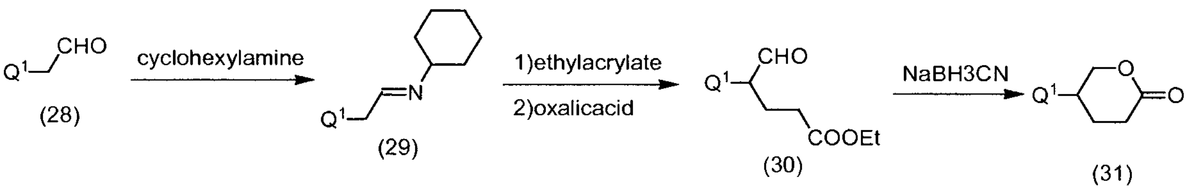


(28) (29) (30) (31)

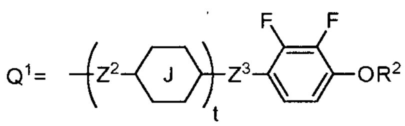

In the compounds (28) to (31), $Q^1$ is a structural unit of formula (1-1). The structural unit is shown in the scheme. In these compounds, the symbols $R^2$, J, $Z^2$, $Z^3$ and t have the same meanings as described in formula (1-1) or (1-2).

Specifically, the compound (29) is prepared by the reaction of the compound (28) with cyclohexylamine. The reaction is preferably carried out in a solvent such as diethyl ether, in the presence of a base such as potassium carbonate at a temperature in the range of room temperature to the boiling point of the solvent. The compound (30) is prepared by the addition reaction of ethyl acrylate to the compound (29) and then by the deprotection of the protective group. In the reaction, ethyl acrylate itself is preferably used as a solvent, and a solvent, such as toluene, that does not react with the compound (29) or ethyl acrylate may be used. A polymerization inhibitor, such as hydroquinone, is preferably added

to prevent the polymerization of ethyl acrylate. The reaction is carried out in the temperature range of room temperature to the boiling point of the solvent when an ordinary glass reactor is employed, and the reaction may be carried out at a temperature higher than the boiling point of the solvent by using a pressure-proof reactor such as a stainless steel autoclave. After the addition reaction has proceeded sufficiently, an acid such as oxalic acid is added to the reaction mixture, eliminating cyclohexylamine and giving the compound (30). The compound (31) is prepared by means of the ring-opening reaction of the compound (30). The reaction is carried out preferably in a solvent, such as isopropanol, in the presence of sodium cyanoborohydride at around room temperature. An acid such as hydrochloric acid may be added to accelerate the reaction.

The compound (28) that is a starting material can be easily prepared by a known method in synthetic organic chemistry.

An example of methods for synthesizing the compound (34) is shown in the following scheme.

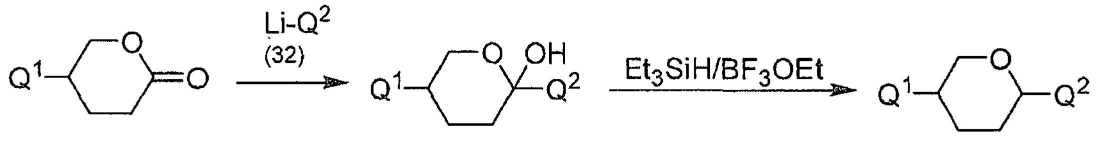


(31) (33) (34)

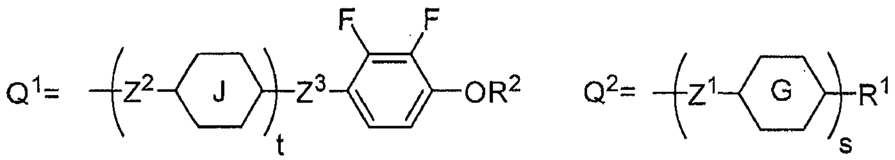

In the compounds (31) to (34), $Q^1$ and $Q^2$ are each a structural unit of formula (1-1) . The structural unit is shown in the scheme. In these compounds, the symbols $R^1$, $R^2$, G, J, $Z^1$, $Z^2$, $Z^3$, s and t have the same meanings as described in formula (1-1) and (1-2).

Specifically, the compound (33) is prepared by the reaction of the compound (31) with the compound (32). The reaction is preferably carried out in a solvent such as tetrahydrofuran at a temperature of -30 °C. The compound (34) is prepared by the reaction of the compound (33) in a solvent such as dichloromethane in the presence of triethylsilane and boron trifluoride-diethyl ether complex at a temperature of -50 °C or lower.

The compound (32) can be easily prepared by a method in synthetic organic chemistry.

The tetrahydropyran compound represented by formula (1-2) can be prepared by a similar method.

The liquid crystal composition of the invention necessarily contains the compound represented by formula (1-1) of the invention as a component A. Also illustrated are compositions containing (1-2). The composition may contain only the component A or may contain the component A and another component that is not shown in this specification. The composition can exhibit various characteristics by the addition of the component B that is at least one compound selected from the group of the compounds represented by formulae (2), (3) and (4), the component C that is at least one compound selected from the group of the compounds represented by formula (5), the component D that is at least one compound selected from the group of the compounds represented by formulae (6), (7), (8), (9) and (10), and/or the component E that is at least one compound selected from the group of the compounds represented by formulae (11), (12) and (13).

These components may be arbitrarily combined according to objective characteristics or use, and examples of desirable combinations of the components include the components A and B, the components A and C, the components A and D, the components A, B and E, the components A, C and E, and the components A, D and E.

Even if an analogue that is composed of an isotope of each element is used as a component, there is no large difference in physical characteristics between the composition of the invention and that of the analogue. The components of the liquid crystal composition of the invention may be analogues thereof containing isotopes of the elements constituting the components since there is no large physical difference among them.

In the component B, desirable examples of the compound represented by formula (2) include compounds represented by formulae (2-1) to (2-16), desirable examples of the compound represented by formula (3) include compounds represented by formulae (3-1) to (3-112), and desirable examples of the compound represented by formula (4) include compounds represented by formulae (4-1) to (4-52).

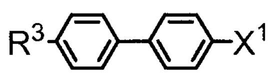
(2-1)

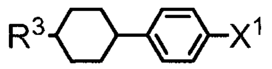
(2-2)

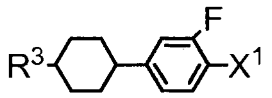
(2-3)

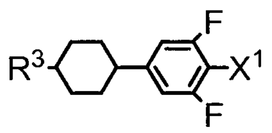
(2-4)

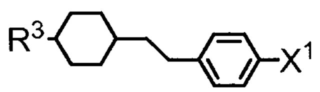
(2-5)

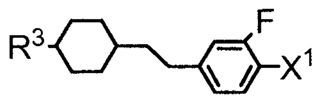
(2-6)

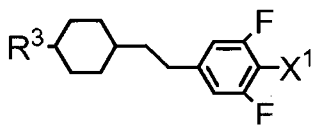
(2-7)

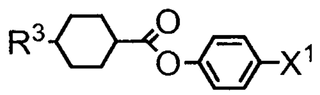
(2-8)

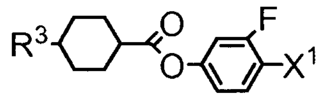
(2-9)

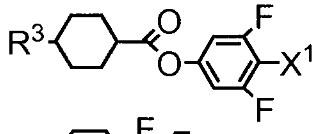
(2-10)

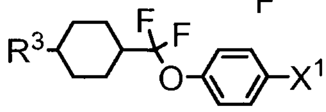
(2-11)

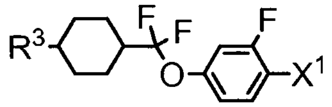
(2-12)

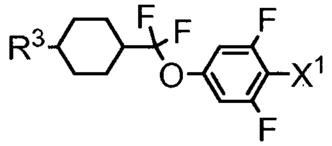
(2-13)

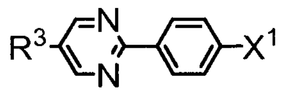
(2-14)

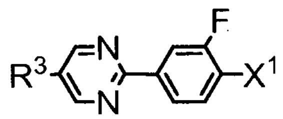
(2-15)

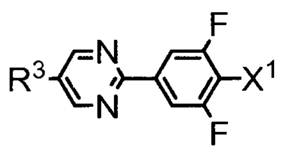
(2-16)

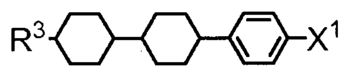
(3-1)

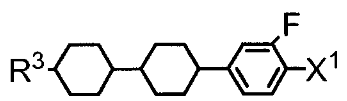
(3-2)

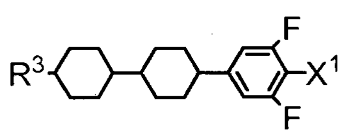
(3-3)

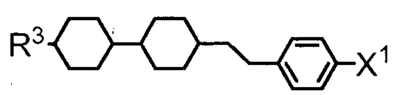
(3-4)

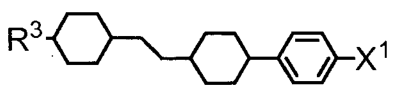
(3-13)

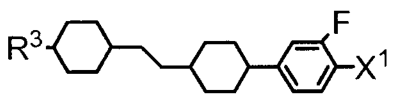
(3-14)

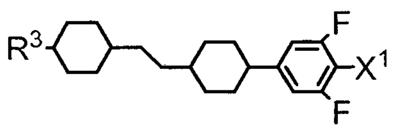
(3-15)

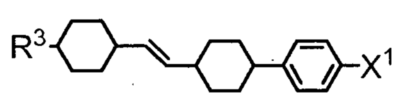
(3-16)

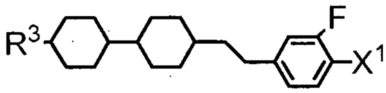 (3-5)

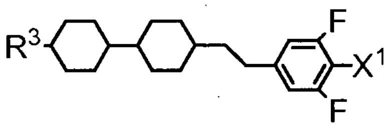 (3-6)

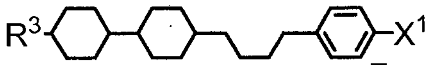 (3-7)

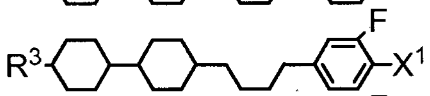 (3-8)

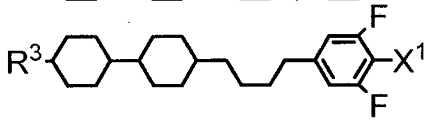 (3-9)

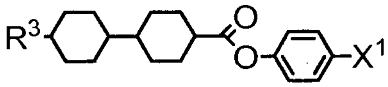 (3-10)

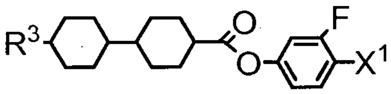 (3-11)

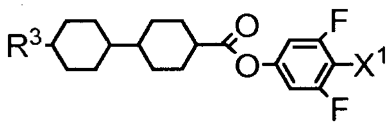 (3-12)

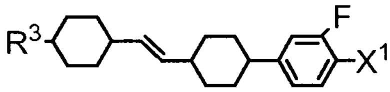 (3-17)

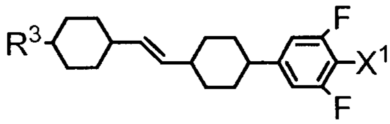 (3-18)

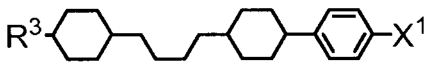 (3-19)

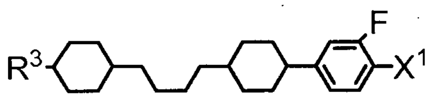 (3-20)

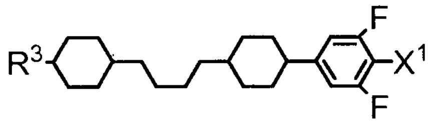 (3-21)

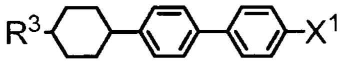 (3-22)

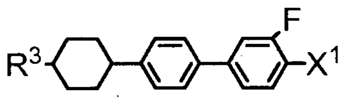 (3-23)

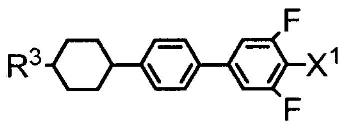 (3-24)

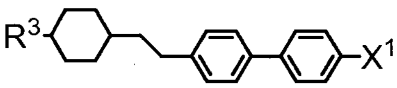 (3-25)

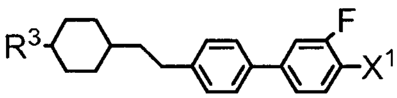 (3-26)

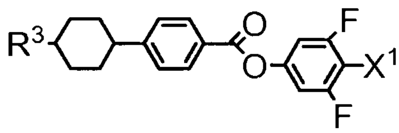 (3-39)

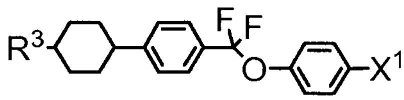 (3-40)

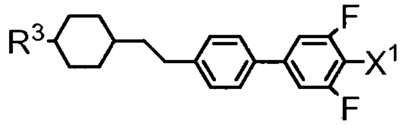 (3-27)

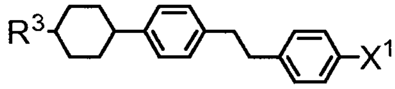 (3-28)

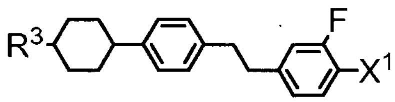 (3-29)

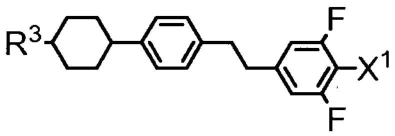 (3-30)

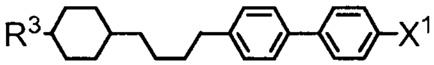 (3-31)

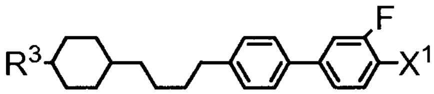 (3-32)

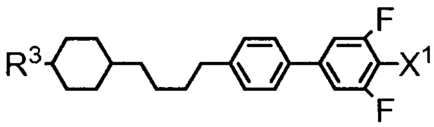 (3-33)

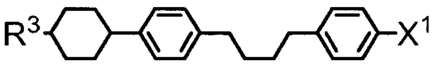 (3-34)

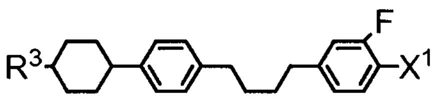 (3-35)

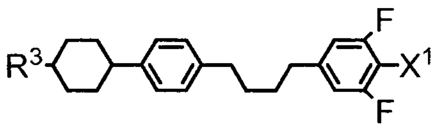 (3-36)

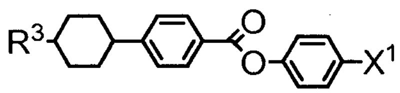 (3-37)

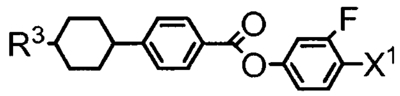 (3-38)

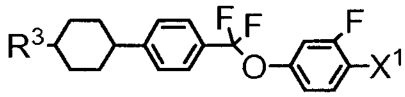 (3-41)

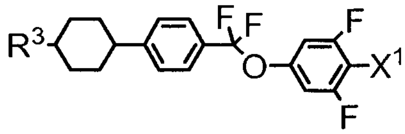 (3-42)

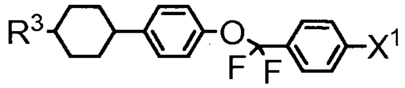 (3-43)

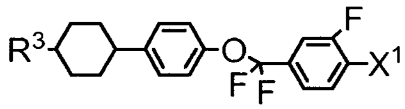 (3-44)

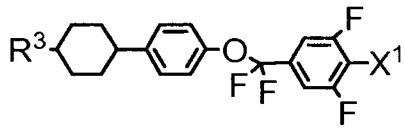 (3-45)

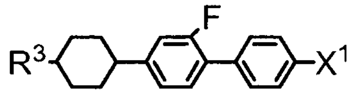 (3-46)

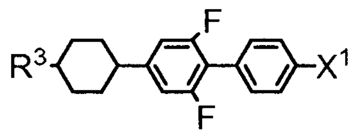 (3-47)

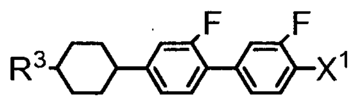 (3-48)

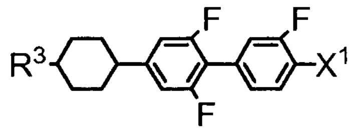 (3-49)

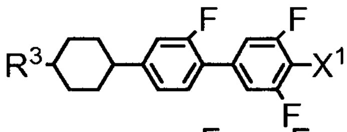 (3-50)

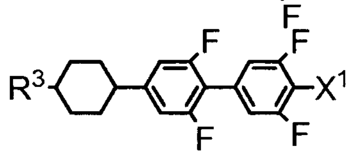 (3-51)

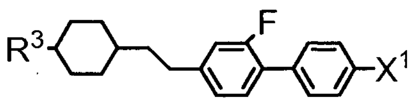 (3-52)

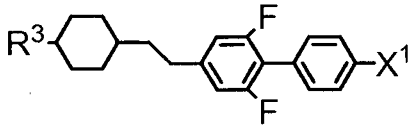 (3-53)

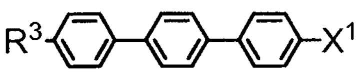 (3-64)

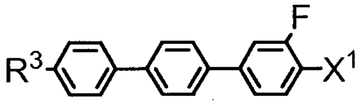 (3-65)

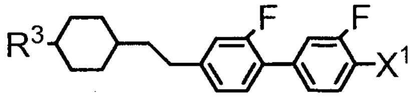
(3-54)

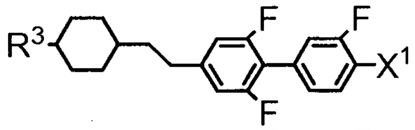
(3-55)

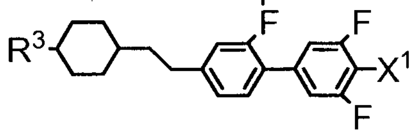
(3-56)

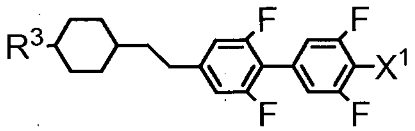
(3-57)

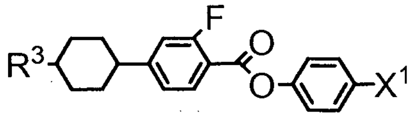
(3-58)

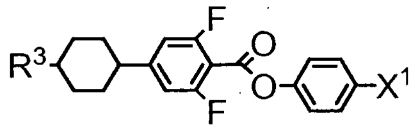
(3-59)

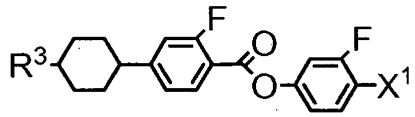
(3-60)

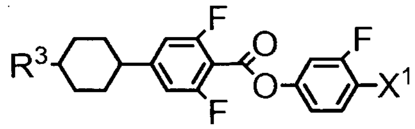
(3-61)

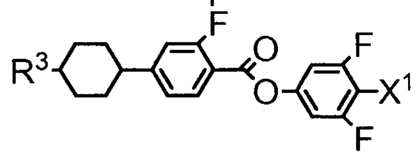
(3-62)

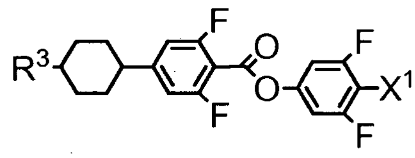
(3-63)

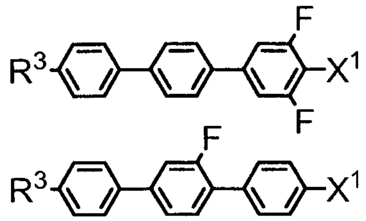
(3-66)

(3-67)

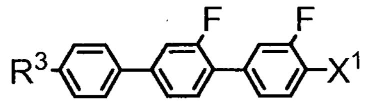
(3-68)

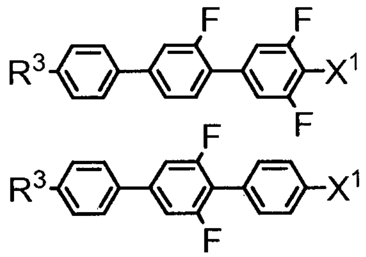
(3-69)

(3-70)

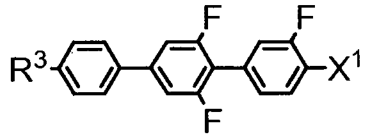
(3-71)

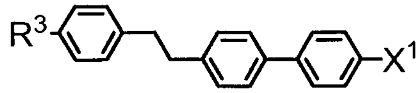
(3-72)

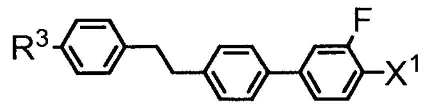
(3-73)

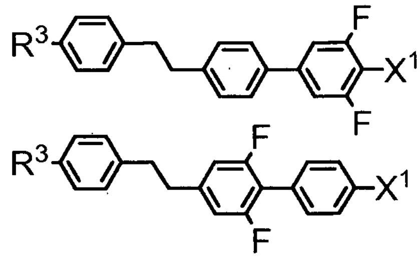
(3-74)

(3-75)

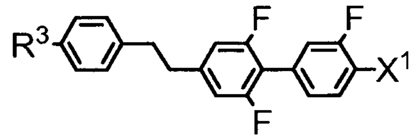
(3-76)

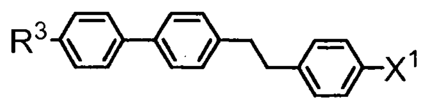
(3-77)

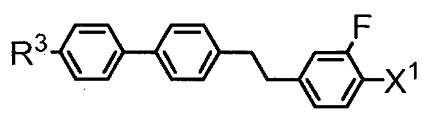
(3-78)

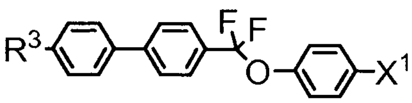
(3-89)

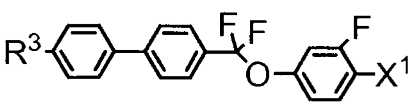
(3-90)

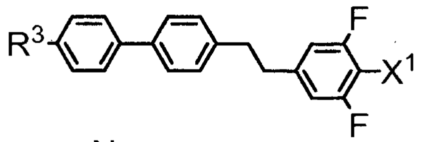
(3-79)

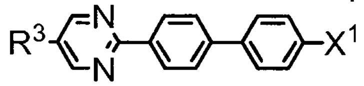
(3-80)

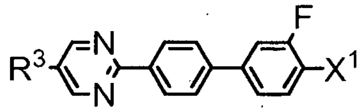
(3-81)

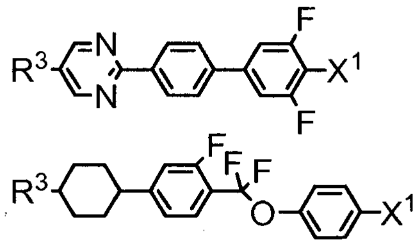
(3-82)

(3-83)

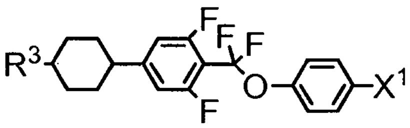
(3-84)

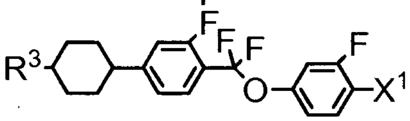
(3-85)

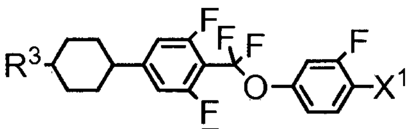
(3-86)

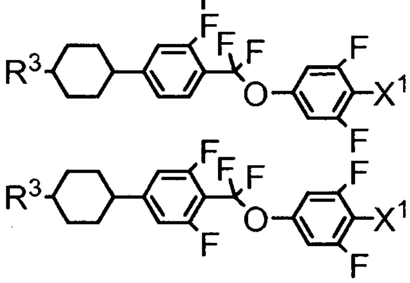
(3-87)

(3-88)

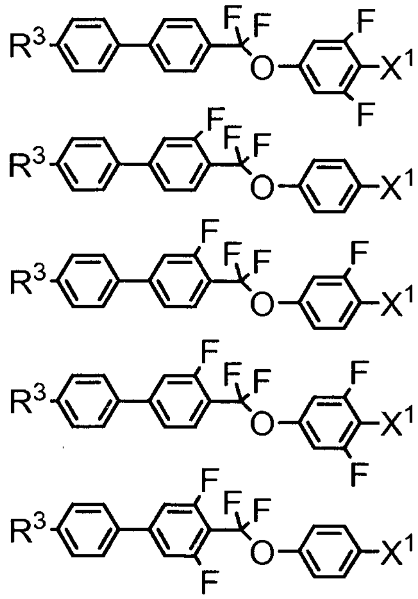
(3-91)

(3-92)

(3-93)

(3-94)

(3-95)

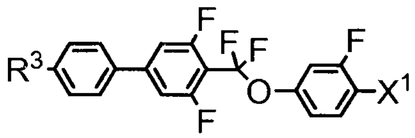
(3-96)

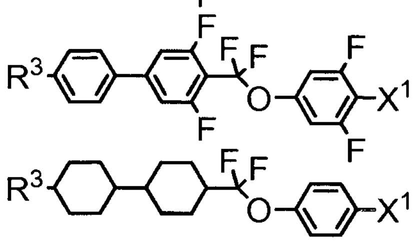
(3-97)

(3-98)

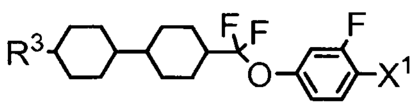
(3-99)

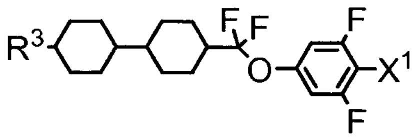
(3-100)

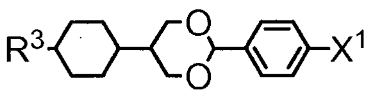
(3-101)

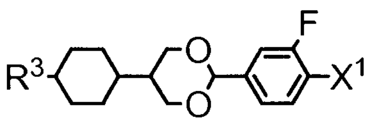
(3-102)

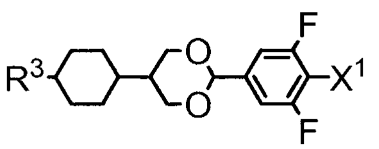
(3-103)

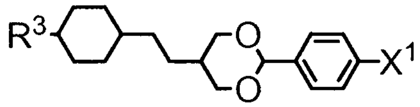
(3-104)

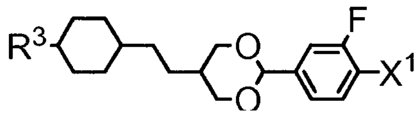
(3-105)

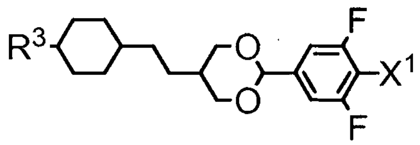
(3-106)

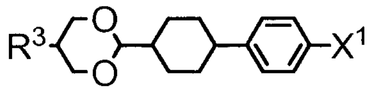
(3-107)

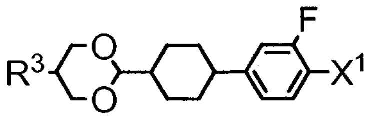
(3-108)

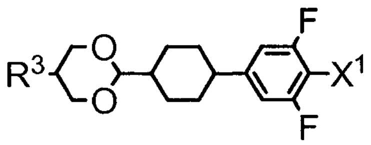
(3-109)

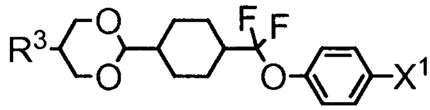
(3-110)

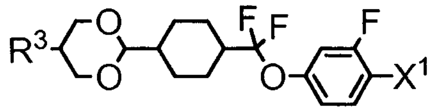
(3-111)

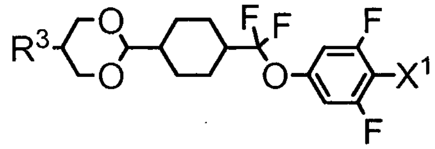
(3-112)

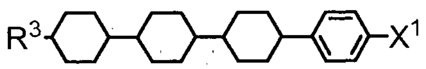 (4-1)

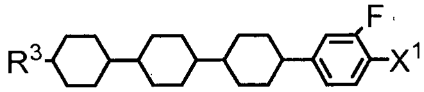 (4-2)

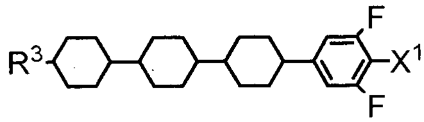 (4-3)

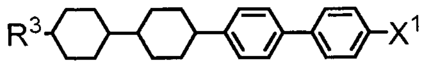 (4-4)

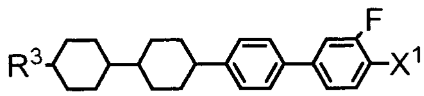 (4-5)

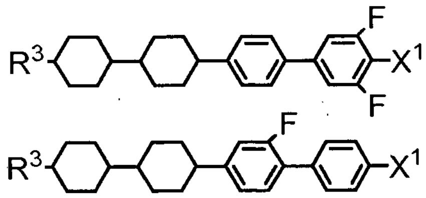 (4-6)

(4-7)

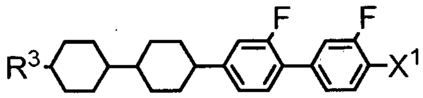 (4-8)

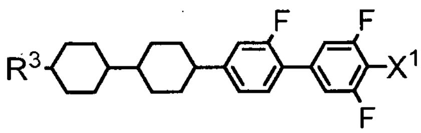 (4-9)

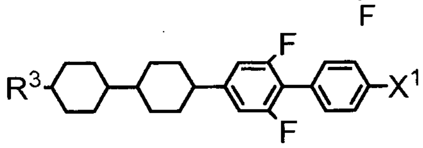 (4-10)

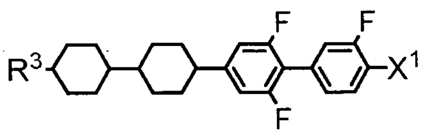 (4-11)

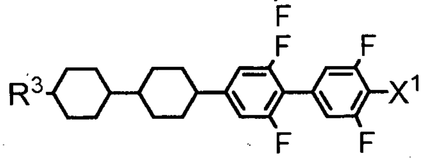 (4-12)

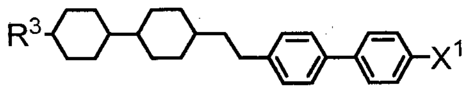 (4-13)

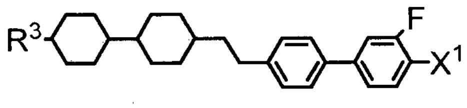 (4-14)

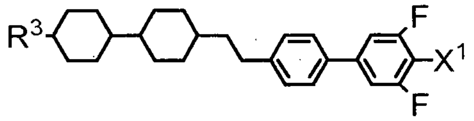 (4-15)

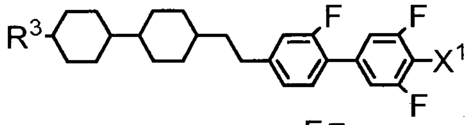 (4-16)

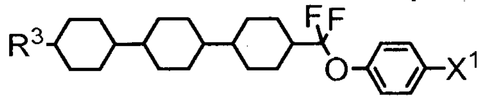 (4-17)

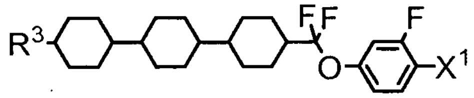 (4-18)

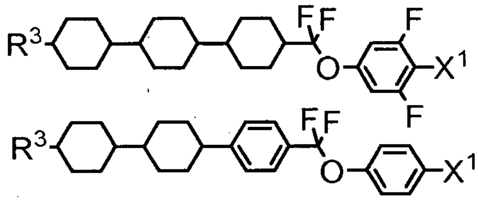 (4-19)

(4-20)

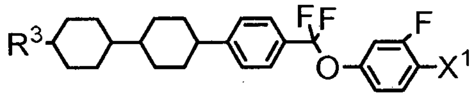 (4-21)

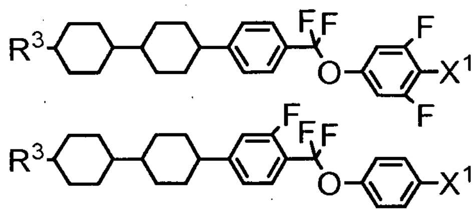 (4-22)

(4-23)

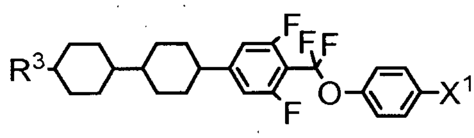 (4-24)

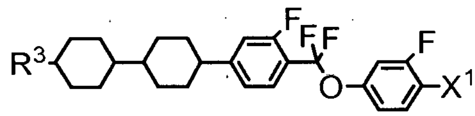 (4-25)

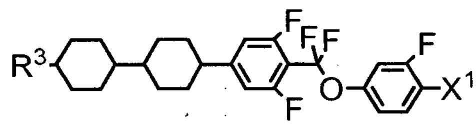 (4-26)

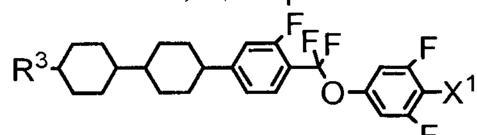 (4-27)

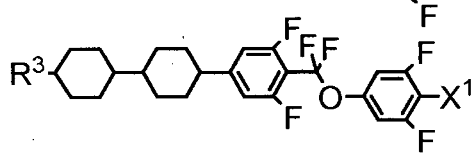 (4-28)

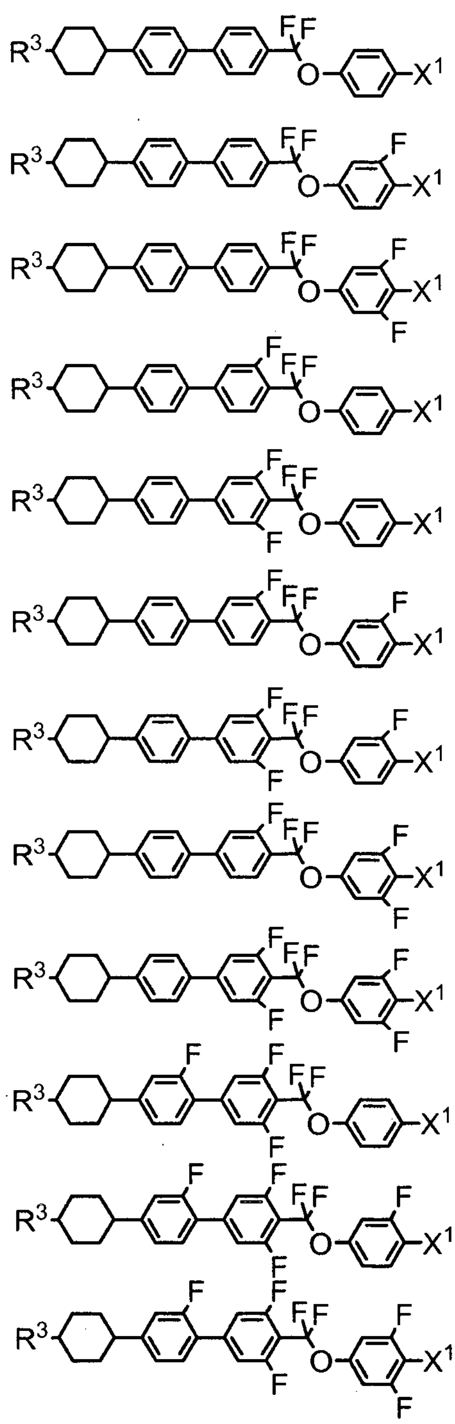

(4-29)

(4-30)

(4-31)

(4-32)

(4-33)

(4-34)

(4-35)

(4-36)

(4-37)

(4-38)

(4-39)

(4-40)

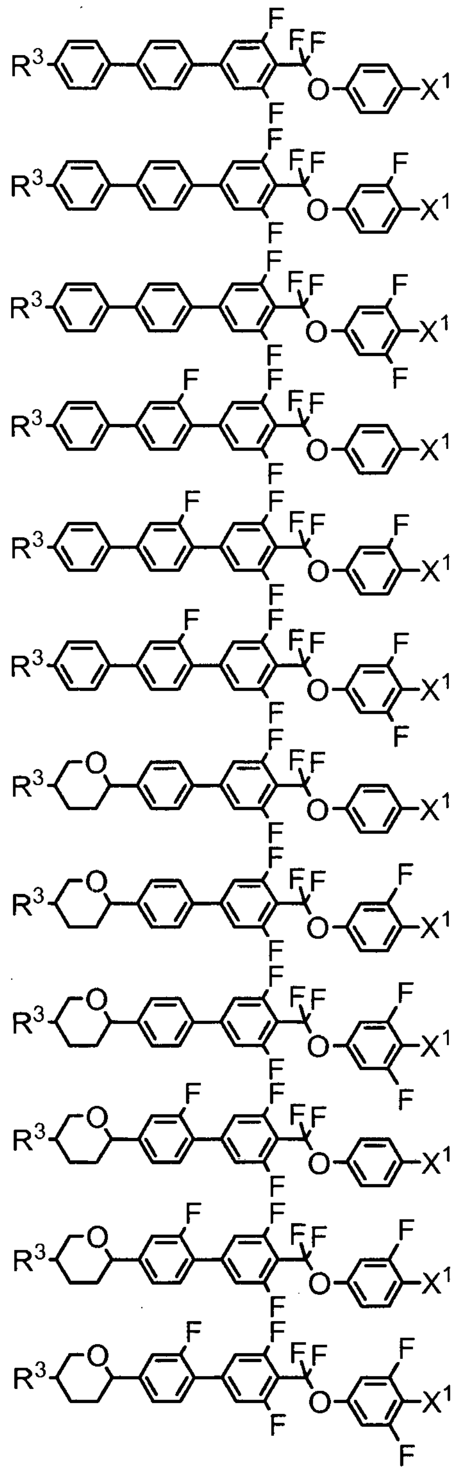

(4-41)

(4-42)

(4-43)

(4-44)

(4-45)

(4-46)

(4-47)

(4-48)

(4-49)

(4-50)

(4-51)

(4-52)

In formulae (2-1) to (2-16), (3-1) to (3-112), and (4-1) to (4-52), $R^3$ and $X^1$ have the same meanings as described above.

The compound represented by formulae (2) to (4), i.e., the component B, is used in the preparation of a liquid crystal composition for a TFT mode device, since it has positive dielectric anisotropy ($\Delta\varepsilon$) and is quite excellent in heat stability and chemical stability. The content of the component B in the liquid crystal composition of the invention is suitably in the range of 1% to 99% by weight, preferably of 10% to 97% by weight, and more preferably of 40% to 95% by weight, based on the total weight of the liquid crystal composition. The viscosity of the composition can be adjusted by further adding a compound represented by formulae (11) to (13) (component E).

Desirable examples of the compound represented by formula (5), i.e., the component C, include compounds represented by formulae (5-1) to (5-64).

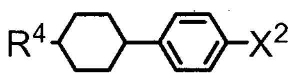
(5-1)

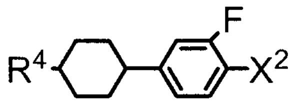
(5-2)

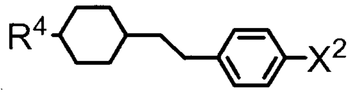
(5-3)

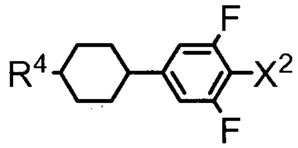
(5-4)

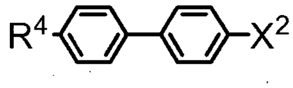
(5-5)

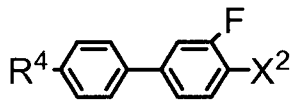
(5-6)

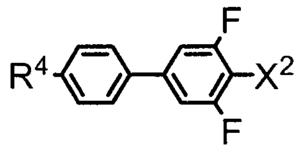
(5-7)

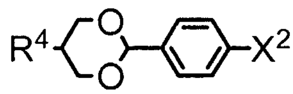
(5-8)

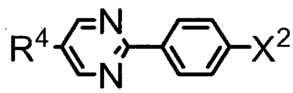
(5-9)

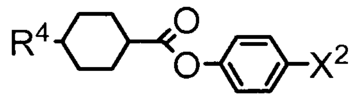
(5-10)

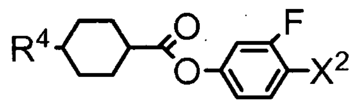
(5-11)

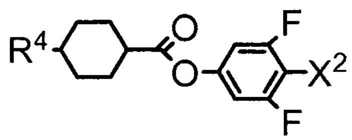
(5-12)

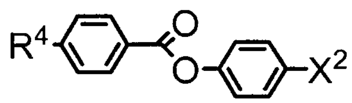
(5-13)

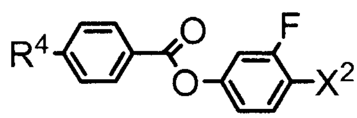
(5-14)

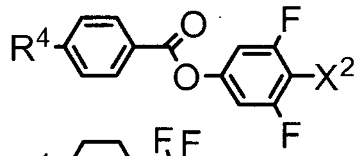
(5-15)

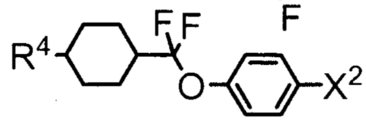
(5-16)

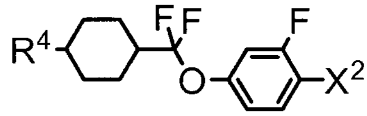
(5-17)

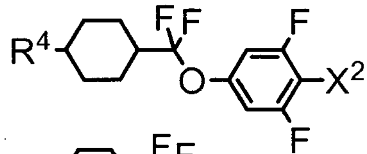
(5-18)

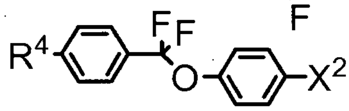
(5-19)

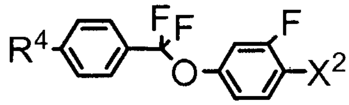
(5-20)

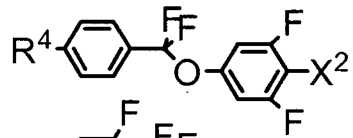
(5-21)

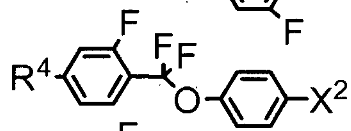
(5-22)

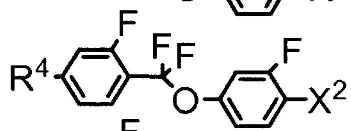
(5-23)

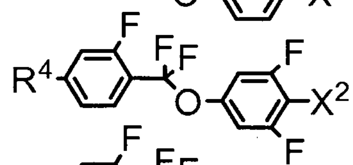
(5-24)

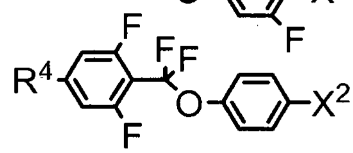
(5-25)

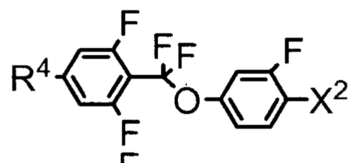
(5-26)

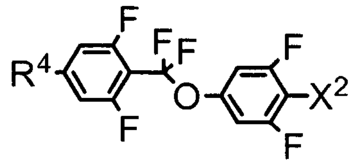
(5-27)

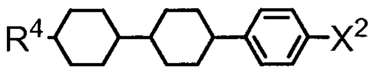 (5-28)

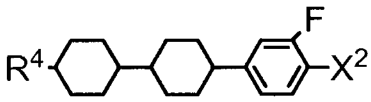 (5-29)

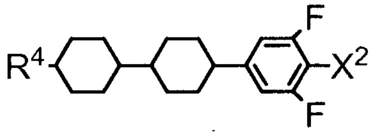 (5-30)

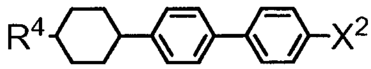 (5-31)

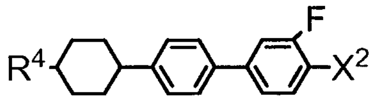 (5-32)

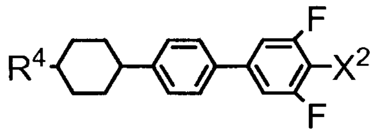 (5-33)

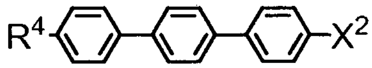 (5-34)

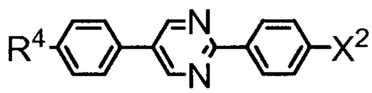 (5-35)

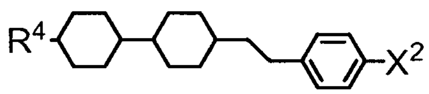 (5-36)

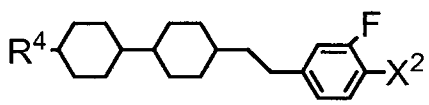 (5-37)

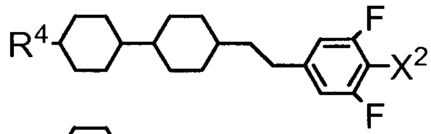 (5-38)

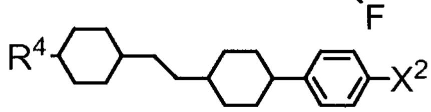 (5-39)

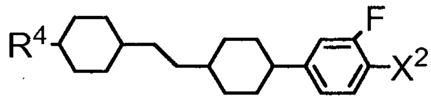 (5-40)

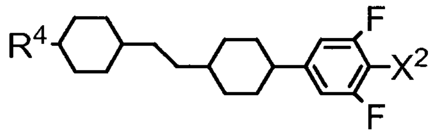 (5-41)

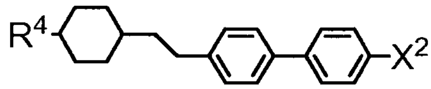 (5-42)

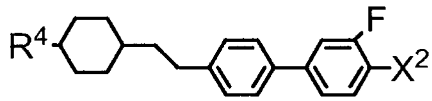 (5-43)

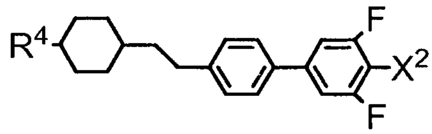 (5-44)

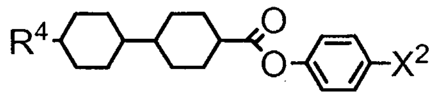 (5-45)

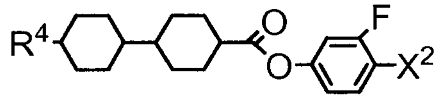 (5-46)

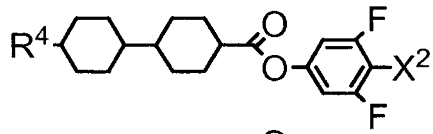 (5-47)

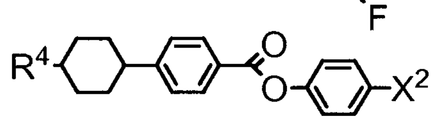 (5-48)

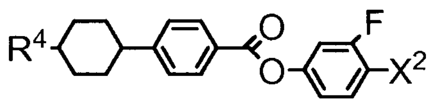 (5-49)

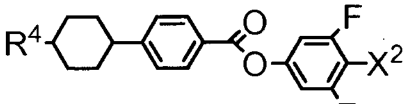
(5-50)

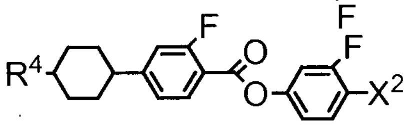
(5-51)

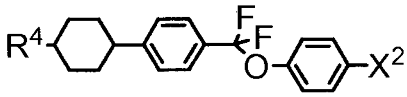
(5-52)

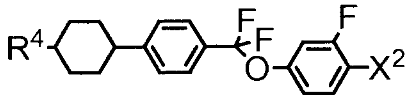
(5-53)

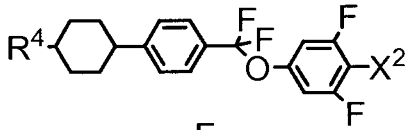
(5-54)

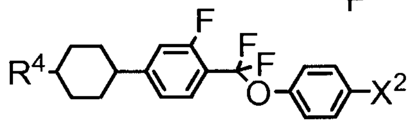
(5-55)

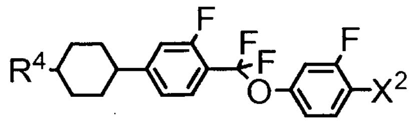
(5-56)

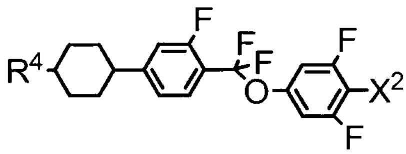
(5-57)

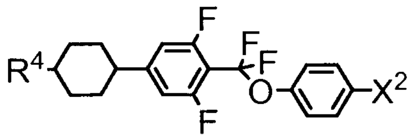
(5-58)

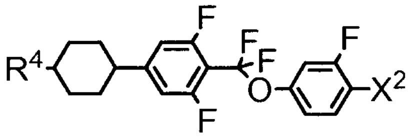
(5-59)

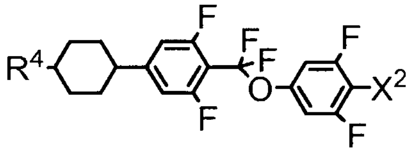
(5-60)

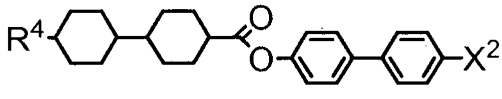
(5-61)

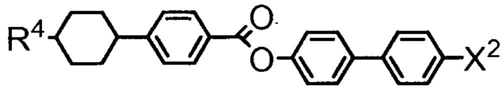
(5-62)

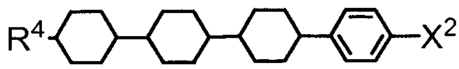
(5-63)

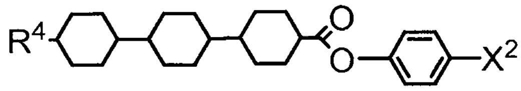
(5-64)

In formulae (5-1) to (5-64), $R^4$ and $X^2$ have the same meanings as described above.

The compound represented by formula (5), i.e., the component C, is used mainly in the preparation of a liquid crystal composition for an STN mode device or a TN mode device, since it has positive dielectric anisotropy ($\Delta\varepsilon$) and its value is very large. The threshold voltage of the composition can be decreased by adding the component C. The viscosity can be adjusted, the refractive index anisotropy ($\Delta n$) can be adjusted, and the temperature range of a liquid crystal phase can be increased by adding the component C. The component C can also be used for an improvement of the steepness.

The content of the component C is suitably in the range of 0.1% to 99.9% by weight in the preparation of a liquid crystal composition for an STN mode device or a TN mode device. The content is preferably in the range of 10% to 97% by weight, and more preferably of 40% to 95% by weight. The threshold voltage, the temperature range of a liquid crystal phase, the refractive index anisotropy ($\Delta n$), the dielectric anisotropy ($\Delta\varepsilon$), the viscosity and so forth can be adjusted by adding components that is described later.

The component D that is at least one compound selected from the group of compounds represented by formulae (6) to (10) is desirable in the preparation of the liquid crystal composition of the invention, in which the dielectric anisotropy ($\Delta\varepsilon$) is negative, for use in a vertical alignment (VA) mode device.

Desirable examples of the compound represented by formulae (6) to (10), i.e., the component D, include compounds represented by formulae (6-1) to (6-5), (7-1) to (7-11), (8-1), (9-1) to (9-3), and (10-1) to (10-11).

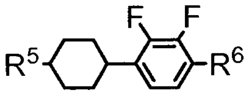
(6-1)

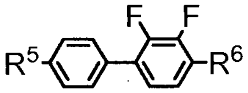
(6-2)

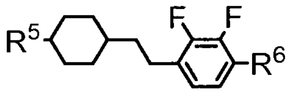
(6-3)

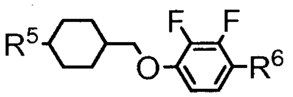
(6-4)

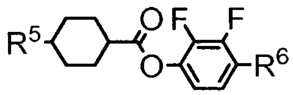
(6-5)

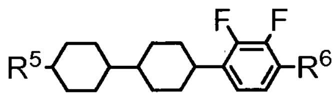
(7-1)

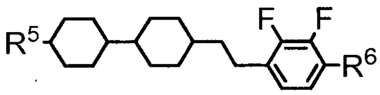
(7-2)

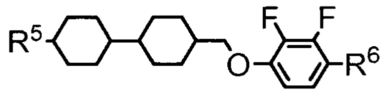
(7-3)

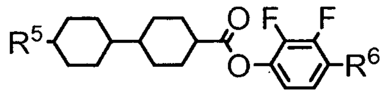
(7-4)

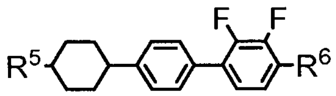
(7-5)

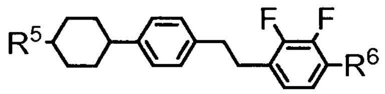
(7-6)

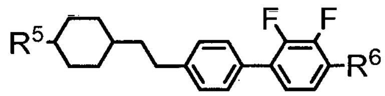
(7-7)

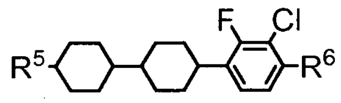
(7-8)

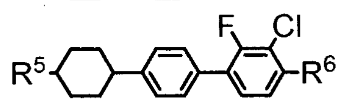
(7-9)

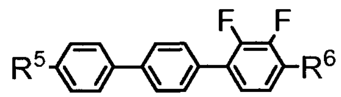
(7-10)

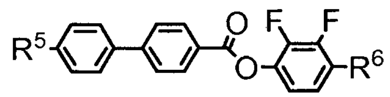
(7-11)

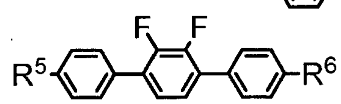
(8-1)

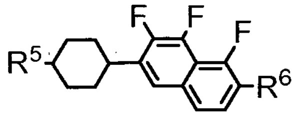
(9-1)

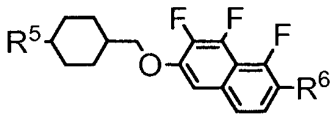
(9-2)

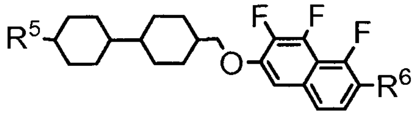
(9-3)

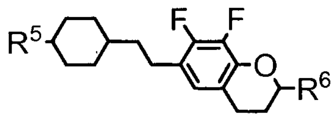
(10-1)

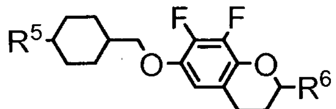
(10-2)

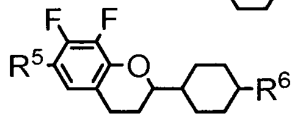
(10-3)

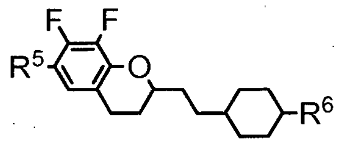
(10-4)

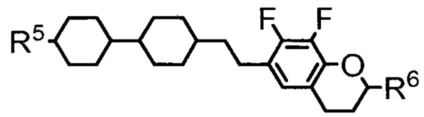
(10-5)

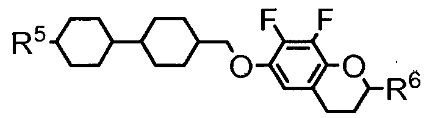
(10-6)

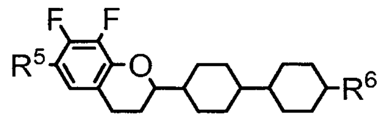
(10-7)

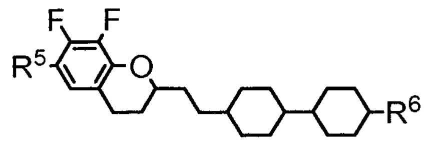
(10-8)

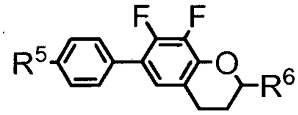
(10-9)

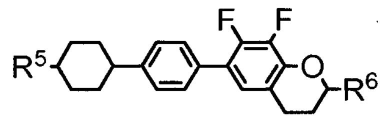
(10-10)

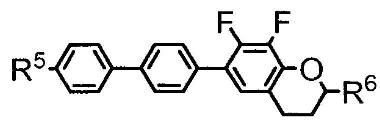
(10-11)

In formulae (6-1) to (6-5), (7-1) to (7-11), (8-1), (9-1) to (9-3), and (10-1) to (10-11), $R^5$ and $R^6$ have the same meanings as described above.

The component D is used mainly in a liquid crystal composition in which the value of the dielectric anisotropy ($\Delta\varepsilon$) is negative, for use in a VA mode device. When the content of the component D increases, the threshold voltage of the composition is decreased, but the viscosity is increased. Accordingly, the content of the component D is preferably smaller, if the demanded value of the threshold value is satisfied. Since the absolute value of the dielectric anisotropy ($\Delta\varepsilon$) is around 5, there are cases where the device may not be driven with voltage when the content of the component is less than 40% by weight.

In the component D, the compound represented by formula (6) has two rings and thus is effective in adjusting the threshold voltage, adjusting the viscosity or adjusting the refractive index anisotropy ($\Delta n$). The compound represented by formulae (7) and (8) has three rings and thus is effective in increasing the clearing point, increasing the temperature range of a nematic phase, decreasing the threshold voltage, increasing the refractive index anisotropy ($\Delta n$), and so forth.

The content of the component D in the preparation of a composition for a VA mode device is preferably 40% by weight or more, and more preferably in the range of 50% to 95% by weight, based on the total amount of the composition. The elastic constant, which relates to the stability of orientation, can be adjusted and the voltage-transmittance curve of the composition can be adjusted by adding the component D. In the case where the component D is added to the composition having positive dielectric anisotropy ($\Delta\varepsilon$), the content is preferably 30% by weight or less based on the total amount of the composition.

The component E is used for adjusting the threshold voltage, the temperature range of a liquid crystal phase, the refractive index anisotropy ($\Delta n$), the dielectric anisotropy ($\Delta\varepsilon$), the viscosity and so forth.

Desirable examples of the compounds represented by formulas (11), (12) and (13), i.e., the component E, include compounds represented by formulae (11-1) to (11-11), (12-1) to (12-18) and (13-1) to (13-6).

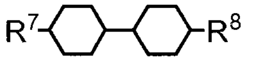 (11-1)

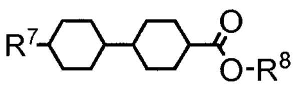 (11-2)

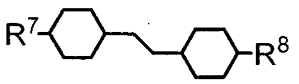 (11-3)

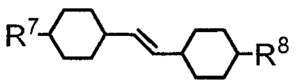 (11-4)

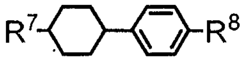 (11-5)

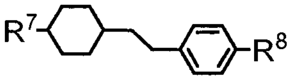 (11-6)

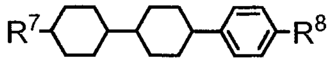 (12-1)

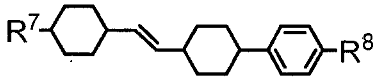 (12-2)

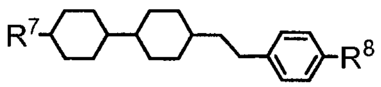 (12-3)

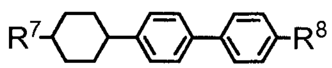 (12-4)

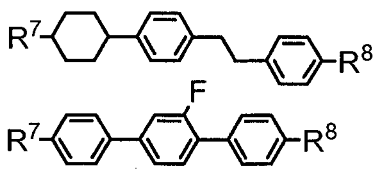 (12-5)

(12-6)

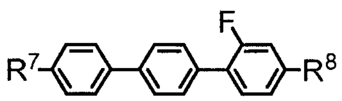 (12-7)

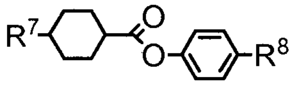
(11-7)

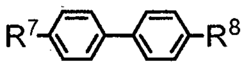
(11-8)

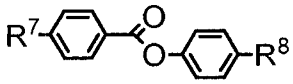
(11-9)

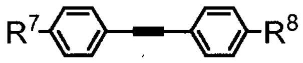
(11-10)

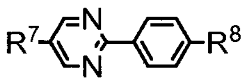
(11-11)

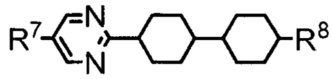
(12-8)

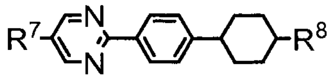
(12-9)

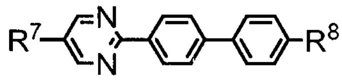
(12-10)

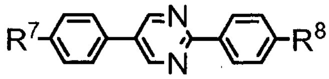
(12-11)

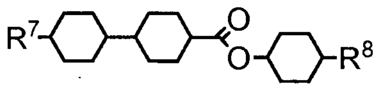
(12-12)

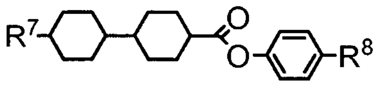
(12-13)

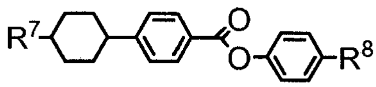
(12-14)

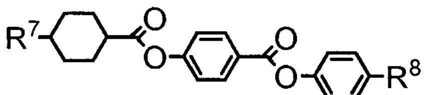
(12-15)

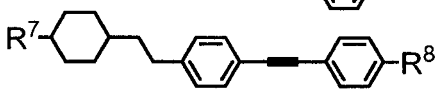
(12-16)

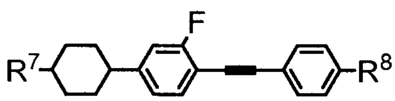
(12-17)

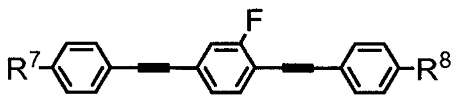
(12-18)

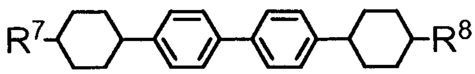
(13-1)

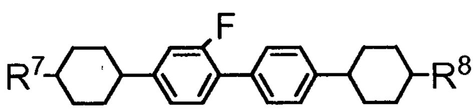
(13-2)

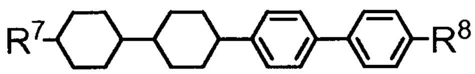
(13-3)

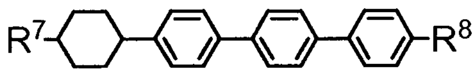
(13-4)

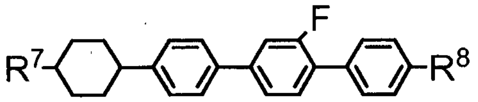

(13-5)

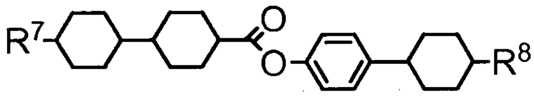

(13-6)

In formulas (11-1) to (11-11), (12-1) to (12-18) and (13-1) to (13-6), $R^7$ and $R^8$ have the same meanings as described above.

The compound represented by formulae (11) to (13), i.e., the component E, has a small absolute value of dielectric anisotropy ($\Delta\varepsilon$) that is to say, the compound is nearly neutral. The compound represented by formula (11) is effective mainly in adjusting the viscosity or adjusting the refractive index anisotropy ($\Delta n$), and the compound represented by formulae (12) and (13) is effective in increasing the temperature range of a nematic phase such as increasing the clearing point, or adjusting the refractive index anisotropy ($\Delta n$)

When the content of the compound of the component E increases, the threshold voltage of the liquid crystal composition is increased and the viscosity is decreased. Accordingly, the content is preferably larger if the demanded value of the threshold voltage of the liquid crystal composition is satisfied. In the case where a liquid crystal composition for a TFT mode device is prepared, the content of the component E is preferably 60% by weight or less, and more preferably 40% by weight or less, based on the total amount of the composition. In the case where a liquid crystal composition for an STN mode device or a TN mode device is prepared, the content of the component E is preferably 70% by weight or less, and more preferably 60% by weight or less, based on the total amount of the composition.

The liquid crystal composition of the invention preferably contains at least one of compounds represented by formula (1-1) or (1-2) of the invention at a rate of 0.1% to 99% by weight for exhibiting excellent characteristics. Corresponding compositions with formula (1-2) compounds are also described.

The liquid crystal composition of the invention can be generally prepared by a known method, for example, by dissolving necessary components at an increased temperature. A liquid crystal composition can be prepared by the addition of an additive that is known to persons skilled in the art depending on the desired application. For example, the liquid crystal composition (f) of the invention containing an optically active compound, which will be described below, and a liquid crystal composition for a GH mode device, in which a dichroic dye is added, can be prepared. Usually, the additive is well known by persons skilled in the art and is reported in detail in the literatures.

The liquid crystal composition (f) of the invention further contains one or more optically active compounds in addition to the liquid crystal composition described above.

A known chiral dopant may be added as the optically active compound. The chiral dopant is effective in inducing a helical structure in liquid crystals, adjusting a twist angle required and then preventing a reverse twist. Examples of the chiral dopant include the following optically active compounds represented by formulae (Op-1) to (Op-13).

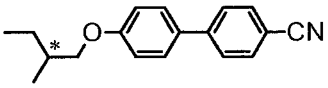

(Op-1)

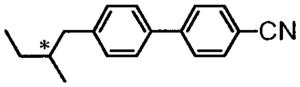

(Op-2)

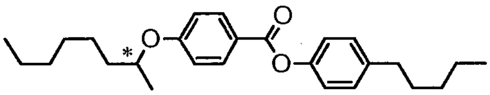

(Op-3)

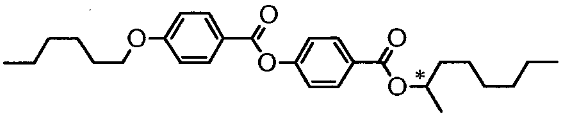

(Op-4)

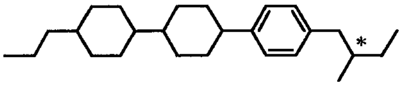

(Op-5)

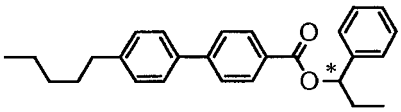

(Op-6)

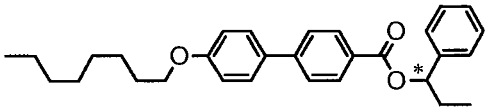

(Op-7)

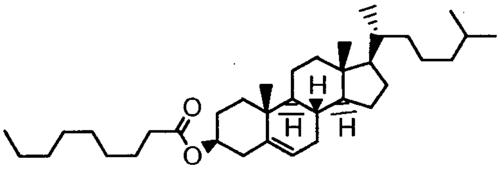

(Op-8)

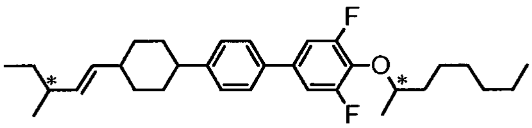

(Op-9)

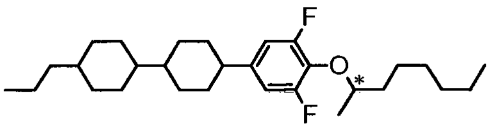

(Op-10)

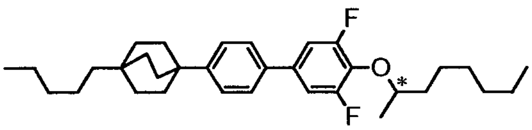

(Op-11)

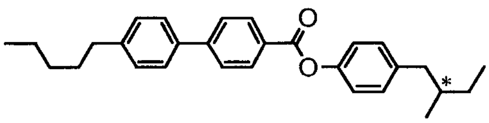

(Op-12)

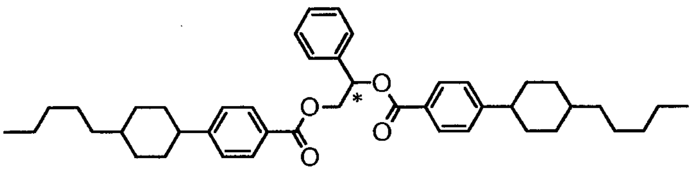

(Op-13)

The helical pitch of the liquid crystal composition (f) of the invention is usually adjusted by adding the optically active compound. The helical pitch is preferably adjusted to the range of 40 μm to 200μ m in the liquid crystal composition for a TFT mode device or a TN mode device. It is preferably adjusted to the range of 6 μm to 20 μm in the liquid crystal composition for an STN mode device. It is preferably adjusted to the range of 1.5 μm to 4 μm in the liquid crystal composition for a bistable TN mode device. Two or more optically active compounds may be added for the purpose of adjusting the temperature dependence of the pitch.

The liquid crystal composition of the invention can be used for a liquid crystal composition for a GH mode device by the addition of a dichroic dye, such as a merocyanine compound, a stylyl compound, an azo compound, an azomethine compound, an azoxy compound, a quinophthalone compound, an anthraquinone compound and a tetrazine compound.

The liquid crystal composition of the invention can be applied to a nematic curvilinear aligned phase (NCAP) device prepared by microcapsulating nematic liquid crystals and a polymer-dispersed liquid crystal display device (PDLCD) produced by forming a three-dimensional network polymer in liquid crystals, for example, a polymer network liquid crystal display device (PNLCD), and also to an electrically controlled birefringence (ECB) mode device and a dynamic scattering (DS) mode device.

## EXAMPLES

The invention will be explained in more detail by means of examples below, but the invention is not construed as being limited to the examples. The term "%" means "% by weight", unless otherwise noted.

The resulting compounds were identified on the basis of magnetic nuclear resonance spectra obtained by $^1$H-NMR analysis, gas chromatograms obtained by gas chromatography (GC) analysis, and so forth. Thus, the analysis methods will be first described.

### $^1$H-NMR Analysis

Bruker DRX-500 (produced by Bruker BioSpin Co., Ltd.) was used for measurement. A sample synthesized in the examples and so forth was dissolved in a deuterated solvent such as $CDCl_3$, in which the sample is soluble, and the measurement was carried out at room temperature and at 500 MHz with integration of 24. Tetramethylsilane (TMS) was used as a standard substance for the zero point of chemical shifts $\delta$.

### GC Analysis

Gas Chromatograph Model GC-14B made by Shimadzu was used for measurement. Capillary column CBP1-M25-025 (length: 25 m, bore: 0.22 mm, film thickness: 0.25 $\mu$m, stationary phase: dimethylpolysiloxane, non-polar) produced by Shimadzu Corp. was used as a column. Helium was used as a carrier gas and adjusted to a flow rate of 1 ml/min. A temperature of the sample injector was 280 °C, and a temperature of the detector (FID) was 300 °C.

The sample was dissolved in toluene to prepare a 1% by weight solution, and 1 $\mu$l of the resulting solution was injected into the sample injector.

Chromatopac Model C-R6A, produced by Shimadzu Corp., or its equivalent was used as a recorder. The resultant gas chromatogram showed the retention time of peaks and the values of peak areas corresponding to the component compounds.

Solvents for diluting the sample may also be chloroform, hexane, and so forth. The following capillary columns may also be used: a capillary column DB-1, produced by Agilent Technologies Inc. (length: 30 m, bore: 0.32 mm, film thickness: 0.25 $\mu$m), a capillary column HP-1, produced by Agilent Technologies Inc. (length: 30 m, bore: 0.32 mm, film thickness: 0.25$\mu$m), a capillary column Rtx-1, produced by Restek Corporation (length: 30 m, bore: 0.32 mm, film thickness: 0.25 $\mu$m), and a capillary column BP-1, produced by SGE International Pty. Ltd. (length: 30 m, bore: 0.32 mm, film thickness: 0.25 $\mu$m).

An area ratio of each peak in the gas chromatogram corresponds to a ratio of each component compound. In general, the percentages by weight of the component compounds of the analyzed sample are not exactly identical to the percentages by area of the peaks of the analyzed sample. According to the invention, however, the percentages by weight of the component compounds of the analyzed sample substantially correspond to the percentages by area of the peaks of the analyzed sample, because the correction coefficient is substantially 1 (one) when the aforementioned columns are used in the invention.

### Sample of Liquid Crystal Compound for Measuring Characteristics

Two kinds of samples are used for measuring physical properties of a liquid crystal compound: one is a compound itself, and the other is a mixture of the compound and mother liquid crystals.

In the latter case where a sample is prepared by mixing the compound with mother liquid crystals, the measurement is carried out in the following manner. A sample was prepared by mixing 15% by weight of the compound with 85% by weight of mother liquid crystals. The values of characteristics of the compound were calculated by extrapolating values obtained by measurement.

Extrapolated Value = (100 x (measured value of sample) - (percentage by weight of mother liquid crystals) x (value measured for mother liquid crystals)) / (percentage by weight of liquid crystal compound)

In the case where a smectic phase was exhibited at 25 °C or crystals were deposited at 25 °C at this ratio of the liquid crystal compound to the mother liquid crystals, the ratio of the compound to the mother liquid crystals was changed step by step in the order of (10% by weight / 90% by weight), (5% by weight / 95% by weight) and (1% by weight / 99% by weight). The values of characteristics of the sample were measured at a ratio where a smectic phase or crystals were not deposited at 25 °C, and extrapolated values were calculated from the aforementioned equation,

which were regarded as the values of characteristics of the liquid crystal compound.

There are various kinds of mother liquid crystals for the measurement, and the composition ratio of the mother liquid crystals A was as follows, for example.

Mother Liquid Crystals A:

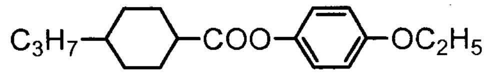 17.2%

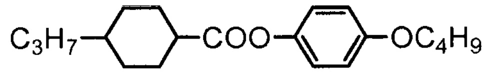 27.6%

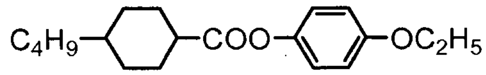 20.7%

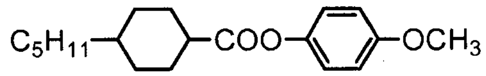 20.7%

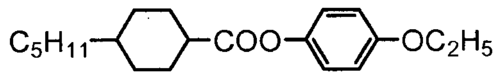 13.8%

As a sample for measuring characteristics of a liquid crystal composition, the liquid crystal composition itself was used.

Method for measuring Characteristics of Liquid Crystal Compound

Measurement of characteristics was carried out according to the following methods. Most methods are described in the Standard of Electric Industries Association of Japan, EIAJ ED-2521 A or those with some modifications. A TFT was not attached to a TN device or a VA device used for measurement.

Among measured values, the values obtained from a liquid crystal compound itself as a sample and the values obtained from a liquid crystal composition itself as a sample were described as experimental data. In the case where the values were obtained from the mixture of a compound and mother liquid crystals, the values obtained by use of extrapolation was regarded as extrapolated values.

**Phase Structure and Phase Transition Temperature (°C)**

The measurement was carried out by the methods (1) and (2) below.

(1) A compound was placed on a hot plate (Hot Stage Model FP-52, produced by Mettler Co., Ltd.) in a melting point apparatus equipped with a polarizing microscope, and while the compound was heated at the rate of 3 °C per minute, the state of the phase and its change were observed with the polarizing microscope to determine the kind of the phase.

(2) A sample was heated and cooled at a rate of 3 °C per minute by using a scanning calorimeter, DSC-7 System or Diamond DSC System, produced by Perkin-Elmer, Inc., whereby a starting point of an endothermic peak or an exothermic peak caused by phase change of the sample was obtained by extrapolation (on set) to determine the phase transition temperature.

In the following description, crystals are denoted by "C". Crystals that are distinguishable into two kinds are denoted by "$C_1$" and "$C_2$". A smectic phase is denoted by "S", and a nematic phase is denoted by "N." A liquid (isotropic phase) is denoted by "Iso". In the case where a smectic phase is distinguished into a smectic B phase and a smectic A phase, these are denoted by "$S_B$" and "$S_A$", respectively. The expression of the phase transition temperature, for example, "C 50.0 N 100.0 Iso", means that the transition temperature from a crystal to a nematic phase (that is, CN) is 50.0 °C, and the transition temperature from a nematic phase to a liquid (that is, NI) is 100.0 °C. The same rule applies to the other expressions.

### Maximum Temperature of Nematic Phase ($T_{NI}$; °C)

A sample (a liquid crystal composition or a mixture of a liquid crystal compound and the mother liquid crystals) was placed on a hot plate (Hot Stage Model FP-52, produced by Mettler Co., Ltd.) in a melting point apparatus equipped with a polarizing microscope. While the sample was heated at the rate of 1 °C per minute, it was observed with the polarizing microscope. A temperature where a part of the sample was changed from a nematic phase to an isotropic liquid was expressed as a maximum temperature of a nematic phase. The maximum temperature of a nematic phase may be abbreviated to "the maximum temperature" in some cases.

### Compatibility at Low Temperature

Samples were prepared by mixing the mother liquid crystals with a liquid crystal compound in order that the ratio of the liquid crystal compound was 20% by weight, 15% by weight, 10% by weight, 5% by weight, 3% by weight and 1% by weight, and then put in glass bottles. The glass bottles were kept in a freezer at -10 °C or -20 °C for a prescribed period of time, and then were observed as to whether or not crystals or a smectic phase was deposited.

### Viscosity (η; measured at 20 °C; mPa·s)

There is a general tendency that the response time is decreased when the viscosity decreases.
The viscosity was measured by means of an E-type viscometer.

### Rotational Viscosity (γ1; measured at 25 °C; mPa·s)

There is a general tendency that response time is decreased when rotational viscosity decreases.
Rotational viscosity was measured according to the method described in M. Imai, et al., Molecular Crystals and Liquid Crystals, vol. 259, p. 37 (1995). A sample (a liquid crystal composition or a mixture of a liquid crystal compound and mother liquid crystals) was put in a VA device in which the cell gap between two glass substrates was 20 μm. A voltage with an increment of 1 volt in the range of 30 V to 50 V was applied stepwise to the device. After a period of 0.2 second with no voltage, a voltage was applied repeatedly under the conditions of only one rectangular wave (rectangular pulse; 0.2 second) and no voltage (2 seconds). The peak current and the peak time of the transient current generated by the applied voltage were measured. The value of rotational viscosity was obtained from the measured values and the calculating equation (8) on page 40 of the paper presented by M. Imai, et al.
The value of the dielectric anisotropy (Δε) necessary for the present calculation was obtained by the method under the heading "dielectric anisotropy (Δε) described below.

### Refractive Index Anisotropy (Δn; measured at 25 °C)

Measurement was carried out by use of an Abbe refractometer with a polarizing plate mounted on the ocular at a temperature of 25 °C, on irradiation with light at a wavelength of 589 nm. The surface of the main prism was rubbed in one direction, and then a sample (a liquid crystal composition or a mixture of a liquid crystal compound and mother liquid crystals) was dropped on the main prism. A refractive index (n||) was measured when the direction of polarized light was parallel to that of the rubbing. A refractive index (n⊥) was measured when the direction of polarized light was perpendicular to that of the rubbing. The value of refractive index anisotropy (Δn) was calculated from the equation: Δn = n|| - n⊥.

### Dielectric Anisotropy (Δε; measured at 25 °C)

A solution of octadecyltriethoxysilane (0.16 ml) in ethanol (20 ml) was coated on a glass substrate that had been fully cleaned. The glass substrate was spun with a spinner and then heated at 150 °C for 1 hour. A VA device in which the distance (cell gap) was 20 μm was set up with two sheets of the glass substrates.
A polyimide alignment film was prepared on a glass substrate in the similar manner. The alignment film on the glass substrate was rubbed, and a TN device was set up in which the distance between two glass substrates was 9 μm and a twist angle was 80 degrees.
A sample (a liquid crystal composition or a mixture of a liquid crystal compound and mother liquid crystals) was put in the VA device, and a voltage of 0.5 V (1 kHz, sine wave) was applied to the device, and then permittivity (ε||) in the major axis direction of the liquid crystal molecules was measured.
The sample (the liquid crystal composition or a mixture of the liquid crystal compound and the mother liquid crystals) was put in the TN device, and a voltage of 0.5 V (1 kHz, sine wave) was applied to the device, and then

permittivity (ε⊥) in the minor axis direction of the liquid crystal molecules was measured.

The dielectric anisotropy (Δε) was calculated from the equation; (Δε) = (ε||) - ($\varepsilon_{\perp}$).

### Synthetic Examples of Liquid Crystal compounds

### Example 1

Preparation of 5-[4-(4-ethoxy-2,3-difluorophenoxymethyl)-cyclohexyl]-2-propyltetrahydropyran (the compound No. 9)

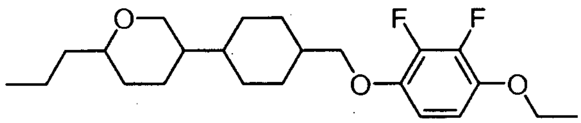

First Step:

THF (200 ml) was added under an atmosphere of nitrogen to methyltriphenylphosphonium bromide (41.2 g) in a reaction vessel, and the mixture was cooled to -20 °C, and t-BuOK (12.9 g) was added thereto and the stirring was continued for another 1 hour. A THF (200 ml) solution of 1, 4-dioxaspiro[4.5]decan-8-one (15.0 g) was added thereto dropwise and the stirring was continued at -20 °C for another 1 hour. The reaction mixture was warmed to room temperature, water (200 ml) was added thereto, and then the mixture was extracted in toluene (300 ml) three times. The resultant organic phase was washed with water, dried over anhydrous magnesium sulfate, and then concentrated under reduced pressure to a volume of about 200 ml. The concentrated solution was poured into n-hexane (1,000 ml) and deposits were filtered off. The solvent was evaporated from the resultant solution under reduced pressure and the residue was purified by silica gel-column chromatography to give 8-methylene-1,4-dioxaspiro[4.5]decane (10.6 g).

Second Step:

8-Methylene-1,4-dioxaspiro[4.5]decane (10.6 g) prepared in First Step was dissolved in THF (50 ml) under an atmosphere of nitrogen in a reaction vessel, and the solution was cooled to 0 °C, and then 9-BBN (0.5 M, in THF; 150 ml) was added dropwise thereto. After 48 hours of stirring at room temperature, the mixture was cooled to 0 °C, and an aqueous solution of sodium hydroxide (6M, 40 ml) was added thereto. Hydrogen peroxide (35% aqueous solution) was added to the solution while being kept at 0 °C. The reaction solution was washed with brine, dried over anhydrous magnesium sulfate, and then the solvent was evaporated under reduced pressure. The residue was purified by silica gel-column chromatography to give (1,4-dioxaspiro[4.5]decen-8-yl)-methanol (7.3 g).

Third Step:

(1,4-Dioxaspiro[4.5]decen-8-yl)-methanol (7.3 g) prepared in Second Step was dissolved in dichloromethane (50 ml) under an atmosphere of nitrogen in a reaction vessel, and toluenesulfonyl chloride (8.1 g) was added thereto. The reaction solution was cooled to 0 °C, and pyridine (6.7 g) was added thereto. The mixture was warmed to room temperature, and the stirring was continued at room temperature for another 2 hours. Toluene was added to the reaction solution and the mixture was washed successively with 2N-hydrochloric acid three times, an aqueous solution of sodium hydroxide and water. The organic phase was dried over anhydrous magnesium sulfate, the solvent was evaporated, and the residue was recrystallized from Solmix-heptane to give (1,4-dioxaspiro[4.5]decen-8-yl)-methanol-p-toluenesulfonic acid ester (13.0 g).

Fourth Step:

(1,4-Dioxaspiro[4.5]decen-8-yl)-methanol-p-toluenesulfonic acid ester (13.0 g) prepared in Third Step and 4-ethoxy-2,3-difluorophenol (8.3 g) were dissolved in N,N-dimethylformamide (200 ml) under an atmosphere of nitrogen in a reaction vessel, and sodium hydride (55%; 2.83 g) was added thereto, and then the stirring was continued at 60 °C for another 3 hours. The reaction solution was cooled with ice, and water (100 ml) was slowly added thereto, separating into two phases. The water phase was extracted in diethyl ether (50 ml) three times, and the combined organic phase was washed with water and dried over anhydrous magnesium sulfate. The solvent was evaporated under reduced pressure, and the residue was purified by silica gel-column chromatography to give 8-(4-ethoxy-2,3-difluorophenoxymethyl)-1,4-dioxaspiro[4.5]decane (11.7 g).

Fifth Step:

8-(4-Ethoxy-2,3-difluorophenoxymethyl)-1,4-dioxaspiro[4.5]decane (11.7 g) prepared in Fourth Step was dissolved in toluene (100 ml) under an atmosphere of nitrogen in a reaction vessel, and formic acid (5 ml) was added thereto, and then the mixture was refluxed for 6 hours. The reaction solution was cooled to room temperature, washed with water, and then dried over anhydrous magnesium sulfate. The solvent was evaporated under reduced pressure and the residue was purified by silica gel-column chromatography to give 4-(4-ethoxy-2,3-difluorophenoxymethyl)-cyclohexanone (8.1 g).

Sixth Step:

THF (50 ml) was added under an atmosphere of nitrogen to methoxymethyltriphenylphosphonium chloride (14.7g) in a reaction vessel and the solution was cooled to -20 °C. t-BuOK (4.8 g) was added thereto and the stirring was continued for another 1 hour. A THF (100 ml) solution of 4-(4-ethoxy-2,3-difluorophenoxymethyl)-cyclohexanone (8.1 g) prepared in Fifth Step was added thereto dropwise and the stirring was continued for another 1 hour. The reaction mixture was warmed to room temperature, water (200 ml) was added thereto, and then the mixture was extracted in toluene (300 ml) three times. The resultant organic phase was washed with water, dried over anhydrous magnesium sulfate, and then concentrated under reduced pressure to a volume of about 200 ml. The concentrated solution was poured into n-hexane (1,000 ml) and deposits were filtered off. The solvent was evaporated from the resultant solution under reduced pressure and the residue was purified by silica gel-column chromatography to give 1-ethoxy-2,3-difluoro-4-(4-methoxymethylenecyclohexylmethoxy)-benzene (8.3 g).

Seventh Step:

1-Ethoxy-2,3-difluoro-4-(4-methoxymethylenecyclohexylmethoxy)-benzene (8.3 g) prepared in Sixth Step was dissolved in acetone (100 ml), and hydrochloric acid (4M; 8 ml) was added thereto, and the stirring was continued at room temperature for another 1 hour. Water (100 ml) was added thereto, and then the mixture was extracted in toluene (50 ml) three times. The combined organic phase was washed with water, and dried over anhydrous magnesium sulfate. The solvent was evaporated under reduced pressure, and toluene (200 ml) and ethanol (200 ml) was added to the residue. Then, an aqueous solution of NaOH (20%; 30 ml) was added to the mixture on an ice bath, and the stirring was continued at room temperature for another 3 hours. The mixture was neutralized by the addition of a saturated aqueous solution of ammonium chloride, and extracted in toluene (100 ml) three times. The combined organic phase was washed with water and dried over anhydrous magnesium sulfate. The solvent was evaporated under reduced pressure, and the residue was purified by silica gel-column chromatography to give 4-(4-ethoxy-2,3-difluoro-phenoxymethyl)-cyclohexanecarboaldehyde (6.6 g).

Eighth Step:

THF (30 ml) was added under an atmosphere of nitrogen to methoxymethyltriphenylphosphonium chloride (9.7 g) in a reaction vessel and cooled to -20 °C, t-BuOK (3.1 g) was added thereto, and the stirring was continued for another 1 hour. A THF (70 ml) solution of 4-(4-ethoxy-2,3-difluoro-phenoxymethyl)-cyclohexanecarboaldehyde (6.6 g) prepared in Seventh Step was added thereto dropwise and the stirring was continued for another 1 hour. The reaction mixture was warmed to room temperature, and water (100 ml) was added thereto, and then the mixture was extracted in toluene (100 ml) three times. The resultant organic phase was washed with water and dried over anhydrous magnesium sulfate, and then concentrated under reduced pressure to a volume of about 100 ml. The concentrated solution was poured into n-hexane (500 ml) and deposits were filtered off. The solvent was evaporated from the resultant solution under reduced pressure, and the residue was purified by silica gel-column chromatography to give 1-ethoxy-2,3-difluoro-4-[4-(2-methoxyvinyl)-cyclohexylmethoxy]-benzene (6.5 g).

Ninth Step:

1-Ethoxy-2,3-difluoro-4-[4-(2-methoxyvinyl)-cyclohexylmethoxy]-benzene (6.5 g) prepared in Eighth Step was dissolved in acetone (100 ml), and hydrochloric acid (4M; 8 ml) was added thereto, and then the stirring was continued at room temperature for another 1 hour. Water (100 ml) was added thereto, and then the mixture was extracted in toluene (50 ml) three times. The combined organic phase was washed with water and dried over anhydrous magnesium sulfate, and the solvent was evaporated under reduced pressure to give [4-(4-ethoxy-2,3-difluorophenoxymethyl)-cyclohexyl]-acetaldehyde (6.1 g).

Tenth Step:

[4-(4-Ethoxy-2,3-difluorophenoxymethyl)-cyclohexyl]-acetaldehyde (6.1 g) prepared in Ninth Step was dissolved in THF (100 ml) under an atmosphere of nitrogen in a reaction vessel, and pyroridine (1.4 g) was added to the mixture under ice-cooling. Potassium carbonate (2.0 g) was added at room temperature and the stirring was continued for another 5 hours. The mixture was filtered and the solvent was evaporated under reduced pressure to give 1-{2-[4-(4-ethoxy-2,3-difluorophenoxymethyl)-cyclohexyl]-vinyl}-pyroridine (7.1 g).

Eleventh Step:

1-{2-[4-(4-Ethoxy-2,3-difluorophenoxymethyl)-cyclohexyl]-vinyl}-pyroridine (7.1 g) prepared in Tenth Step was dissolved in toluene (20 ml) under an atmosphere of nitrogen in a reaction vessel, and then ethyl acrylate (5.9 g) and hydroquinone (0.3 g) were added. The reaction solution was stirred at 80 °C for another 5 hours, and cooled to room temperature, and a saturated aqueous solution of oxalic acid (50 ml) was added thereto. The mixture was extracted in diethyl ether (30 ml) three times, and the combined organic phase was washed with water and dried over anhydrous magnesium sulfate. The solvent was evaporated under reduced pressure and the residue was purified by silica gel-column chromatography to give 4-[4-(4-ethoxy-2,3-difluorophenoxymethyl)-cyclohexyl]-5-oxo-pentanoic acid ethylester (5.4 g).

Twelfth Step:

Ethanol (20 ml) and sodium cyanoborohydride (0.4 g) were added under an atmosphere of nitrogen to 4-[4-(4-ethoxy-2,3-difluorophenoxymethyl)-cyclohexyl]-5-oxo-pentanoic acid ethyl ester (5.4 g) prepared in Eleventh Step in a reaction vessel, and pH of the mixture was adjusted so as to be about 3 by the addition of hydrochloric acid (2M), and then the stirring was continued at room temperature for another 15 hours. Water (30 ml) was added thereto, and then the mixture was extracted in toluene (20 ml) three times. The combined organic phase was washed with water and dried over anhydrous magnesium sulfate, and the solvent was evaporated under reduced pressure. The residue was put in a flask equipped with a Dean-Stark apparatus, and toluene (100 ml) and p-Toluenesulfonic acid monohydrate (0.1 g) were added thereto, and then the mixture was refluxed for 1 hour while part of the solvent was distilled off with a Dean-Stark apparatus. The solution was cooled to room temperature, washed with water, and dried over anhydrous magnesium sulfate. The solvent was evaporated under reduced pressure and the residue was purified by silica gel-column chromatography to give 5-[4-(4-ethoxy-2,3-difluorophenoxymethyl)-cyclohexyl]-tetrahydropyran-2-one (4.5 g).

Thirteenth Step:

THF (100 ml) was added under an atmosphere of nitrogen to 5-[4-(4-ethoxy-2,3-difluorophenoxymethyl)-cyclohexyl]-tetrahydropy ran-2-one (4.5 g) prepared in Twelfth Step in a reaction vessel, and the solution was cooled to -70 °C, and then n-propyllithium (about 1.0 mmol; 15 ml) was added dropwise thereto. The mixture was stirred at -70 °C for another 1 hour and warmed to room temperature. A saturated aqueous solution of ammonium chloride (50 ml) was added thereto and the mixture was separated into two phases. The water phase was extracted in diethyl ether (30 ml) three times and the combined organic phase was washed with water and dried over anhydrous magnesium sulfate. The solvent was evaporated under reduced pressure and the residue was purified by silica gel-column chromatography to give 5-[4-(4-ethoxy-2,3-difluorophenoxymethyl)-cyclohexyl]-2-propyltetrahydropyran-2-ol (3.6 g).

Fourteenth Step:

Dichloromethane (100 ml) and acetonitrile (20 ml) were added under an atmosphere of nitrogen to 5-[4-(4-ethoxy-2,3-difluorophenoxymethyl)-cyclohexyl]-2-propyltetrahydropyran-2-ol (3.6 g) prepared in Thirteenth Step in a reaction vessel. After the solution had been cooled to -20 °C, triethylsilane (2.7 ml) was added dropwise, and boron trifluoride-ethyl ether complex (1.5 ml) was successively added dropwise. The reaction solution was warmed to 0 °C, ice-water (50 ml) was added thereto, and the mixture was extracted in diethyl ether (30 ml) three times. The combined organic phase was washed with water and dried over anhydrous magnesium sulfate. The solvent was evaporated under reduced pressure and the residue was purified by silica gel-column chromatography and by recrystallization to give 5-[4-(4-ethoxy-2,3-difluorophenoxymethyl)-cyclohexyl]-2-propyltetrahydropyran (2.0 g).

The chemical shift ($\delta$, ppm) in $^1$H-NMR analysis was described below, and the compound obtained was identified as 5-[4-(4-ethoxy-2,3-difluorophenoxymethyl)-cyclohexyl]-2-propyltetrahydropyran. The solvent for measurement was

$CDCl_3$.

Chemical shift (δ, ppm); 6.60(m, 2H), 4.05(q, 2H), 4.00(m, 1H), 3.75(d, 2H), 3.17(m, 1H), 3.14(t, 2H), 2.0-1.6(m, 6H), 1.6-1.3(m, 8H), 1.20(m, 2H), 1.1-1.0(m, 5H) and 0.91(t, 3H).

The phase transition temperature of the compound No. 9 was as follows.

Phase transition temperature: C 59.4 N 126.6 Iso.

**Example 2**

Preparation of 5-[4-(4-ethoxy-2,3-difluorophenoxymethyl)-cyclohexyl]-2-pentyltetrahydropyran (the compound No. 11)

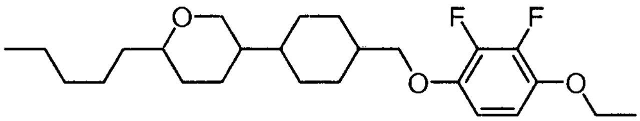

5-[4-(4-Ethoxy-2,3-difluorophenoxymethyl)-cyclohexyl]-2-pentyltetrahydropyran-2-pentyltetrahydropyran was prepared in the same manner as described in Thirteenth Step of Example 1, using n-pentyllithium instead of n-propyllithium.

The chemical shift (δ, ppm) in $^1H$-NMR analysis was described below, and the compound obtained was identified as 5-[4-(4-ethoxy-2,3-difluorophenoxymethyl)-cyclohexyl]-2-pentyltetrahydropyran. The solvent for measurement was $CDCl_3$.

Chemical shift (δ, ppm); 6.60(m, 2H), 4.05(q, 2H), 4.00(m, 1H), 3.75(d, 2H), 3.14(m, 1H), 3.13(t, 2H), 2.0-1.6(m, 6H), 1.6-1.1(m, 14H), 1.1-1.0(m, 5H) and 0.91(t, 3H).

The phase transition temperature of the compound No. 11 was as follows.

Phase transition temperature: C 47.3 N 126.2 Iso.

**Example 3**

Preparation of 5-[4-(4-ethoxy-2,3-difluorophenoxymethyl)-cyclohexyl]-2-vinyltetrahydropyran (the compound No. 12)

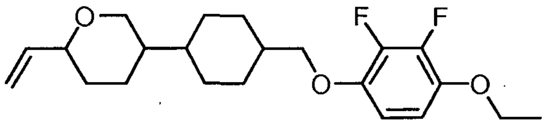

First Step:

Trimethylsilylacetylene (2.55 g) was dissolved in THF (20 ml) and n-butyllithium (1.66 M, in hexane; 15.7 ml) was added dropwise thereto at -70 °C, and the stirring was continued at the same temperature for another 1 hour. A THF (30 ml) solution of 5-[4-(4-ethoxy-2,3-difluorophenoxymethyl)-cyclohexyl]-tetrahydropyran-2-one (9.21 g) prepared in Twelfth Step of Example 1 was added dropwise thereto at -70 °C. The stirring was continued for another 1 hour, and then the mixture was gradually warmed to room temperature. The reaction mixture was poured into a saturated aqueous solution of ammonium chloride (50 ml), and extracted in diethyl ether. The organic phase was washed with water and concentrated under reduced pressure. The resultant brown residue was purified by silica gel-column chromatography to give 5-[4-(4-ethoxy-2,3-difluorophenoxymethyl)-cyclohexyl]-2-trimethylsilanylethynyltetrahydropyran-2-ol (10.7 g) as brown solids.

Second Step:

5-[4-(4-Ethoxy-2,3-difluorophenoxymethyl)-cyclohexyl]-2-trimethylsilanylethynyltetrahydropyran-2-ol (10.7g) prepared in First Step was dissolved in dichloromethane (150 ml), and triethylsilane (5.5 ml) was added dropwise to the solution at -50 °C and boron trifluoride-diethyl ether complex (3.5 ml) was successively added dropwise. The mixture was gradually warmed to 0 °C, and then poured into ice-water (150 ml), and extracted in n-heptane. The organic phase was washed with water and was concentrated under reduced pressure. The resultant brown residue was dissolved in dichloromethane (100 ml), and then methanol (100 ml) and an aqueous solution of sodium hydroxide (1M, 20 ml) were

added thereto, and the stirring was continued at room temperature for another 2 hours. The mixture was neutralized by hydrochloric acid (1M) and extracted in diethyl ether. The organic phase was washed with water and concentrated under reduced pressure. The resultant brown residue was purified by silica gel-column chromatography to give 5-[4-(4-ethoxy-2,3-difluorophenoxymethyl)-cyclohexyl]-2-ethynyltetrahydropyran (7.55 g) as a yellow oil.

Third Step:

5-[4-(4-Ethoxy-2,3-difluorophenoxymethyl)-cyclohexyl]-2-ethynyltetrahydropyran (7.55 g) prepared in Second Step was dissolved in n-heptane (200 ml), and quinoline (1.5 ml) and Lindlar's catalyst (0.15 g) were added thereto. After the pressure of a reaction vessel had been reduced with a vacuum pump, hydrogen under normal pressure was introduced and the stirring was continued over night at room temperature. The catalyst was removed from the reaction mixture by filtration and the mixture was concentrated under reduced pressure. The resultant colorless residue was purified by silica gel-column chromatography to give 5-[4-(4-ethoxy-2,3-difluorophenoxymethyl)-cyclohexyl]-2-vinyltetrahydropyran (7.4 g) as a colorless oil. The product was further purified by repeated recrystallization to give pure 5-[4-(4-ethoxy-2,3-difluorophenoxymethyl)-cyclohexyl]-2-vinyltetra hydropyran (2.6 g).

The chemical shift (δ, ppm) in $^1$H-NMR analysis was described below, and the compound obtained was identified as 5-[4-(4-ethoxy-2,3-difluorophenoxymethyl)-cyclohexyl]-2-vinyltetrahydropyran. The solvent for measurement was $CDCl_3$.

Chemical shift (δ, ppm); 6.60(m, 2H), 5.85(m, 1H), 5.23(d, 1H), 5.09(d, 1H), 4.06(m, 3H), 3.76(d, 1H), 3.71(m, 1H), 3.21(t, 2H), 2.0-1.6(m, 7H), 1.5-1.3(m, 5H), 1.23(m, 1H) and 1.05(m, 5H).

The phase transition temperature of the compound No. 12 was as follows.

Phase transition temperature: C 70.4 N 126.2 Iso.

The following compounds can be prepared according to the methods described in Examples 1 to 3 and the methods described before, those outside the scope of claim 1 are reference compounds for illustrative purposes only.

| No. | | No. | |
|---|---|---|---|
| 1 | 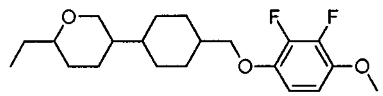 | 15 | 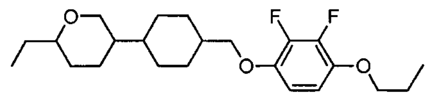 |
| 2 | 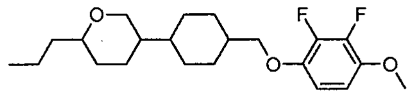 | 16 | 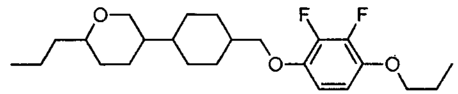 |
| 3 | 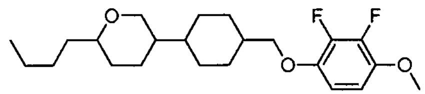 | 17 | 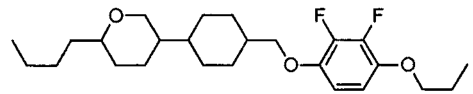 |
| 4 | 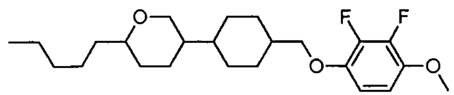 | 18 | 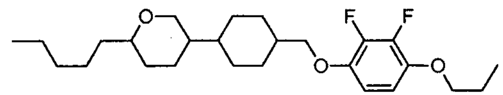 |
| 5 | 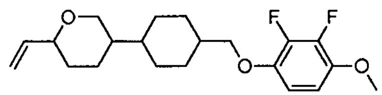 | 19 | 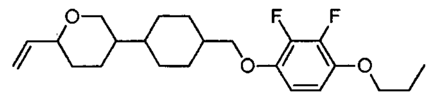 |
| 6 | 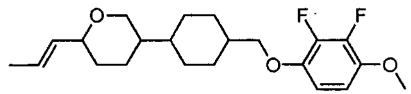 | 20 | 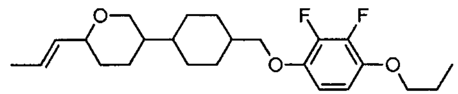 |
| 7 | 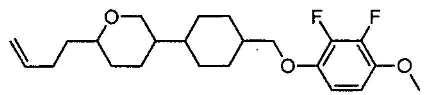 | 21 | 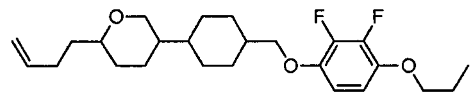 |
| 8 | 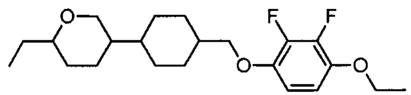 | 22 | 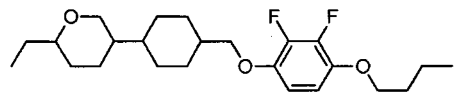 |
| 9 | 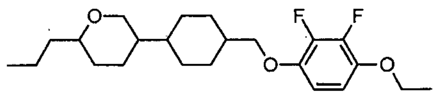 | 23 | 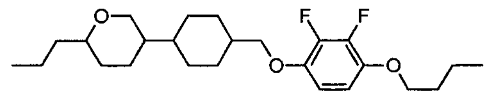 |
| 10 | 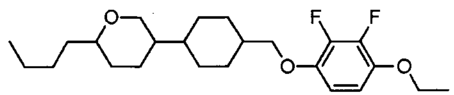 | 24 | 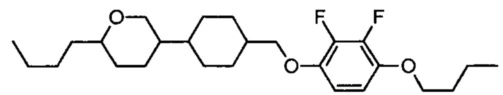 |
| 11 | 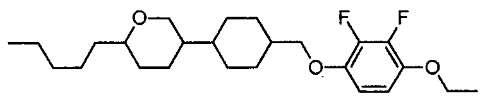 | 25 | 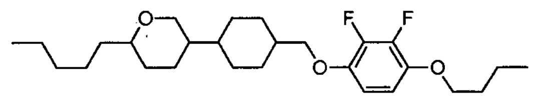 |

(continued)

| No. | | No. | |
|---|---|---|---|
| 12 | 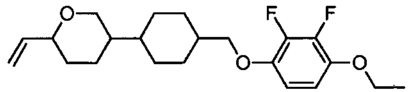 | 26 | 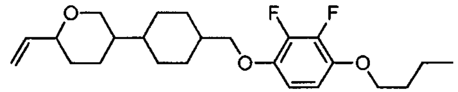 |
| 13 | 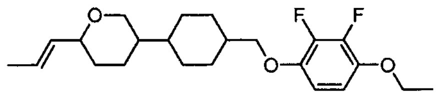 | 27 | 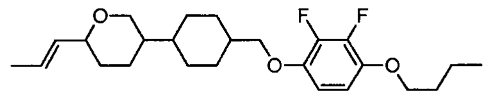 |
| 14 | 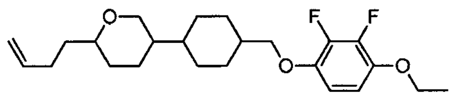 | 28 | 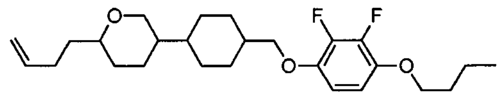 |
| 29 | 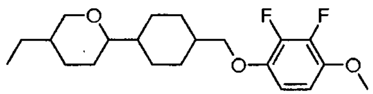 | 43 | 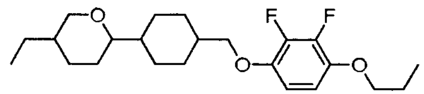 |
| 30 | 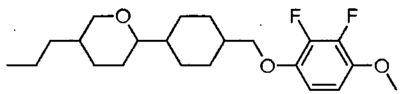 | 44 | 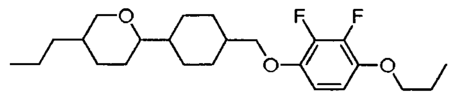 |
| 31 | 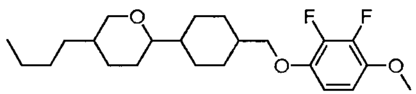 | 45 | 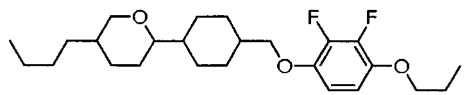 |
| 32 | 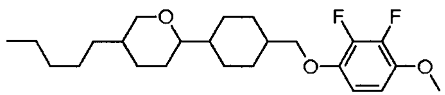 | 46 | 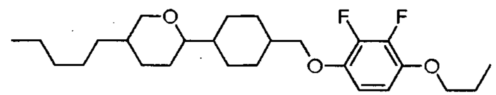 |
| 33 | 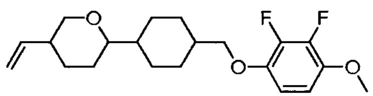 | 47 | 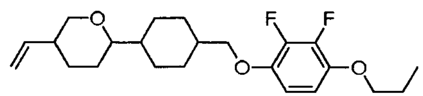 |
| 34 | 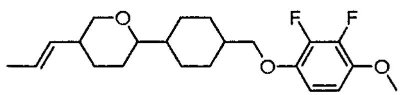 | 48 | 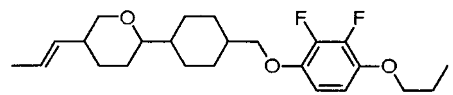 |
| 35 | 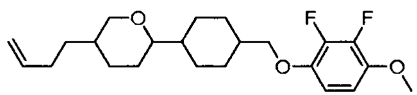 | 49 | 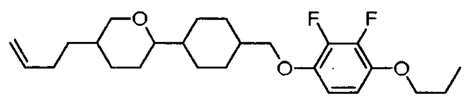 |
| 36 | 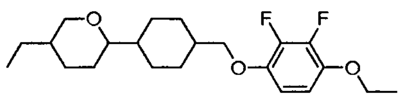 | 50 | 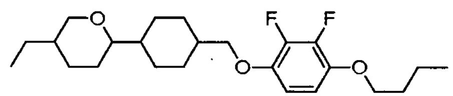 |

(continued)

| No. | | No. | |
|---|---|---|---|
| 37 | 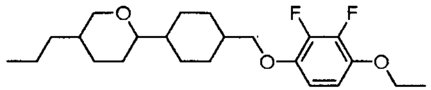 | 51 | 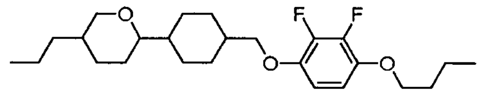 |
| 38 | 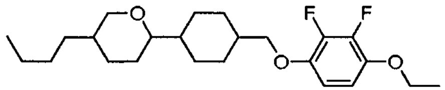 | 52 | 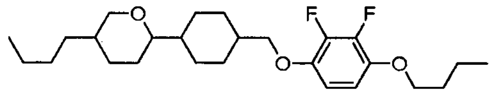 |
| 39 | 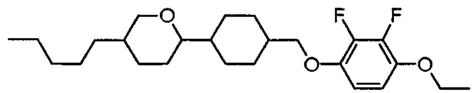 | 53 | 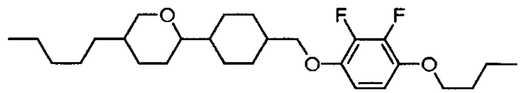 |
| 40 | 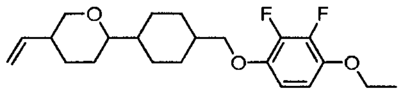 | 54 | 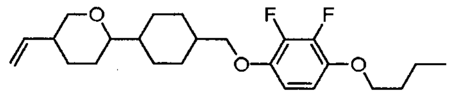 |
| 41 | 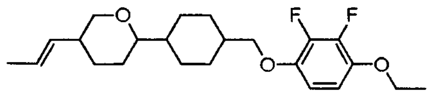 | 55 | 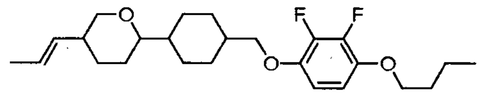 |
| 42 | 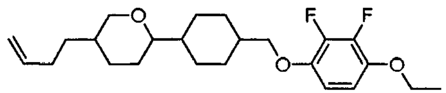 | 56 | 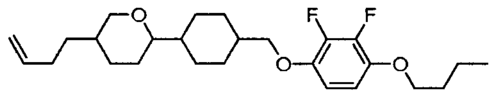 |
| 57 | 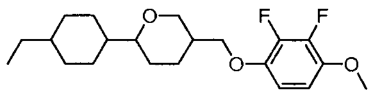 | 71 | 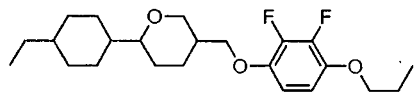 |
| 58 | 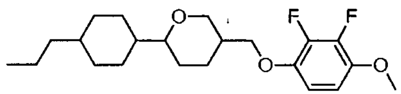 | 72 | 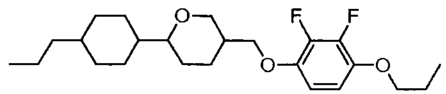 |
| 59 | 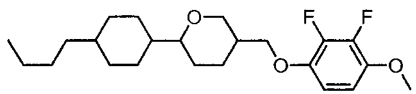 | 73 | 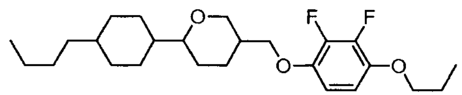 |
| 60 | 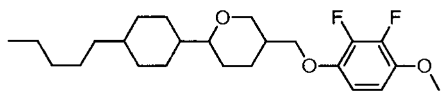 | 74 | 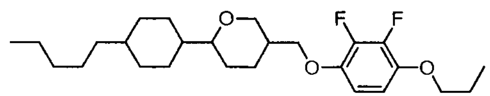 |
| 61 | 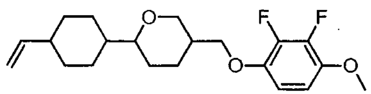 | 75 | 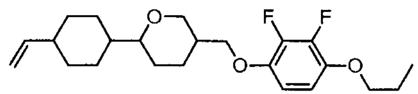 |

(continued)

| No. | | No. | |
|---|---|---|---|
| 62 | 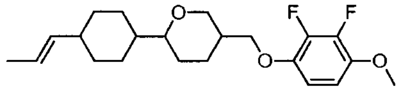 | 76 | 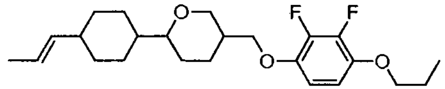 |
| 63 | 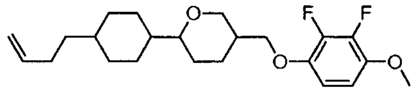 | 77 | 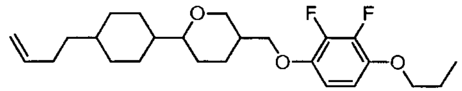 |
| 64 | 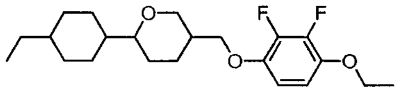 | 78 | 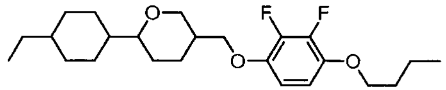 |
| 65 | 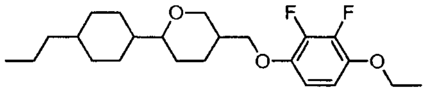 | 79 | 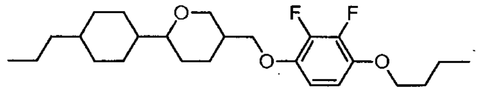 |
| 66 | 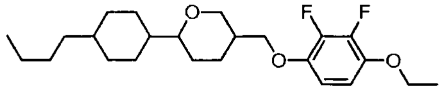 | 80 | 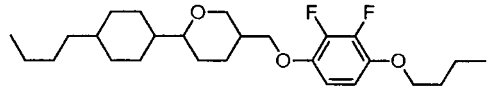 |
| 67 | 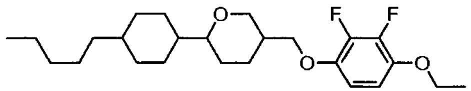 | 81 | 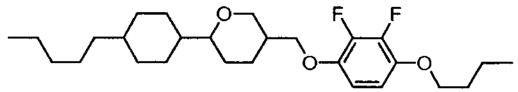 |
| 68 | 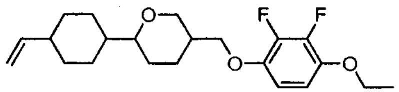 | 82 | 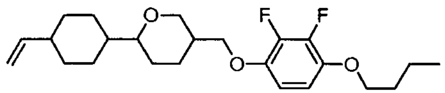 |
| 69 | 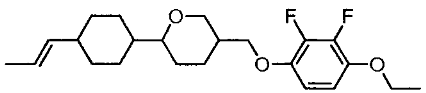 | 83 | 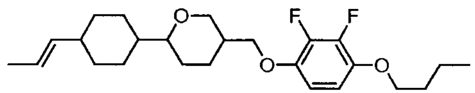 |
| 70 | 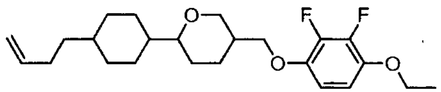 | 84 | 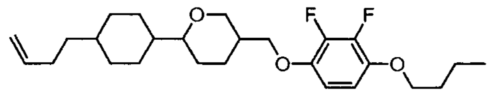 |
| 85 | 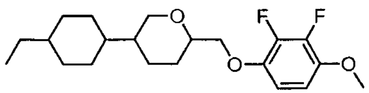 | 99 | 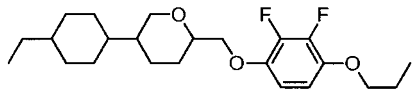 |
| 86 | 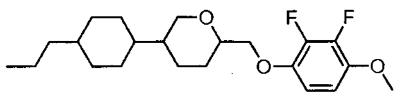 | 100 | 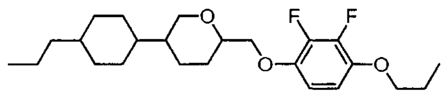 |

(continued)

| No. | | No. | |
|---|---|---|---|
| 87 | 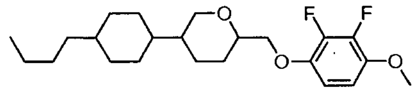 | 101 | 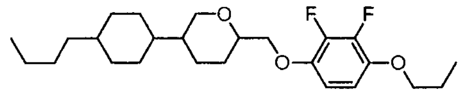 |
| 88 | 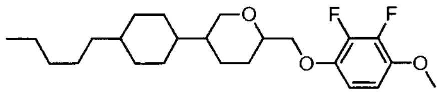 | 102 | 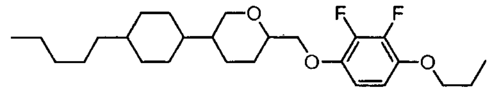 |
| 89 | 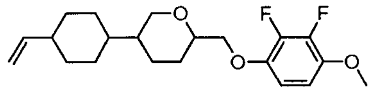 | 103 | 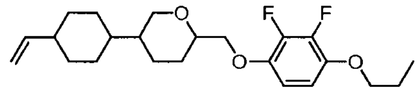 |
| 90 | 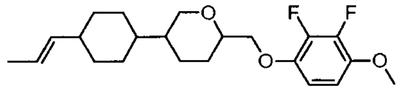 | 104 | 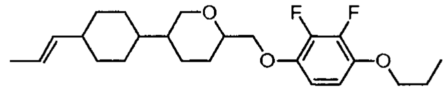 |
| 91 | 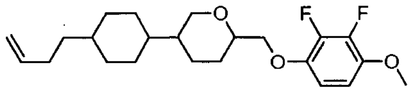 | 105 | 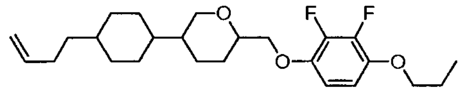 |
| 92 | 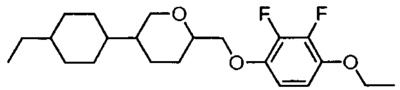 | 106 | 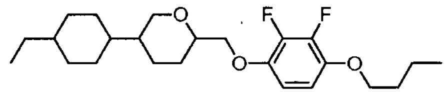 |
| 93 | 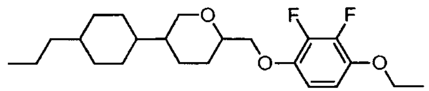 | 107 | 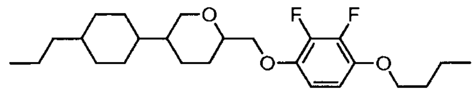 |
| 94 | 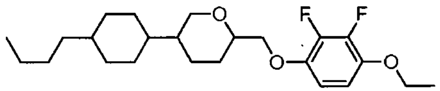 | 108 | 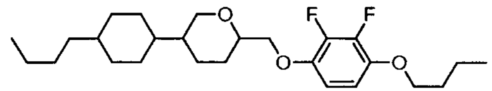 |
| 95 | 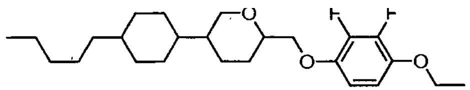 | 109 | 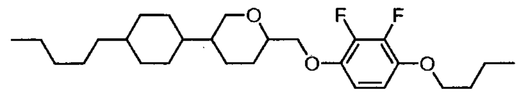 |
| 96 | 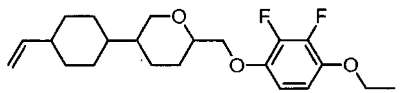 | 110 | 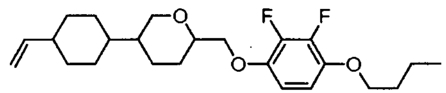 |
| 97 | 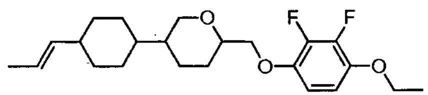 | 111 | 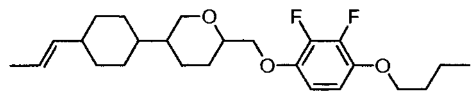 |

(continued)

| No. | | No. | |
| --- | --- | --- | --- |
| 98 | 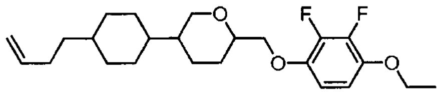 | 112 | 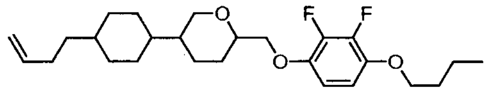 |
| 113 | 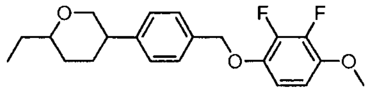 | 127 | 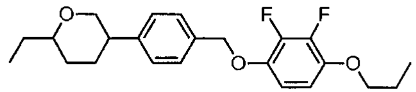 |
| 114 | 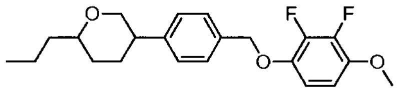 | 128 | 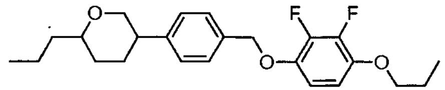 |
| 115 | 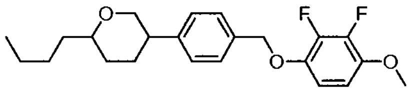 | 129 | 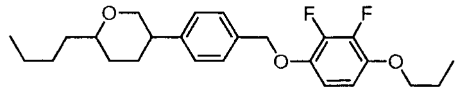 |
| 116 | 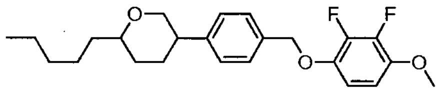 | 130 | 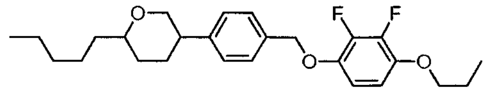 |
| 117 | 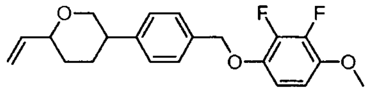 | 131 | 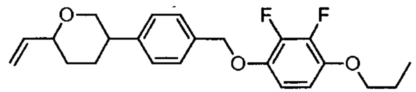 |
| 118 | 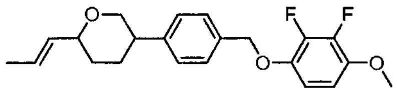 | 132 | 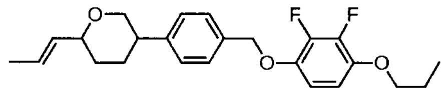 |
| 119 | 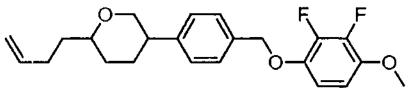 | 133 | 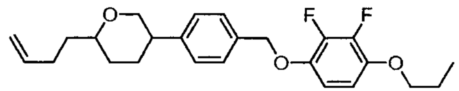 |
| 120 | 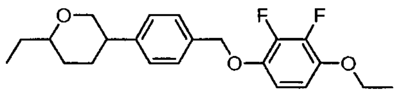 | 134 | 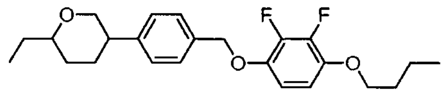 |
| 121 | 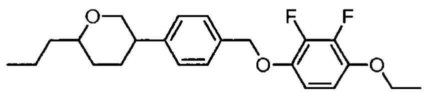 | 135 | 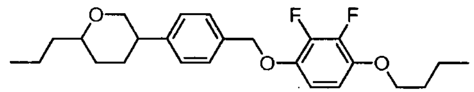 |
| 122 | 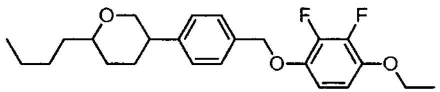 | 136 | 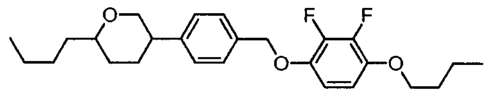 |

(continued)

| No. | | No. | |
|---|---|---|---|
| 123 | 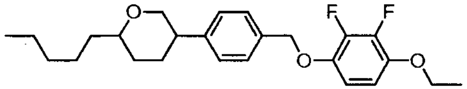 | 137 | 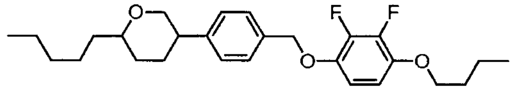 |
| 124 | 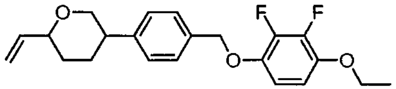 | 138 | 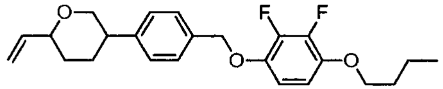 |
| 125 | 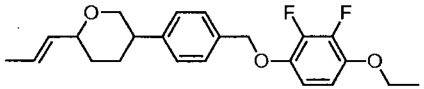 | 139 | 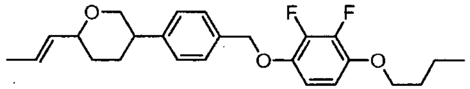 |
| 126 | 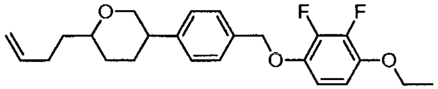 | 140 | 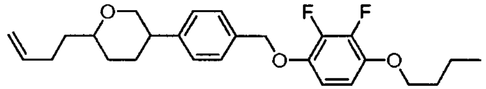 |
| 141 | 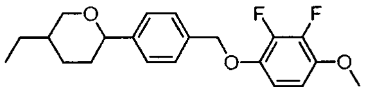 | 155 | 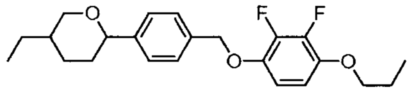 |
| 142 | 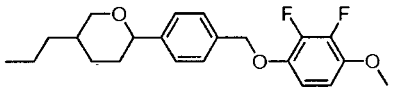 | 156 | 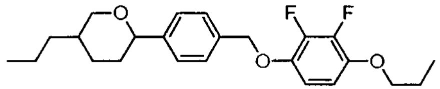 |
| 143 | 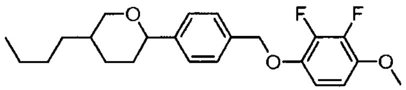 | 157 | 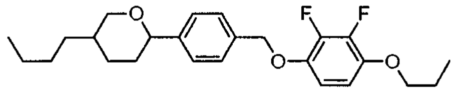 |
| 144 | 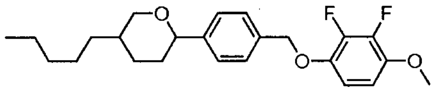 | 158 | 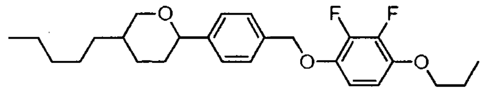 |
| 145 | 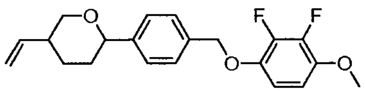 | 159 | 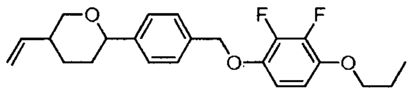 |
| 146 | 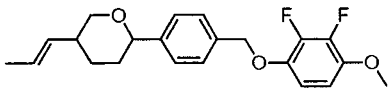 | 160 | 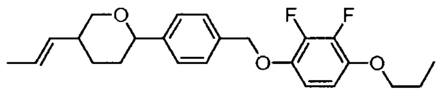 |
| 147 | 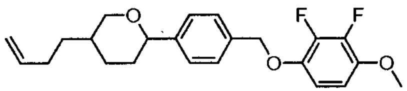 | 161 | 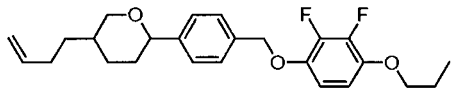 |

(continued)

| No. | | No. | |
|---|---|---|---|
| 148 | 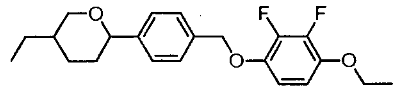 | 162 | 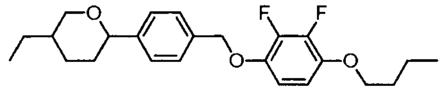 |
| 149 | 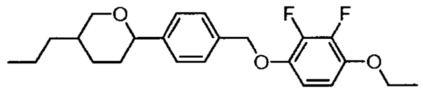 | 163 | 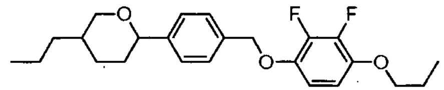 |
| 150 | 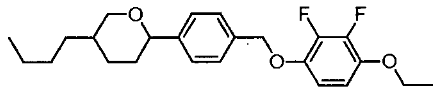 | 164 | 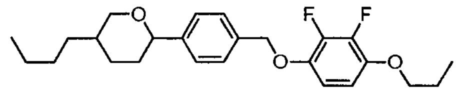 |
| 151 | 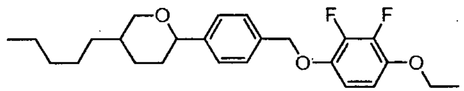 | 165 | 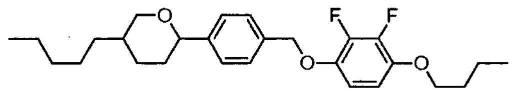 |
| 152 | 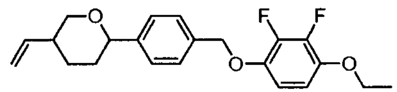 | 166 | 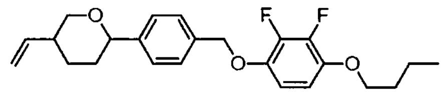 |
| 153 | 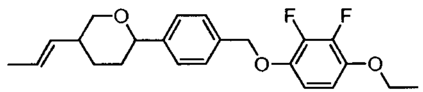 | 167 | 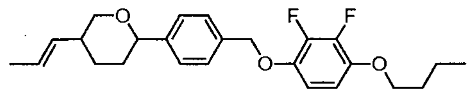 |
| 154 | 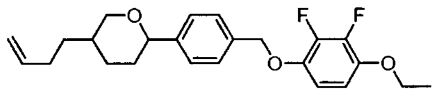 | 168 | 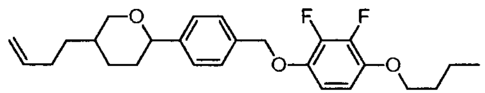 |
| 169 | 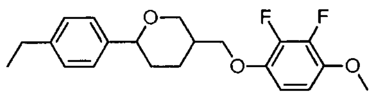 | 183 | 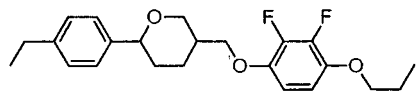 |
| 170 | 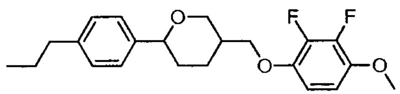 | 184 | 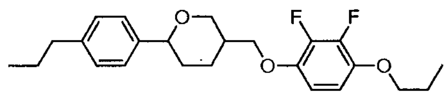 |
| 171 | 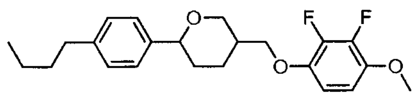 | 185 | 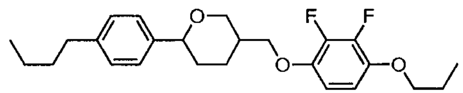 |

(continued)

| No. | | No. | |
|---|---|---|---|
| 172 | 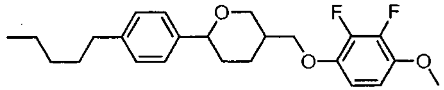 | 186 | 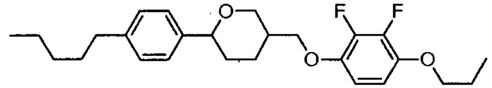 |
| 173 | 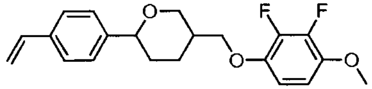 | 187 | 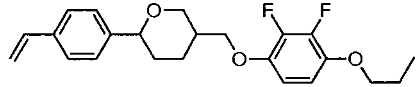 |
| 174 | 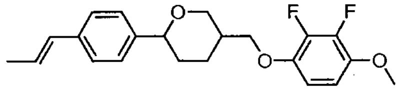 | 188 | 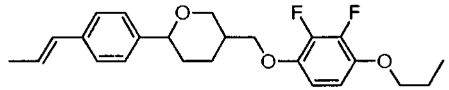 |
| 175 | 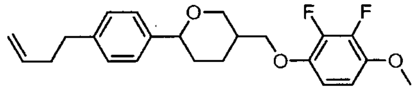 | 189 | 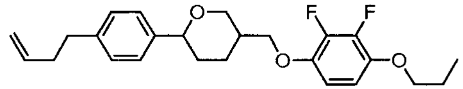 |
| 176 | 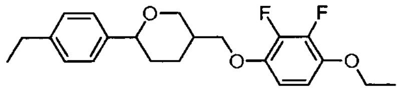 | 190 | 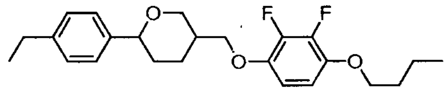 |
| 177 | 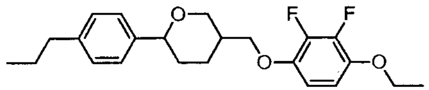 | 191 | 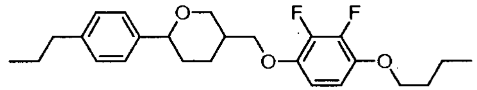 |
| 178 | 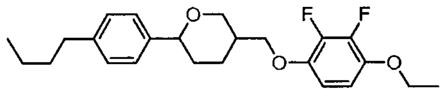 | 192 | 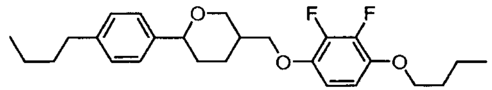 |
| 179 | 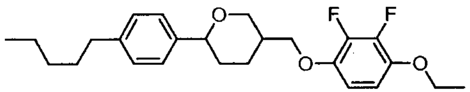 | 193 | 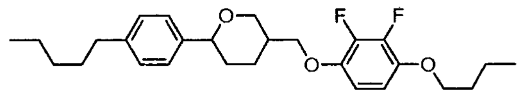 |
| 180 | 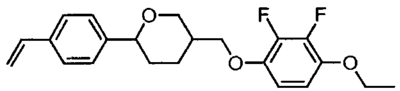 | 194 | 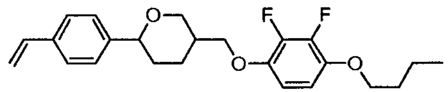 |
| 181 | 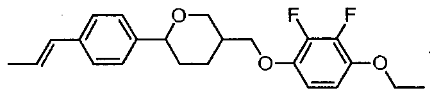 | 195 | 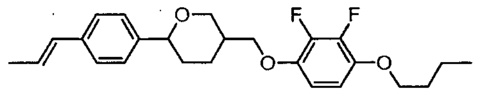 |
| 182 | 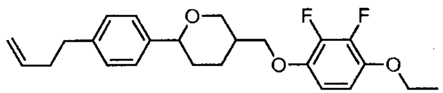 | 196 | 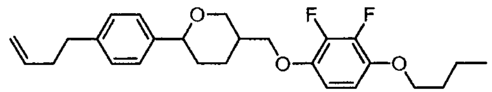 |

(continued)

| No. | | No. | |
|---|---|---|---|
| 197 | 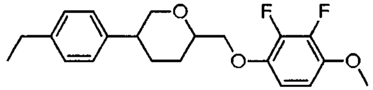 | 211 | 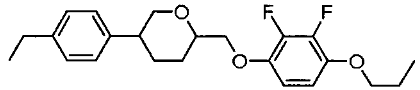 |
| 198 | 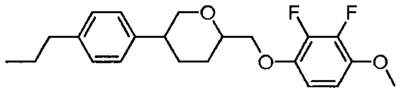 | 212 | 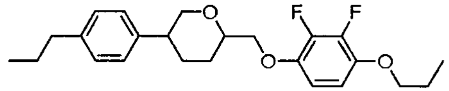 |
| 199 | 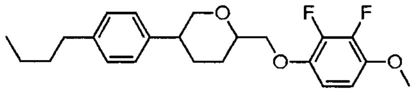 | 213 | 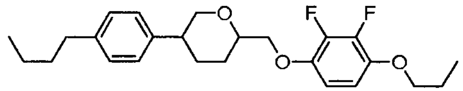 |
| 200 | 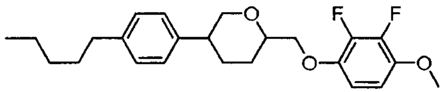 | 214 | 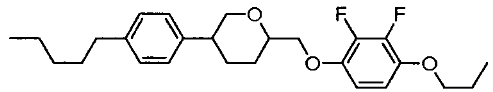 |
| 201 | 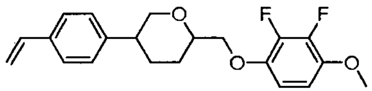 | 215 | 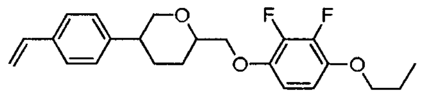 |
| 202 | 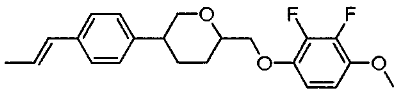 | 216 | 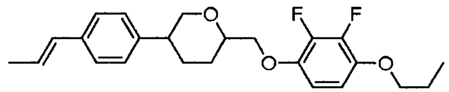 |
| 203 | 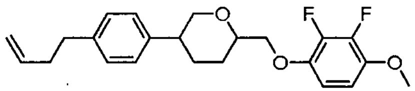 | 217 | 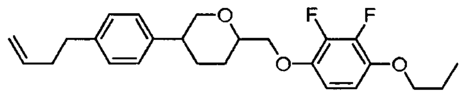 |
| 204 | 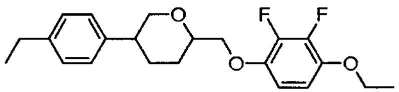 | 218 | 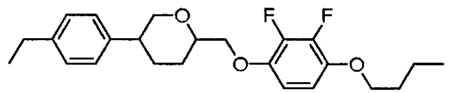 |
| 205 | 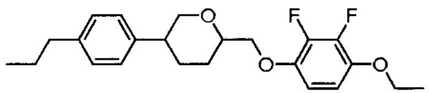 | 219 | 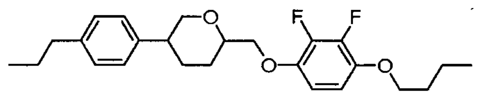 |
| 206 | 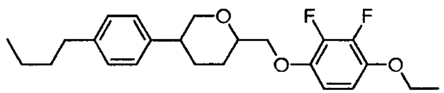 | 220 | 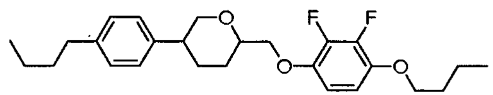 |
| 207 | 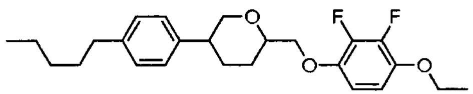 | 221 | 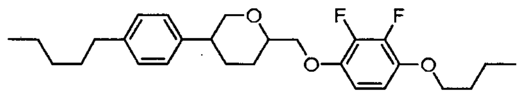 |

(continued)

| No. | | No. | |
|---|---|---|---|
| 208 | 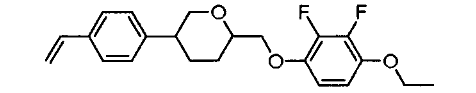 | 222 | 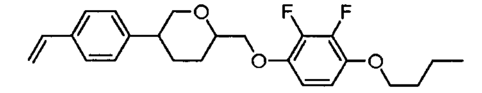 |
| 209 | 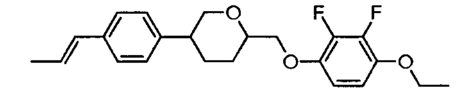 | 223 | 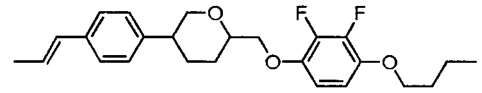 |
| 210 | 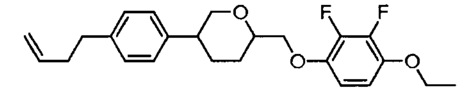 | 224 | 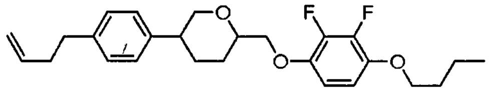 |
| 225 | 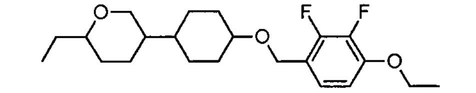 | 237 | 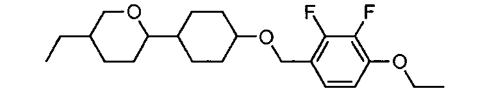 |
| 226 | 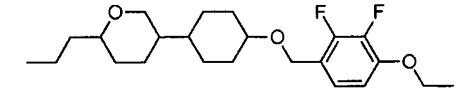 | 238 | 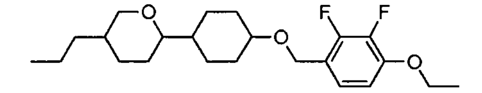 |
| 227 | 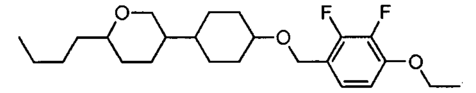 | 239 | 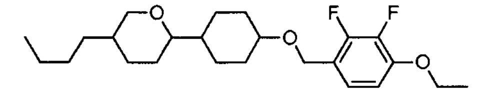 |
| 228 | 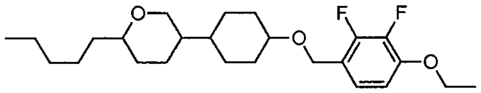 | 240 | 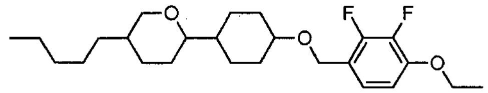 |
| 229 | 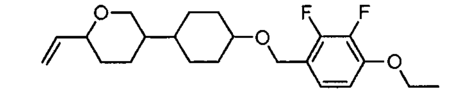 | 241 | 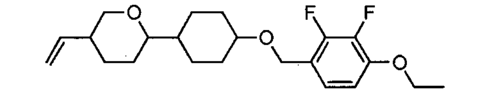 |
| 230 | 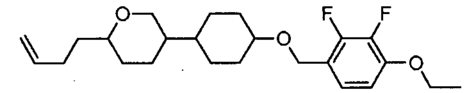 | 242 | 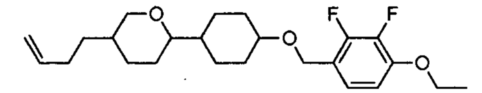 |
| 231 | 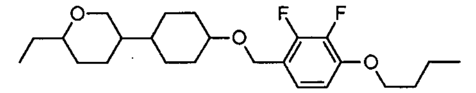 | 243 | 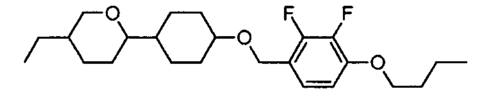 |
| 232 | 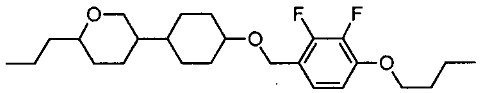 | 244 | 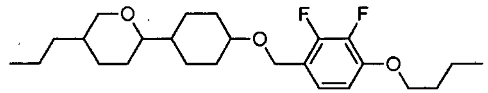 |

(continued)

| No. | | No. | |
|---|---|---|---|
| 233 | 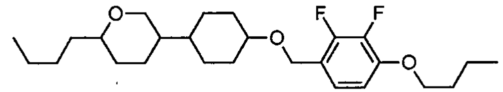 | 245 | 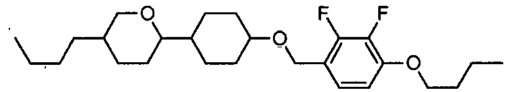 |
| 234 | 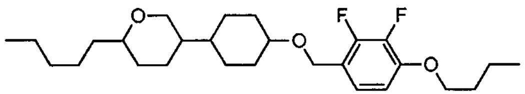 | 246 | 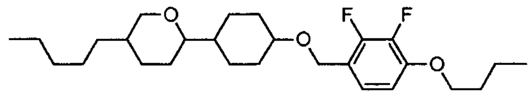 |
| 235 | 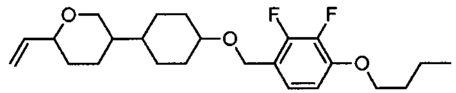 | 247 | 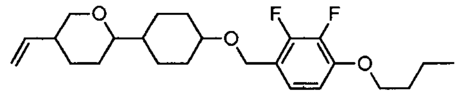 |
| 236 | 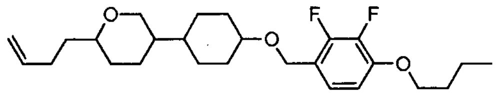 | 248 | 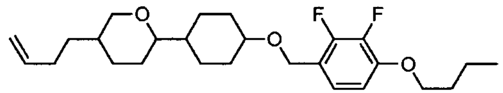 |
| 249 | 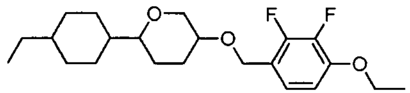 | 251 | 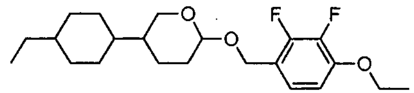 |
| 250 | 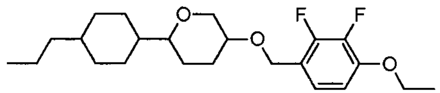 | 252 | 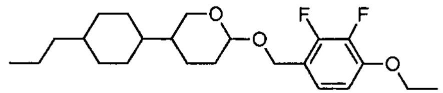 |
| 251 | 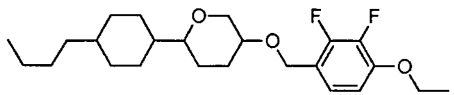 | 253 | 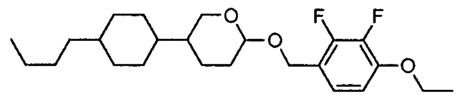 |
| 252 | 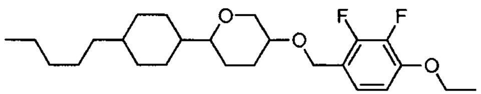 | 254 | 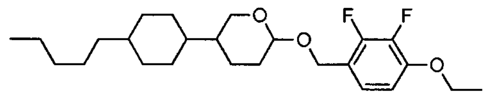 |
| 253 | 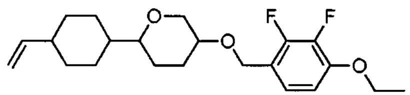 | 255 | 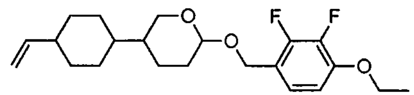 |
| 254 | 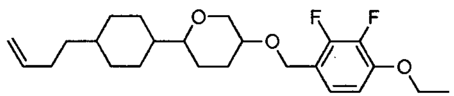 | 256 | 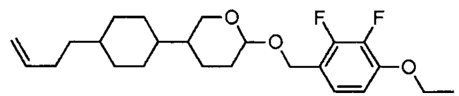 |
| 255 | 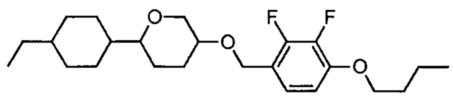 | 257 | 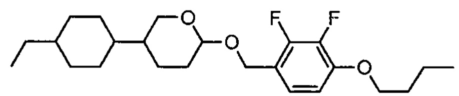 |

(continued)

| No. | | No. | |
|---|---|---|---|
| 256 | 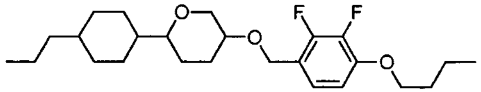 | 258 | 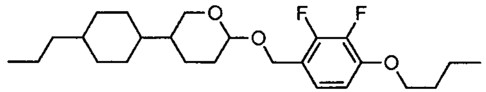 |
| 257 | 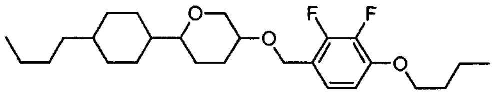 | 259 | 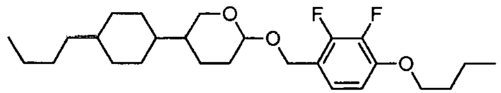 |
| 258 | 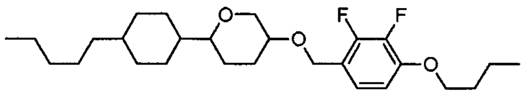 | 260 | 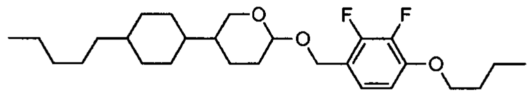 |
| 259 | 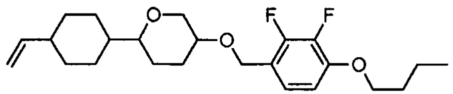 | 261 | 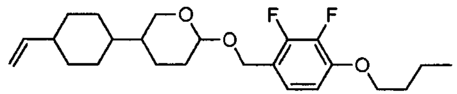 |
| 260 | 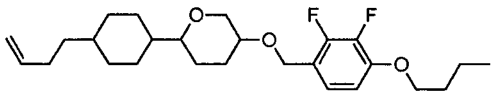 | 262 | 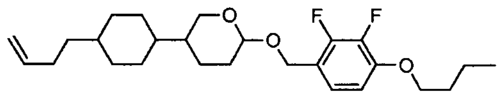 |
| 263 | 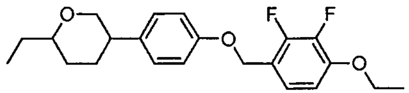 | 275 | 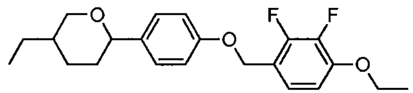 |
| 264 | 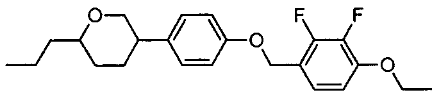 | 276 | 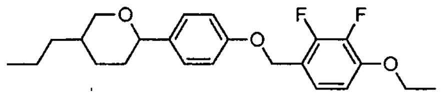 |
| 265 | 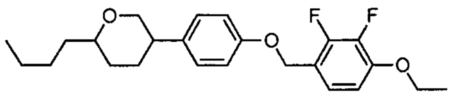 | 277 | 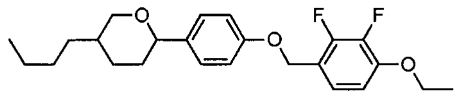 |
| 266 | 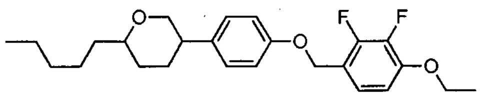 | 278 | 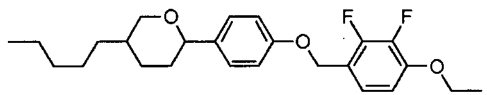 |
| 267 | 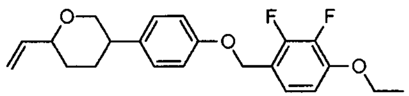 | 279 | 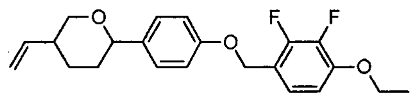 |
| 268 | 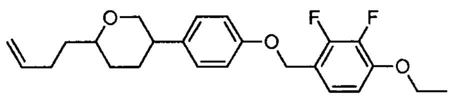 | 280 | 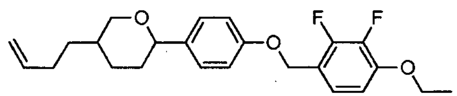 |

(continued)

| No. | | No. | |
|---|---|---|---|
| 269 | 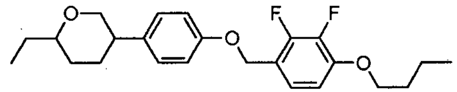 | 281 | 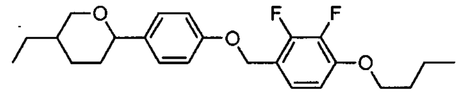 |
| 270 | 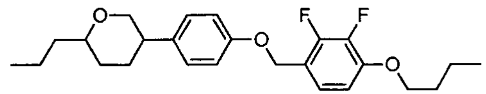 | 282 | 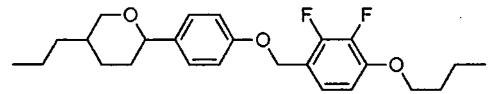 |
| 271 | 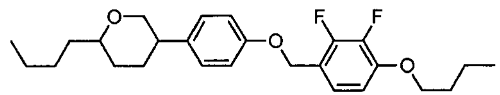 | 283 | 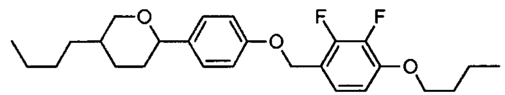 |
| 272 | 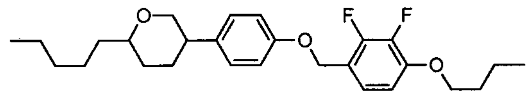 | 284 | 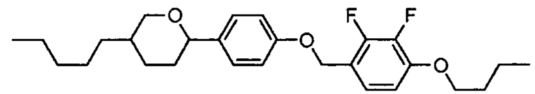 |
| 273 | 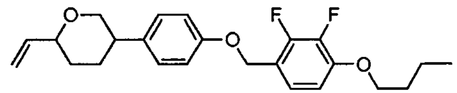 | 285 | 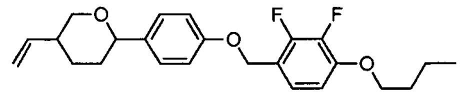 |
| 274 | 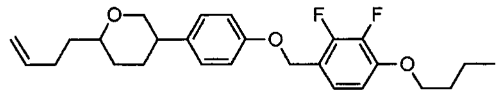 | 28E | 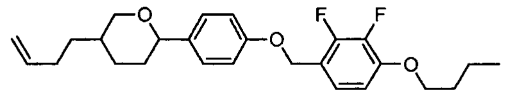 |
| 287 | 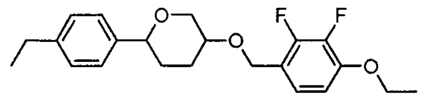 | 299 | 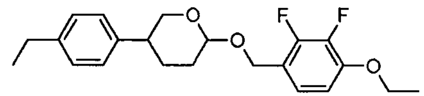 |
| 288 | 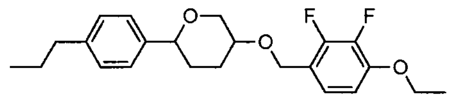 | 300 | 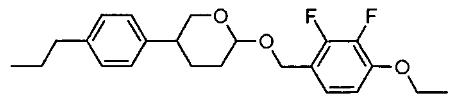 |
| 289 | 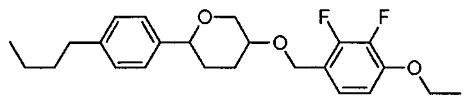 | 301 | 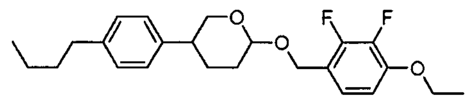 |
| 290 | 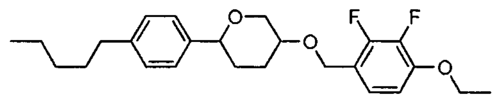 | 302 | 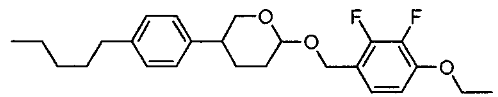 |
| 291 | 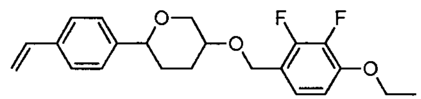 | 303 | 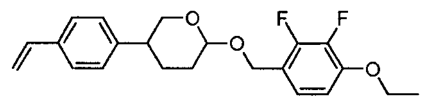 |

(continued)

| No. | | No. | |
|---|---|---|---|
| 292 | 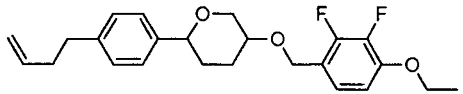 | 304 | 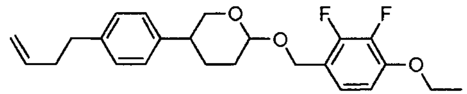 |
| 293 | 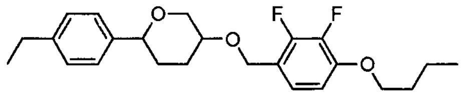 | 305 | 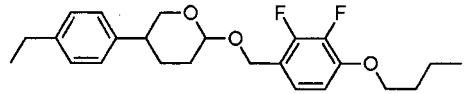 |
| 294 | 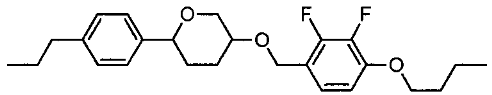 | 306 | 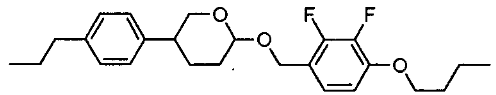 |
| 295 | 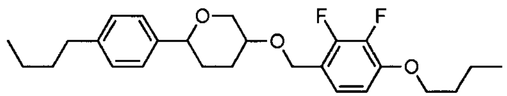 | 307 | 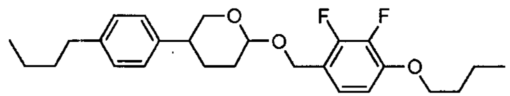 |
| 296 | 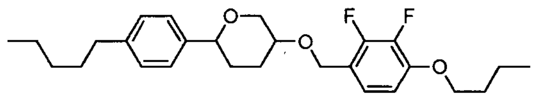 | 308 | 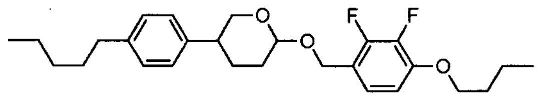 |
| 297 | 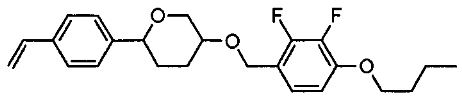 | 309 | 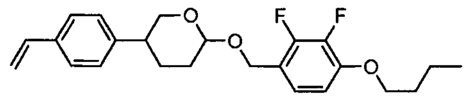 |
| 298 | 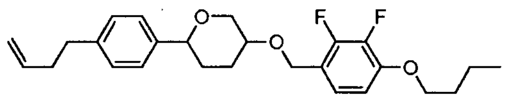 | 310 | 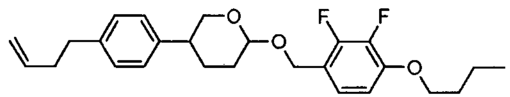 |
| 311 | 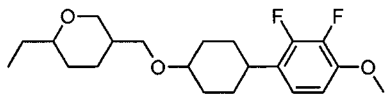 | 325 | 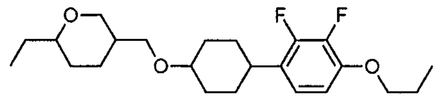 |
| 312 | 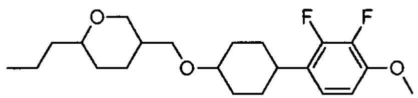 | 326 | 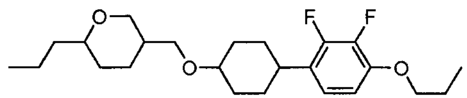 |
| 313 | 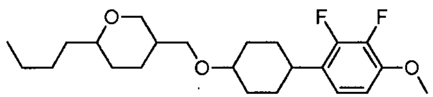 | 327 | 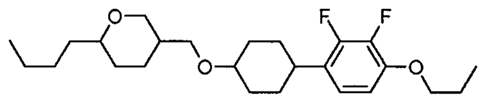 |

(continued)

| No. | | No. | |
|---|---|---|---|
| 314 | 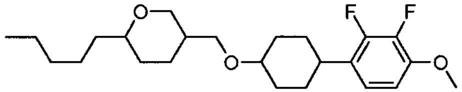 | 328 | 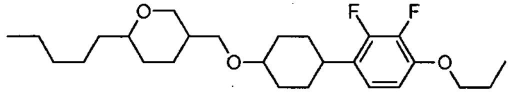 |
| 315 | 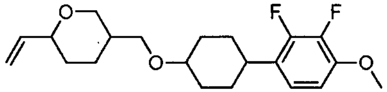 | 329 | 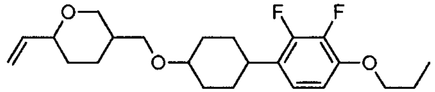 |
| 316 | 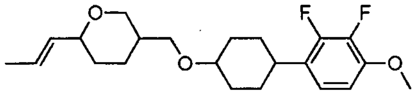 | 330 | 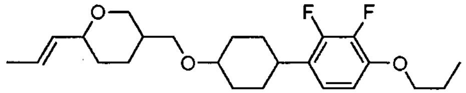 |
| 317 | 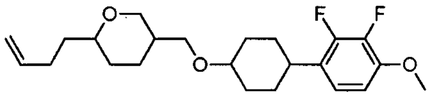 | 331 | 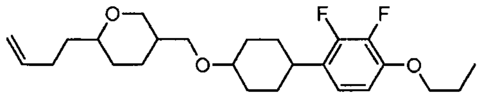 |
| 318 | 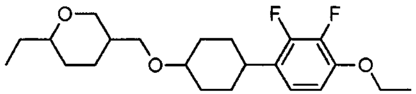 | 332 | 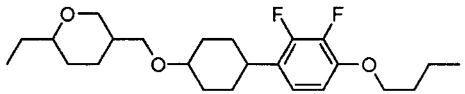 |
| 319 | 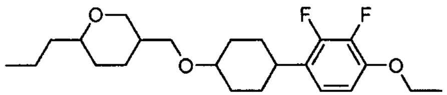 | 333 | 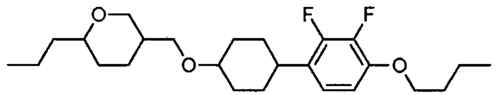 |
| 320 | 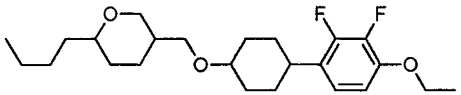 | 334 | 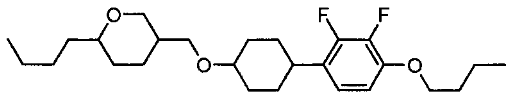 |
| 321 | 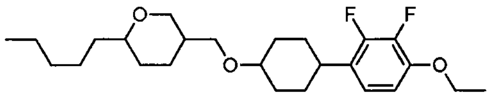 | 335 | 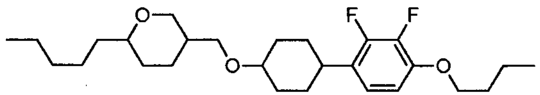 |
| 322 | 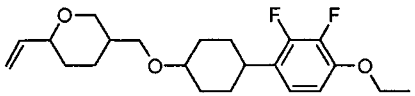 | 336 | 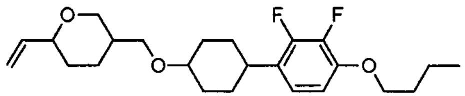 |
| 323 | 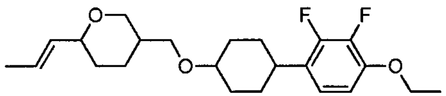 | 337 | 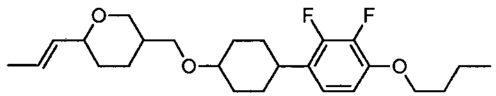 |

(continued)

| No. | | No. | |
|---|---|---|---|
| 324 | 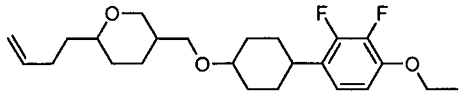 | 338 | 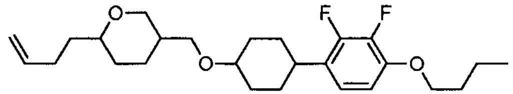 |
| 339 | 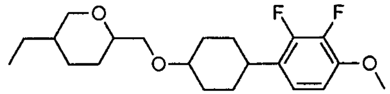 | 353 | 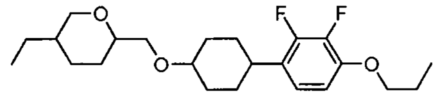 |
| 340 | 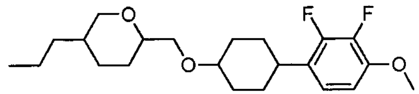 | 354 | 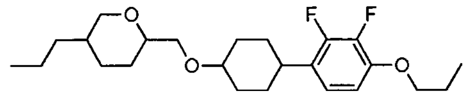 |
| 341 | 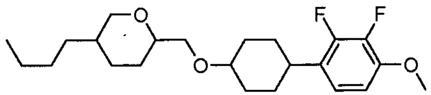 | 355 | 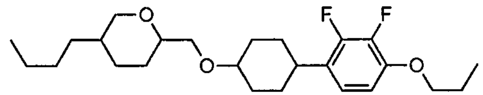 |
| 342 | 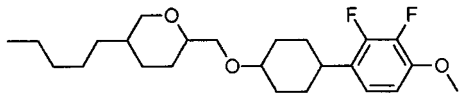 | 356 | 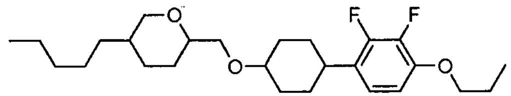 |
| 343 | 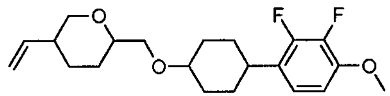 | 357 | 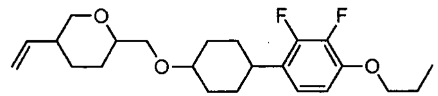 |
| 344 | 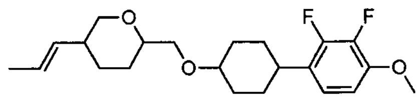 | 358 | 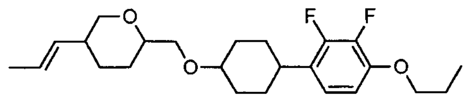 |
| 345 | 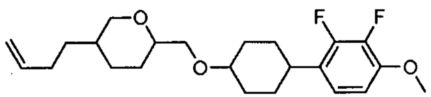 | 359 | 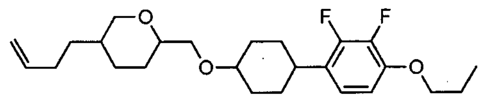 |
| 346 | 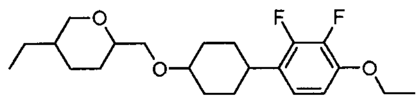 | 360 | 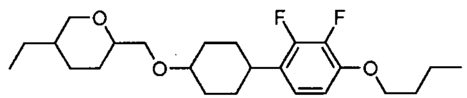 |
| 347 | 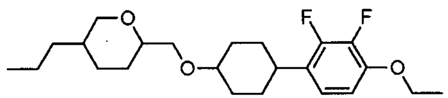 | 361 | 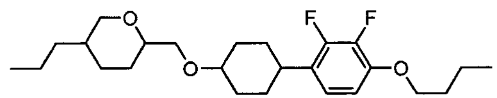 |

(continued)

| No. | | No. | |
|---|---|---|---|
| 348 | 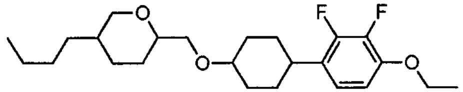 | 362 | 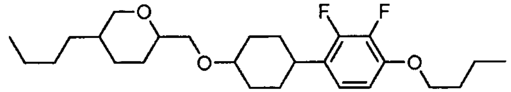 |
| 349 | 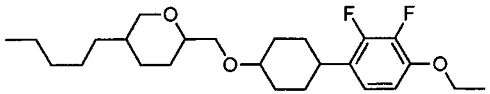 | 363 | 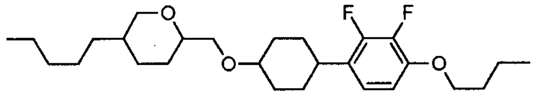 |
| 350 | 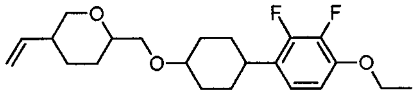 | 364 | 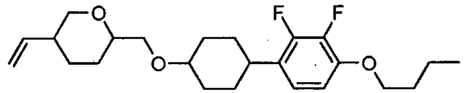 |
| 351 | 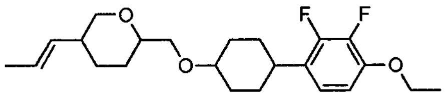 | 365 | 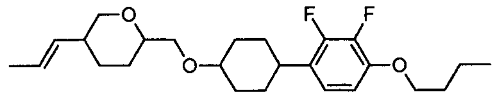 |
| 352 | 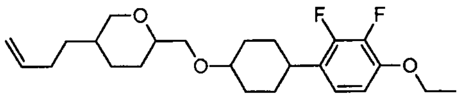 | 366 | 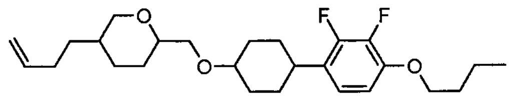 |
| 367 | 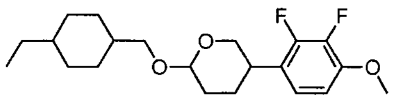 | 381 | 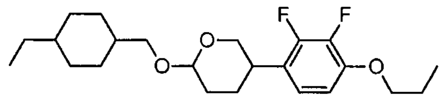 |
| 368 | 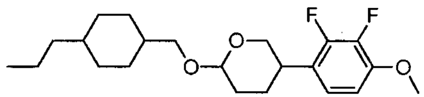 | 382 | 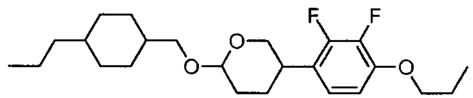 |
| 369 | 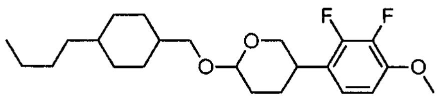 | 383 | 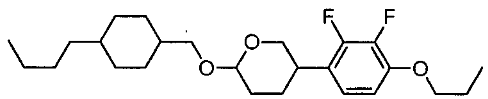 |
| 370 | 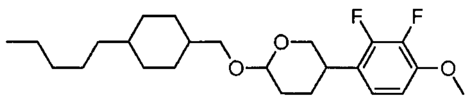 | 384 | 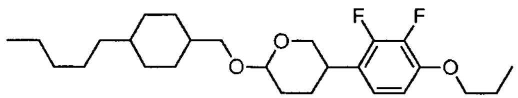 |
| 371 | 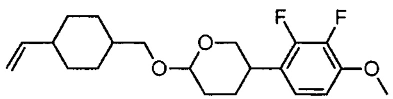 | 385 | 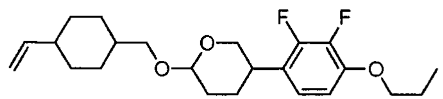 |

(continued)

| No. | | No. | |
|---|---|---|---|
| 372 | 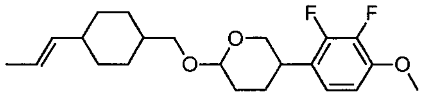 | 386 | 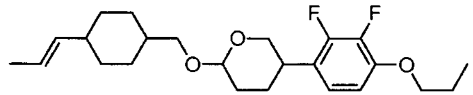 |
| 373 | 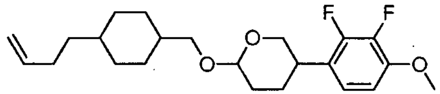 | 387 | 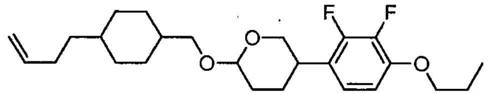 |
| 374 | 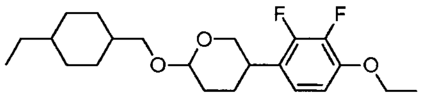 | 388 | 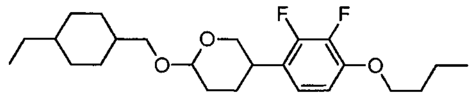 |
| 375 | 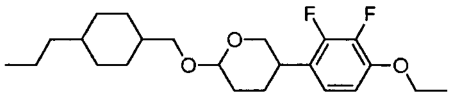 | 389 | 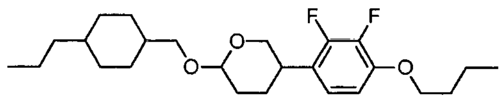 |
| 376 | 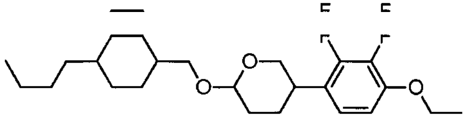 | 390 | 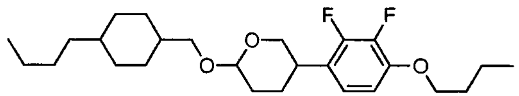 |
| 377 | 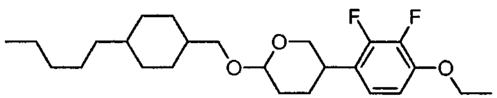 | 391 | 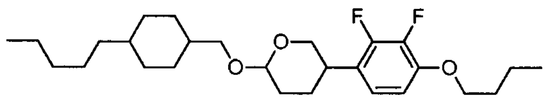 |
| 378 | 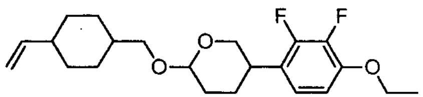 | 392 | 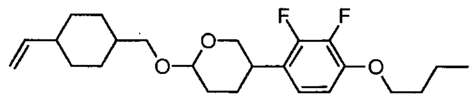 |
| 379 | 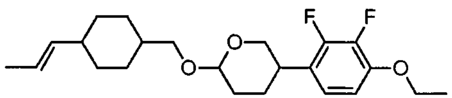 | 393 | 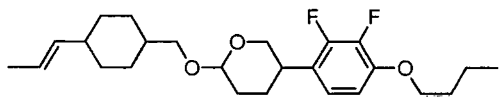 |
| 380 | 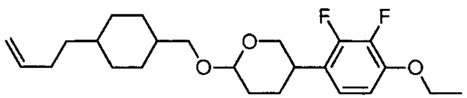 | 394 | 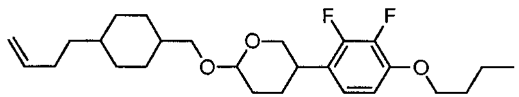 |
| 395 | 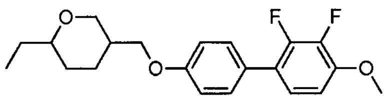 | 409 | 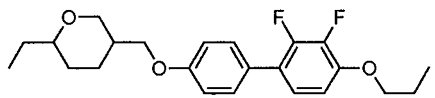 |

(continued)

| No. | | No. | |
|---|---|---|---|
| 396 | 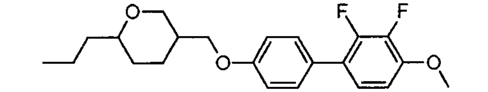 | 410 | 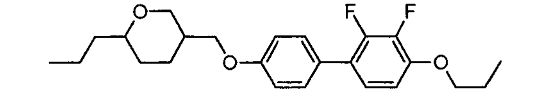 |
| 397 | 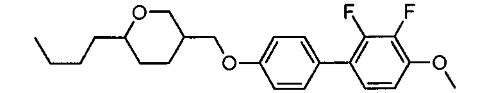 | 411 | 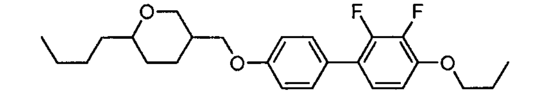 |
| 398 | 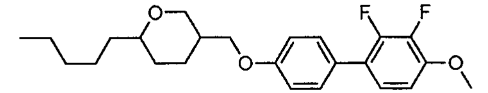 | 412 | 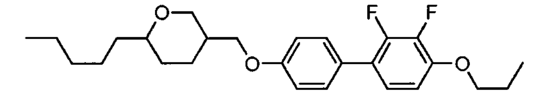 |
| 399 | 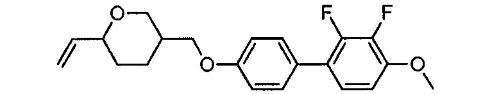 | 413 | 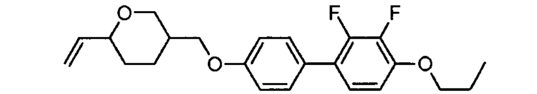 |
| 400 | 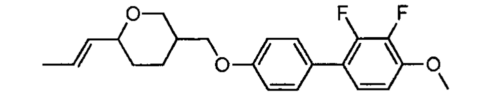 | 414 | 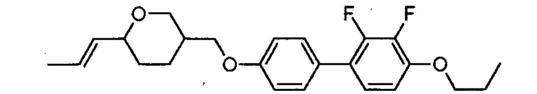 |
| 401 | 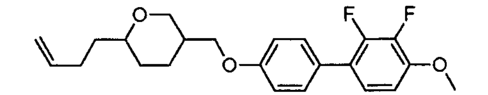 | 415 | 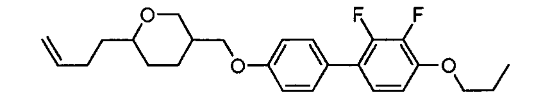 |
| 402 | 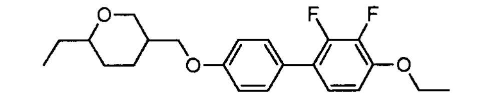 | 416 | 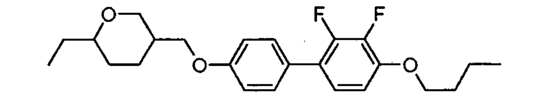 |
| 403 | 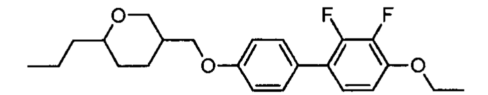 | 417 | 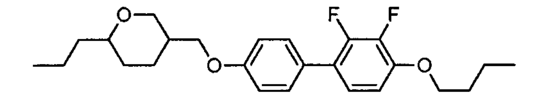 |
| 404 | 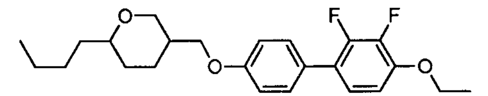 | 418 | 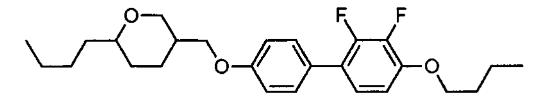 |
| 405 | 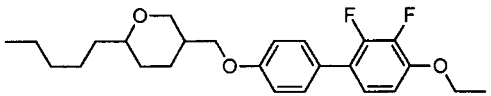 | 419 | 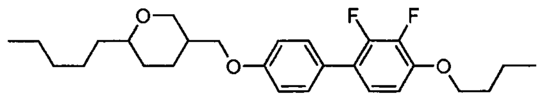 |

(continued)

| No. | | No. | |
|---|---|---|---|
| 406 | 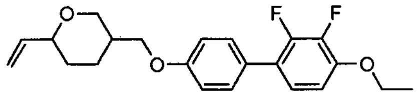 | 420 | 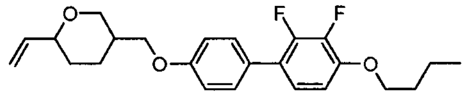 |
| 407 | 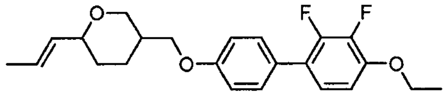 | 421 | 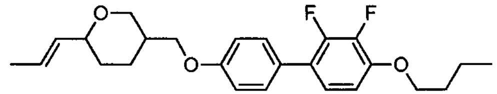 |
| 408 | 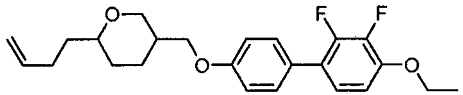 | 422 | 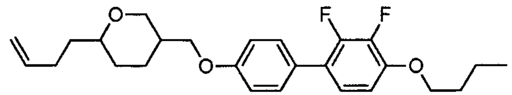 |
| 423 | 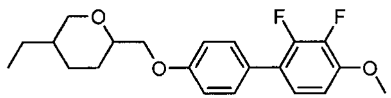 | 437 | 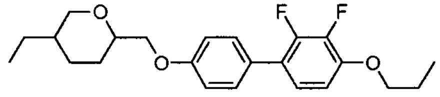 |
| 424 | 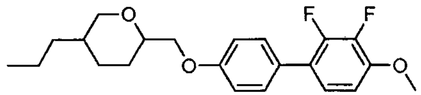 | 438 | 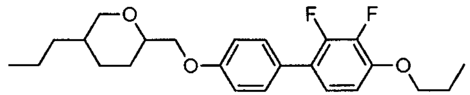 |
| 425 | 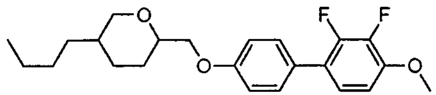 | 439 | 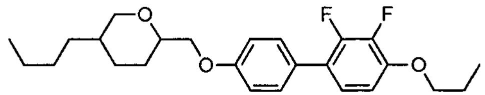 |
| 426 | 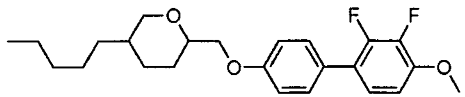 | 440 | 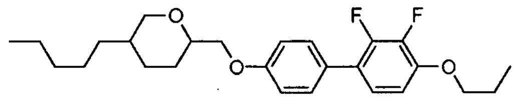 |
| 427 | 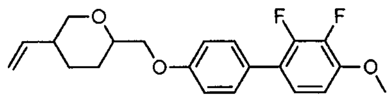 | 441 | 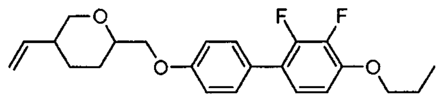 |
| 428 | 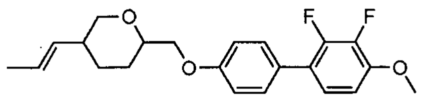 | 442 | 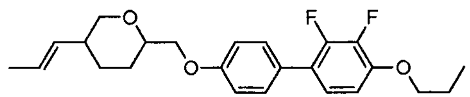 |
| 429 | 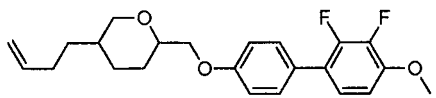 | 443 | 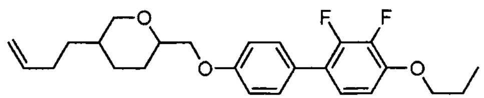 |

(continued)

| No. | | No. | |
|---|---|---|---|
| 430 | 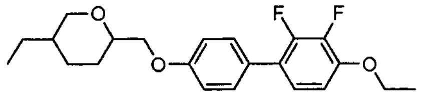 | 444 | 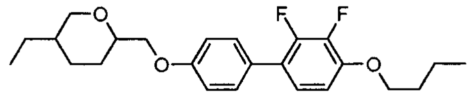 |
| 431 | 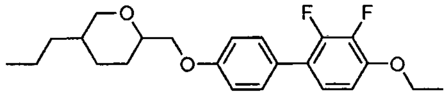 | 445 | 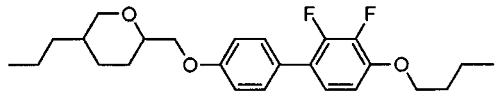 |
| 432 | 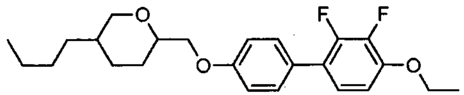 | 446 | 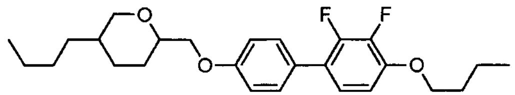 |
| 433 | 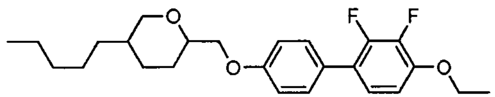 | 447 | 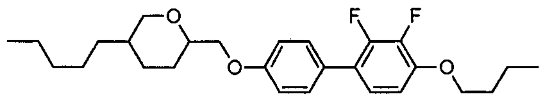 |
| 434 | 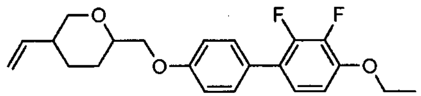 | 448 | 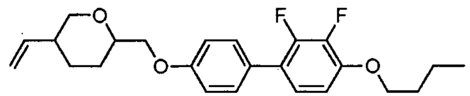 |
| 435 | 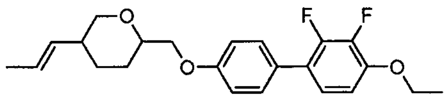 | 449 | 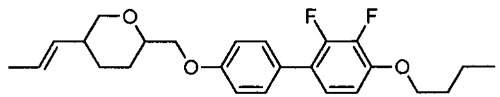 |
| 436 | 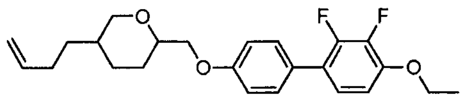 | 450 | 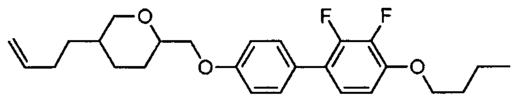 |
| 451 | 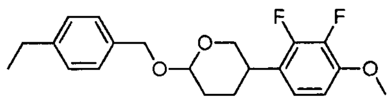 | 465 | 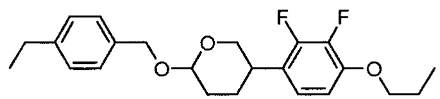 |
| 452 | 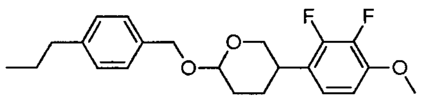 | 466 | 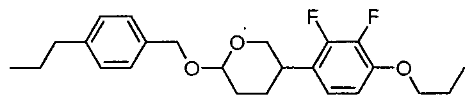 |
| 453 | 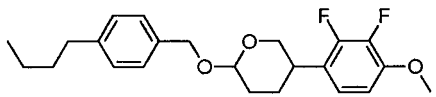 | 467 | 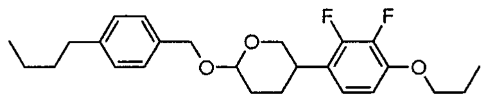 |

(continued)

| No. | | No. | |
|---|---|---|---|
| 454 | 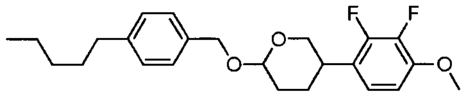 | 468 | 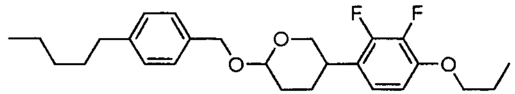 |
| 455 | 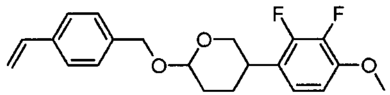 | 469 | 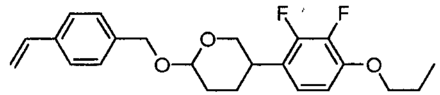 |
| 456 | 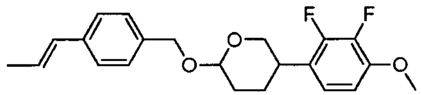 | 460 | 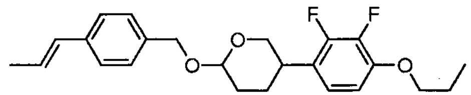 |
| 457 | 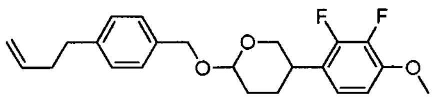 | 461 | 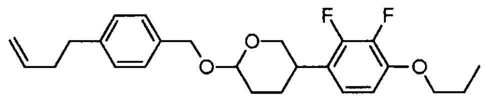 |
| 458 | 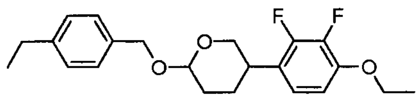 | 462 | 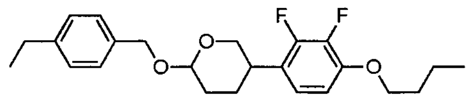 |
| 459 | 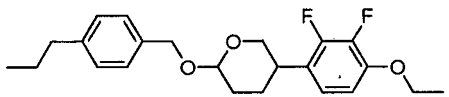 | 463 | 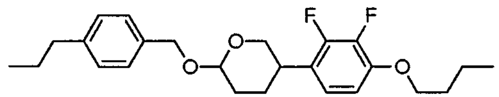 |
| 460 | 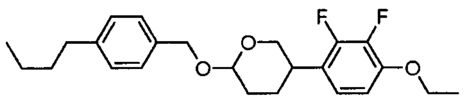 | 464 | 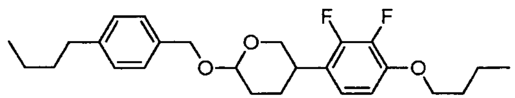 |
| 461 | 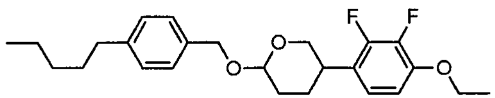 | 465 | 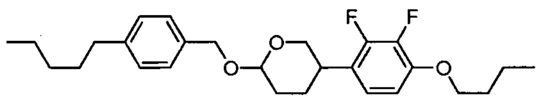 |
| 462 | 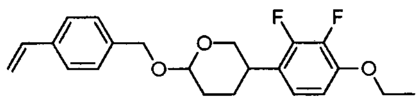 | 466 | 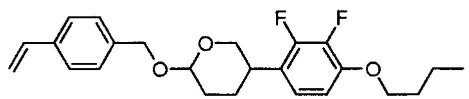 |
| 463 | 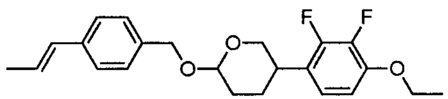 | 467 | 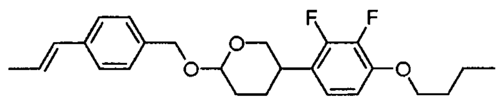 |

(continued)

| No. | | No. | |
|---|---|---|---|
| 464 | 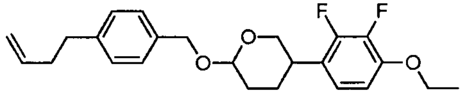 | 468 | 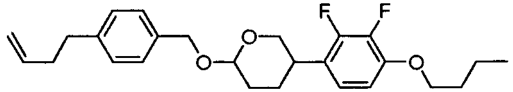 |
| 469 | 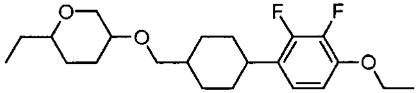 | 481 | 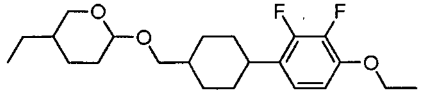 |
| 470 | 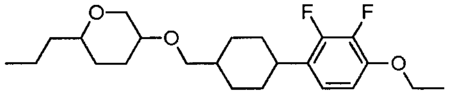 | 482 | 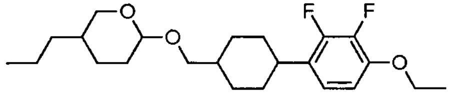 |
| 471 | 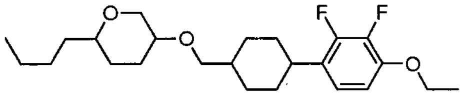 | 483 | 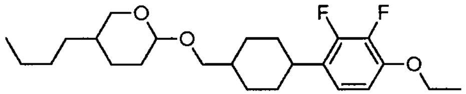 |
| 472 | 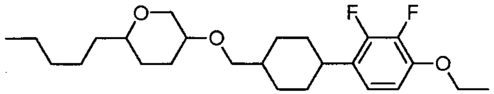 | 484 | 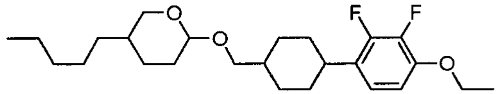 |
| 473 | 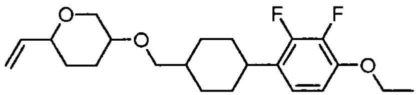 | 485 | 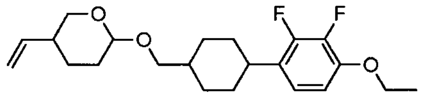 |
| 474 | 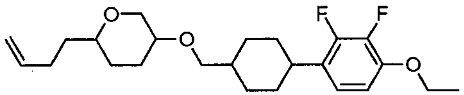 | 486 | 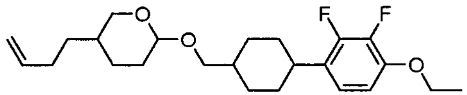 |
| 475 | 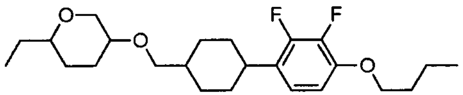 | 487 | 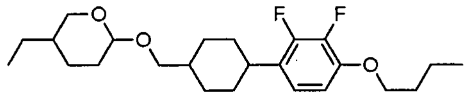 |
| 476 | 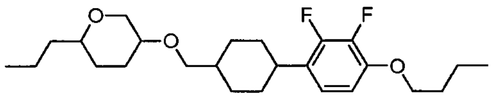 | 488 | 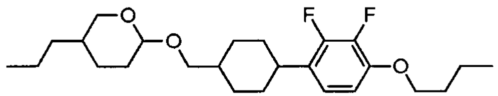 |
| 477 | 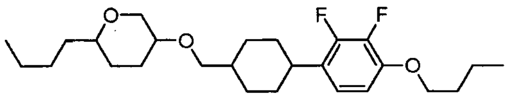 | 489 | 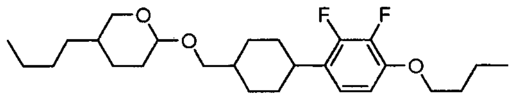 |

(continued)

| No. | | No. | |
|---|---|---|---|
| 478 | 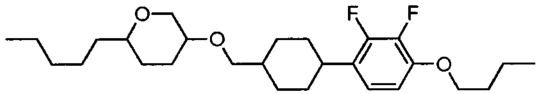 | 490 | 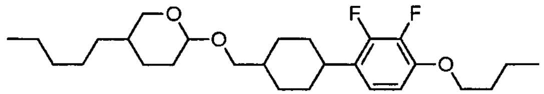 |
| 479 | 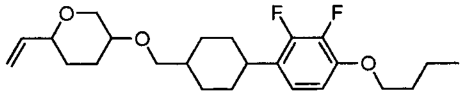 | 491 | 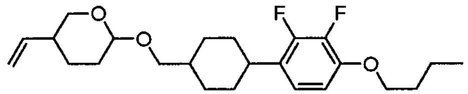 |
| 480 | 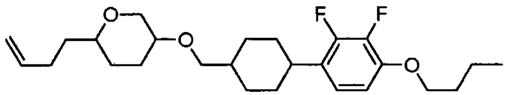 | 492 | 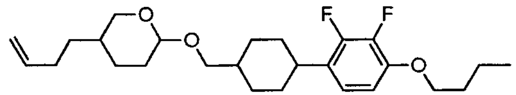 |
| 493 | 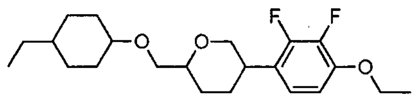 | 505 | 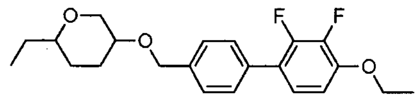 |
| 494 | 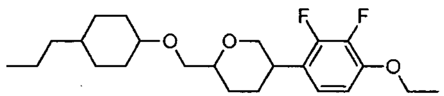 | 506 | 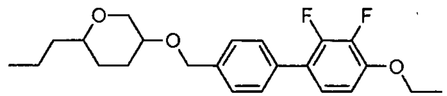 |
| 495 | 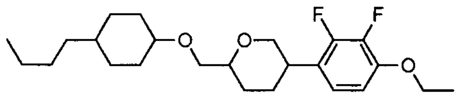 | 507 | 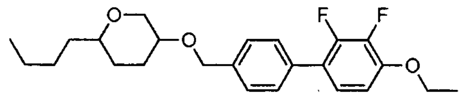 |
| 496 | 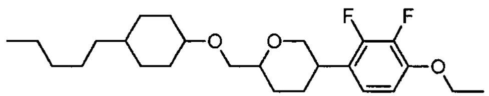 | 508 | 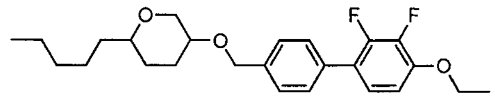 |
| 497 | 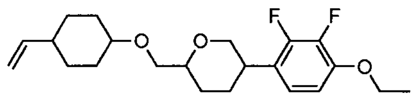 | 509 | 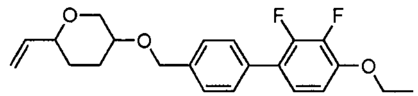 |
| 498 | 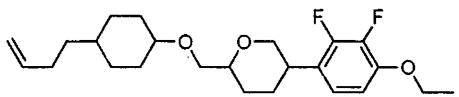 | 510 | 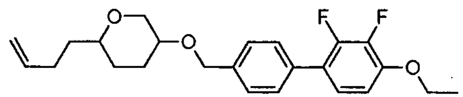 |
| 499 | 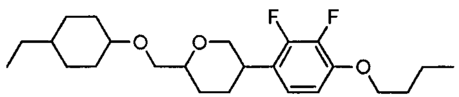 | 511 | 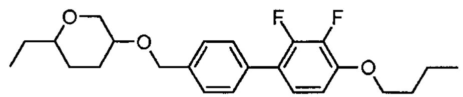 |

(continued)

| No. | | No. | |
|---|---|---|---|
| 500 | 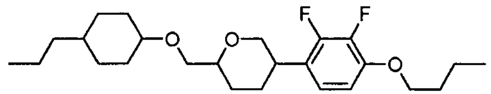 | 512 | 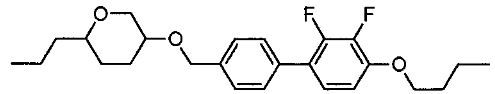 |
| 501 | 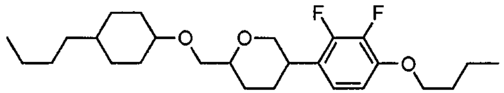 | 513 | 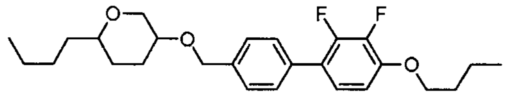 |
| 502 | 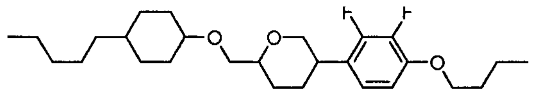 | 514 | 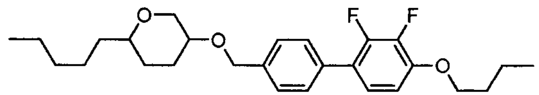 |
| 503 | 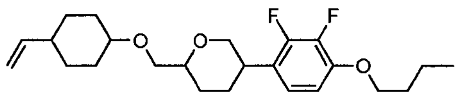 | 515 | 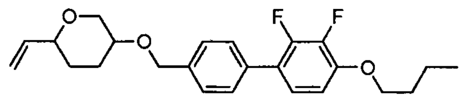 |
| 504 | 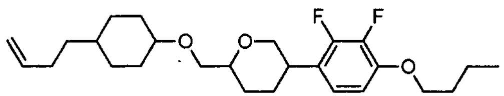 | 516 | 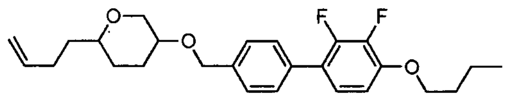 |
| 517 | 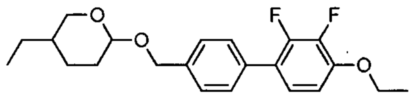 | 529 | 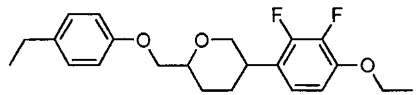 |
| 518 | 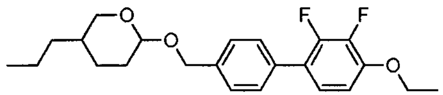 | 530 | 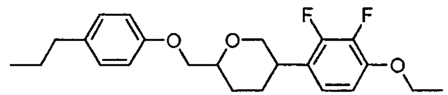 |
| 519 | 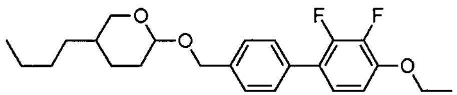 | 531 | 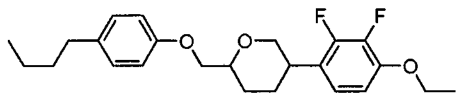 |
| 520 | 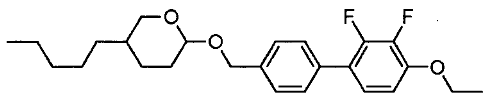 | 532 | 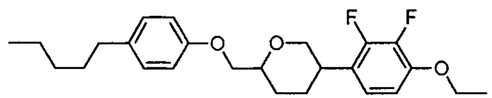 |
| 521 | 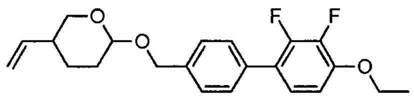 | 533 | 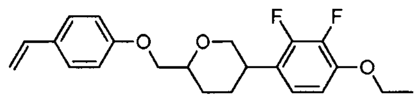 |

(continued)

| No. | | No. | |
|---|---|---|---|
| 522 | 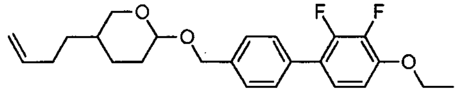 | 534 | 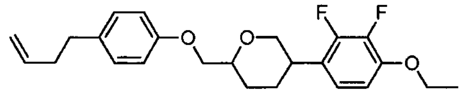 |
| 523 | 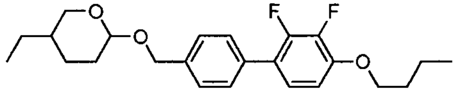 | 535 | 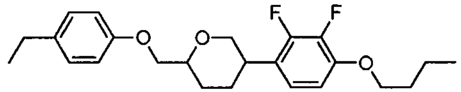 |
| 524 | 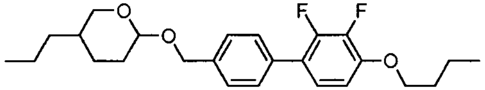 | 536 | 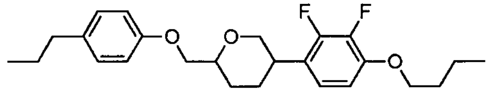 |
| 525 | 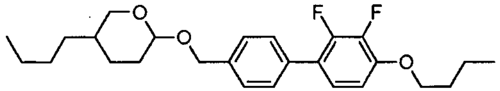 | 537 | 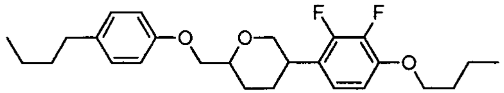 |
| 526 | 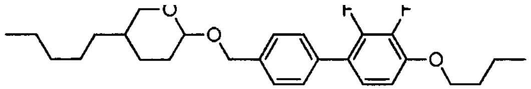 | 538 | 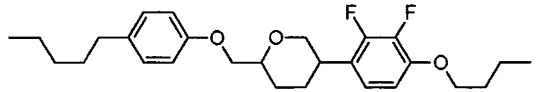 |
| 527 | 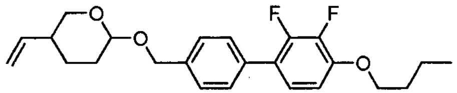 | 539 | 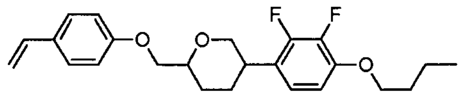 |
| 528 | 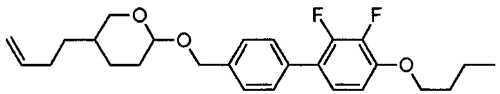 | 540 | 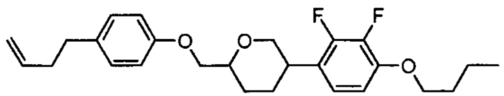 |
| 541 | 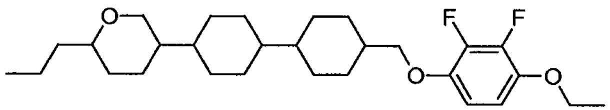 | | |
| 542 | 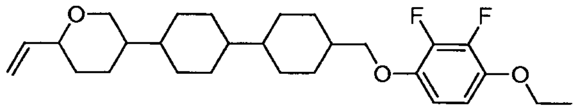 | | |
| 543 | 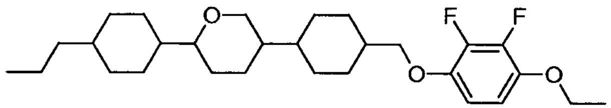 | | |

(continued)

| No. | | No. | |
|---|---|---|---|
| 544 | 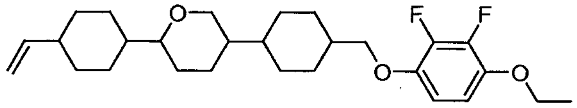 | | |
| 545 | 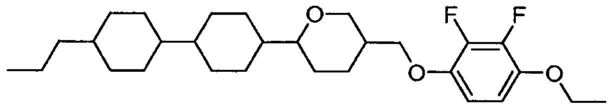 | | |
| 546 | 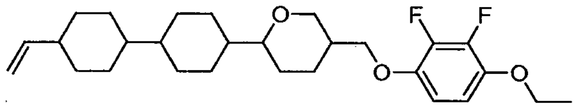 | | |
| 547 | 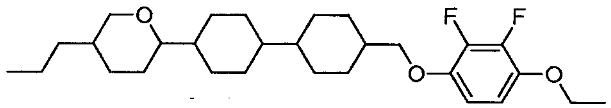 | | |
| 548 | 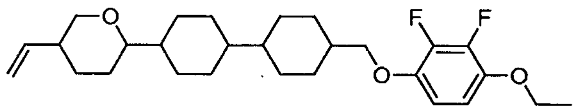 | | |
| 549 | 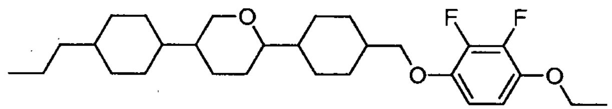 | | |
| 550 | 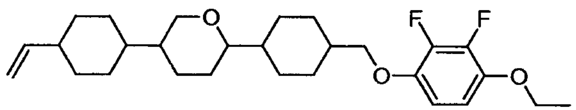 | | |
| 551 | 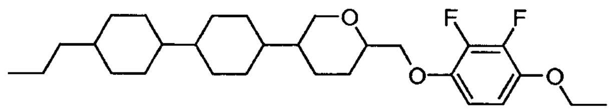 | | |
| 552 | 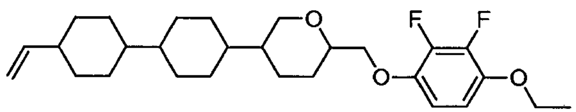 | | |

(continued)

| No. | | No. |
|---|---|---|
| 553 | 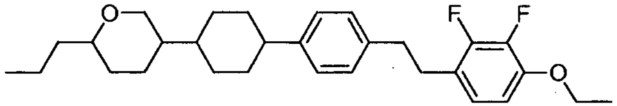 | |
| 554 | 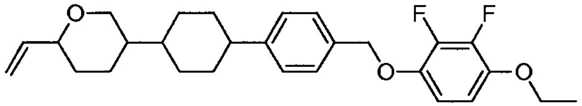 | |
| 555 | 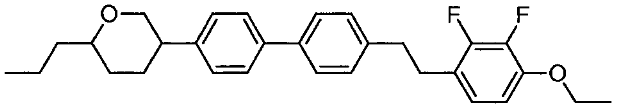 | |
| 556 | 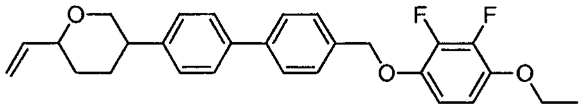 | |
| 557 | 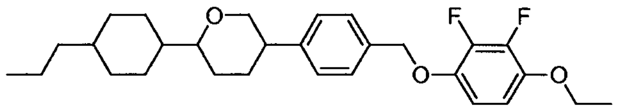 | |
| 558 | 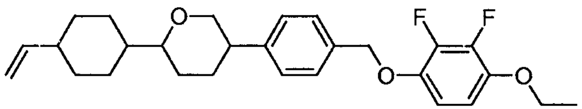 | |
| 559 | 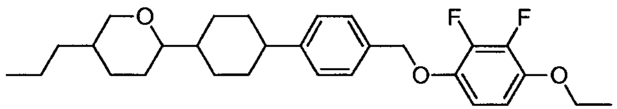 | |
| 560 | 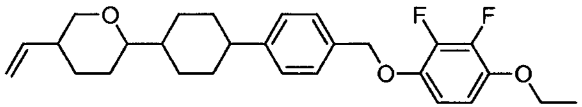 | |
| 561 | 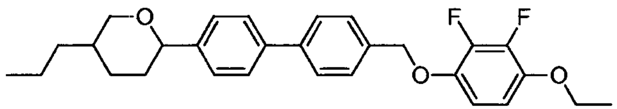 | |

(continued)

| No. | | No. |
|---|---|---|
| 562 | 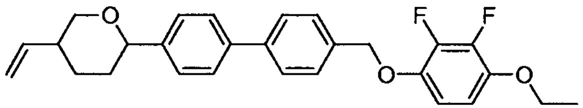 | |
| 563 | 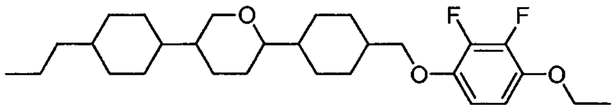 | |
| 564 | 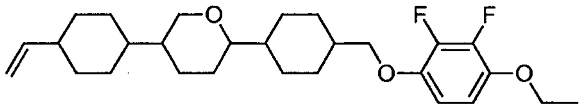 | |
| 565 | 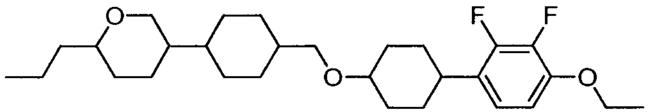 | |
| 566 | 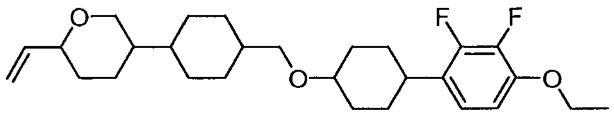 | |
| 567 | 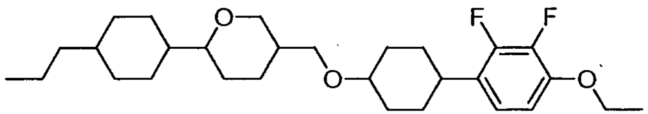 | |
| 568 | 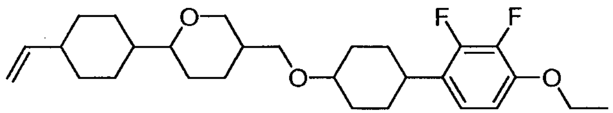 | |
| 569 | 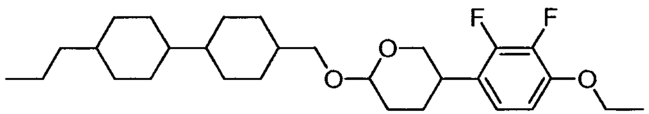 | |
| 570 | 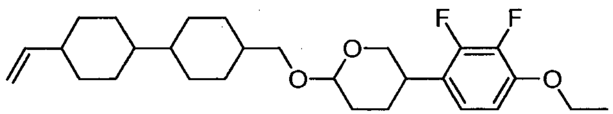 | |

(continued)

| No. | |
|---|---|
| 571 | 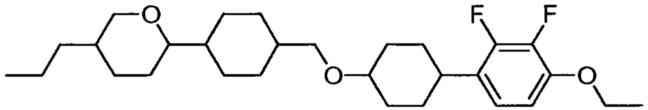 |
| 572 | 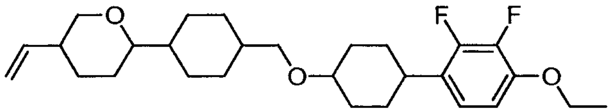 |
| 573 | 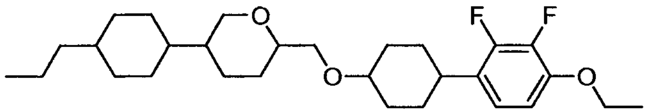 |
| 574 | 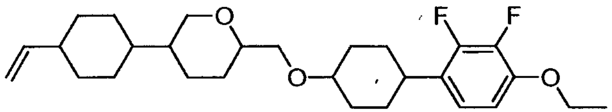 |
| 575 | 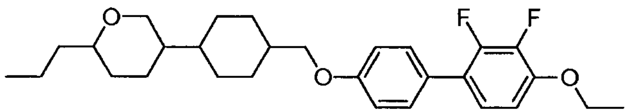 |
| 576 | 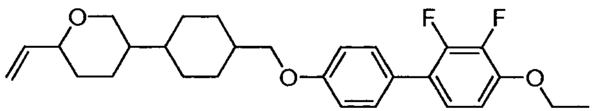 |
| 577 | 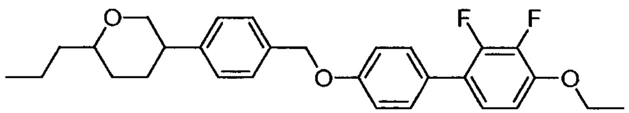 |
| 578 | 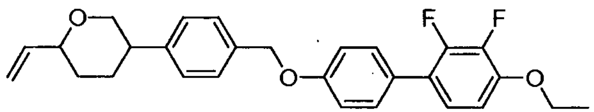 |
| 579 | 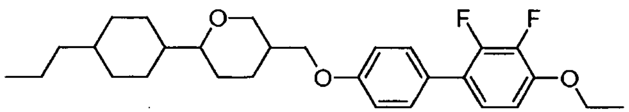 |

(continued)

| No. | No. |
|---|---|
| 580 | 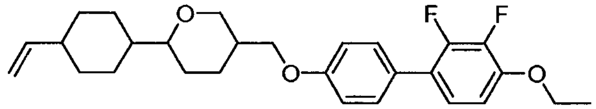 |
| 581 | 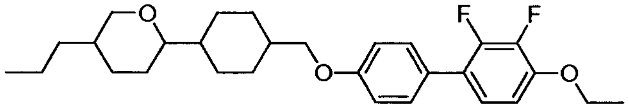 |
| 582 | 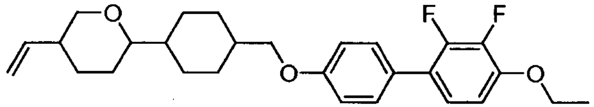 |
| 583 | 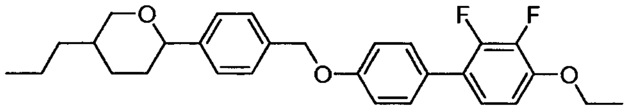 |
| 574 | 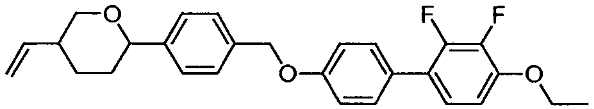 |
| 585 | 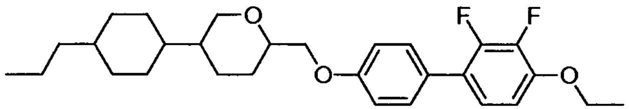 |
| 586 | 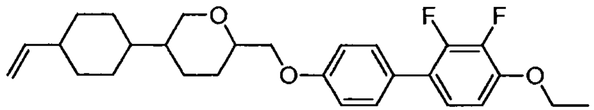 |
| 587 | 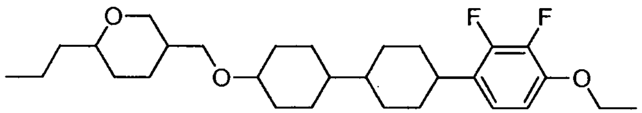 |
| 588 | 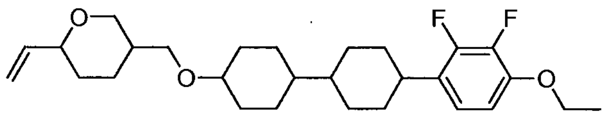 |

(continued)

| No. | |
|---|---|
| 589 | 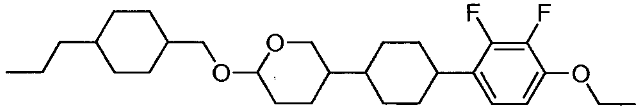 |
| 590 | 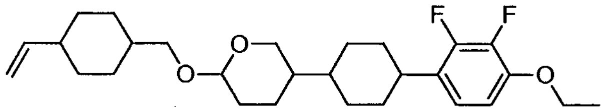 |
| 591 | 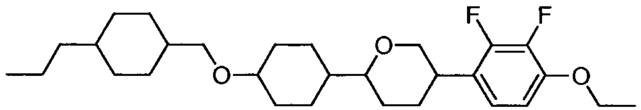 |
| 592 | 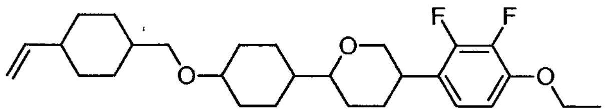 |
| 593 | 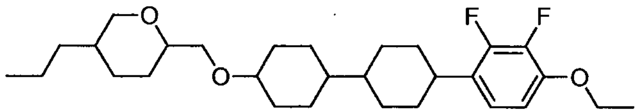 |
| 594 | 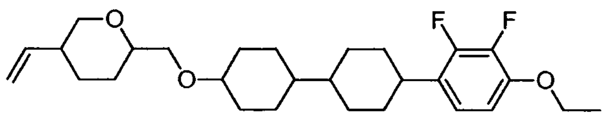 |
| 595 | 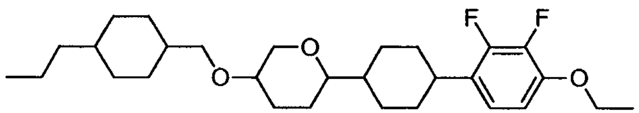 |
| 596 | 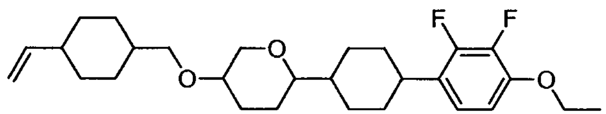 |
| 597 | 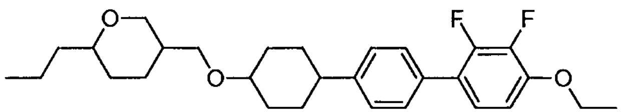 |

(continued)

| No. | No. |
|---|---|
| 598 | 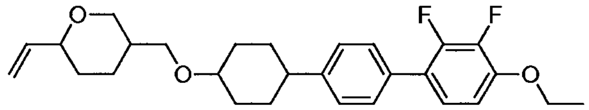 |
| 599 | 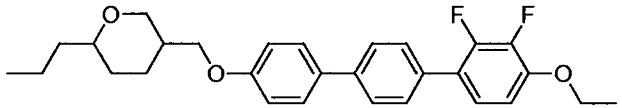 |
| 600 | 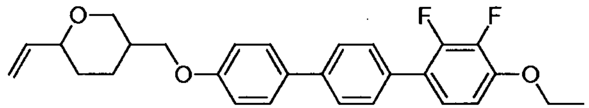 |
| 601 | 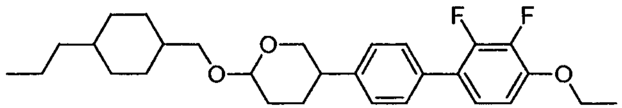 |
| 602 | 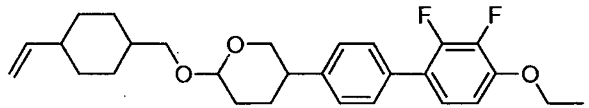 |
| 603 | 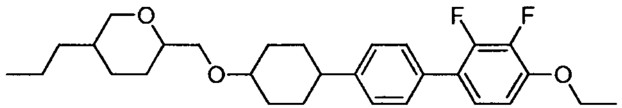 |
| 604 | 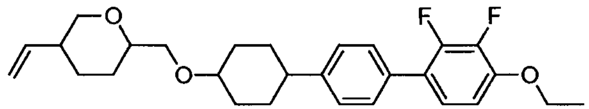 |
| 605 | 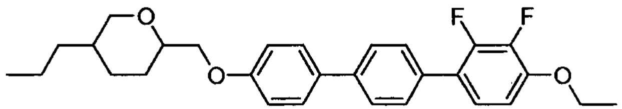 |
| 606 | 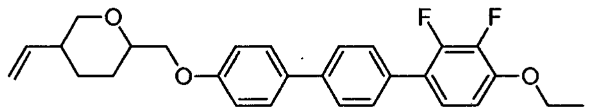 |

(continued)

| No. | | No. |
|---|---|---|
| 607 | 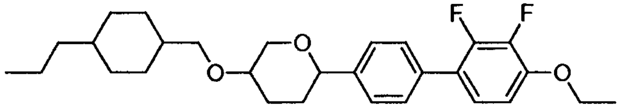 | |
| 608 | 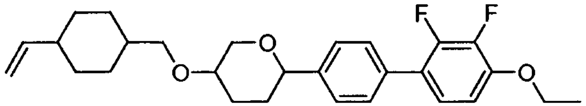 | |
| 609 | 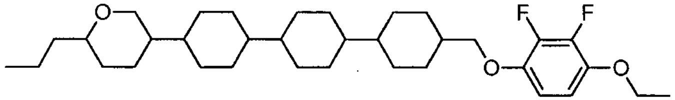 | |
| 610 | 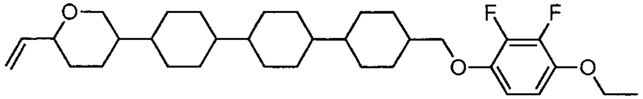 | |
| 611 | 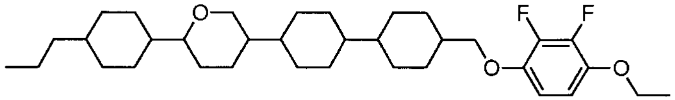 | |
| 612 | 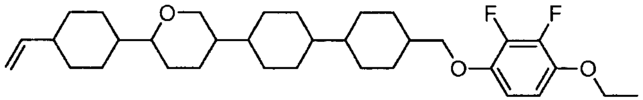 | |
| 613 | 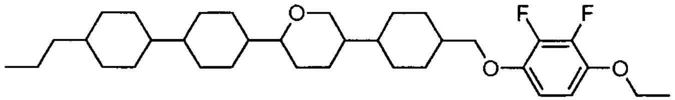 | |
| 614 | 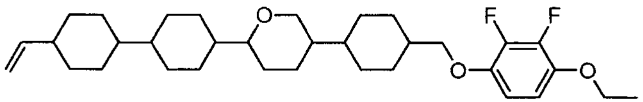 | |
| 615 | 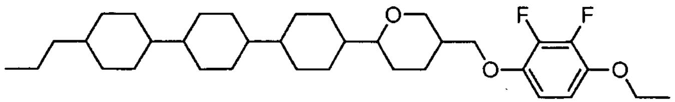 | |
| 616 | 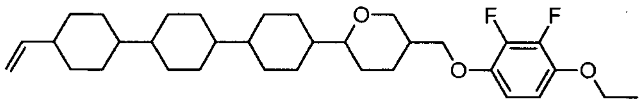 | |

(continued)

| No. | | No. |
|---|---|---|
| 617 | 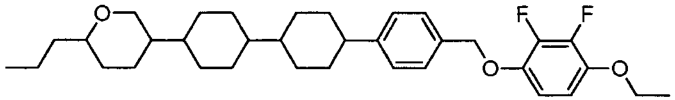 | |
| 618 | 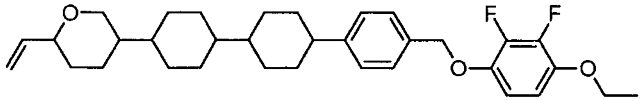 | |
| 619 | 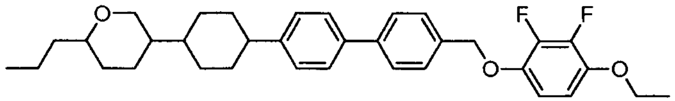 | |
| 620 | 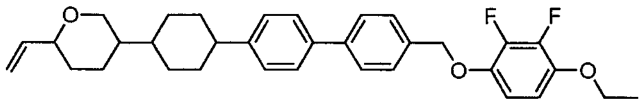 | |
| 621 | 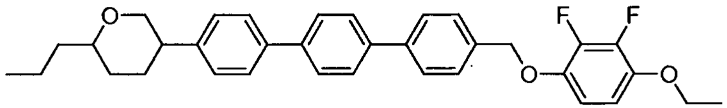 | |
| 622 | 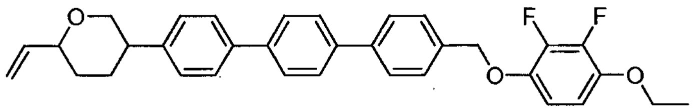 | |
| 623 | 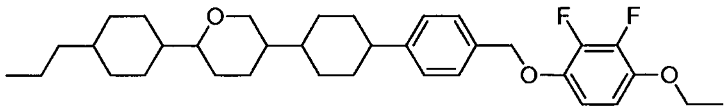 | |
| 624 | 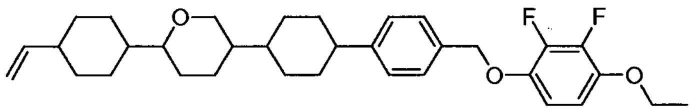 | |
| 625 | 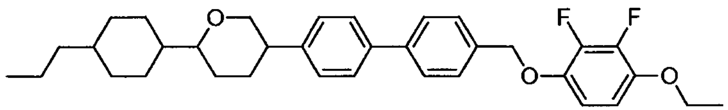 | |

(continued)

| No. | |
|---|---|
| 626 | 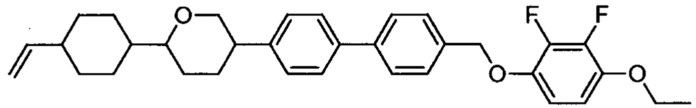 |
| 627 | 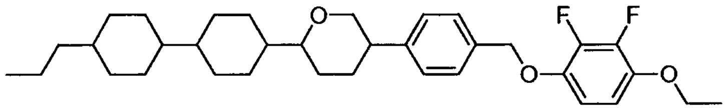 |
| 628 | 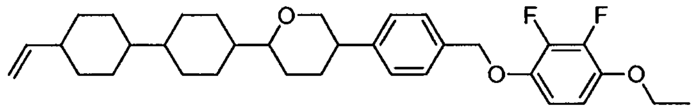 |
| 629 | 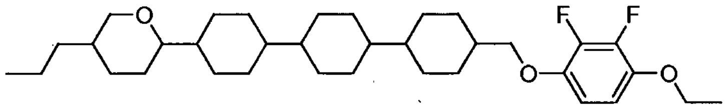 |
| 630 | 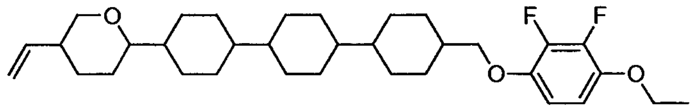 |
| 631 | 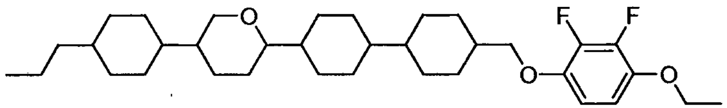 |
| 632 | 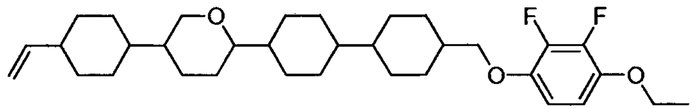 |
| 633 | 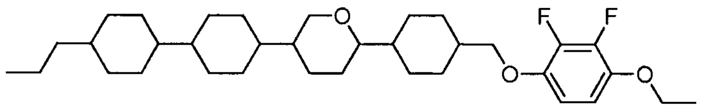 |
| 634 | 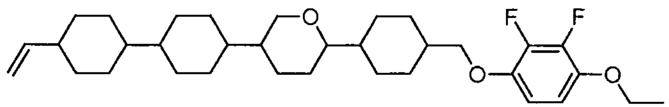 |

(continued)

| No. | No. |
|---|---|
| 635 | 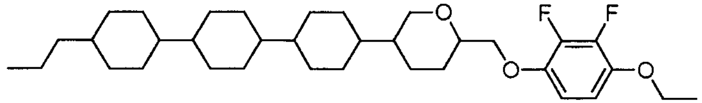 |
| 636 | 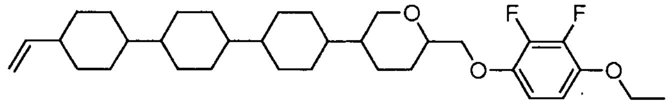 |
| 637 | 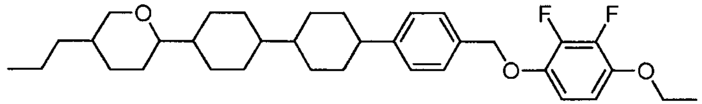 |
| 638 | 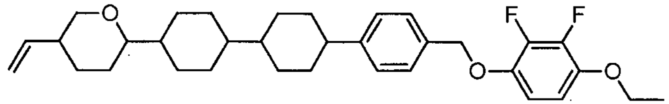 |
| 639 | 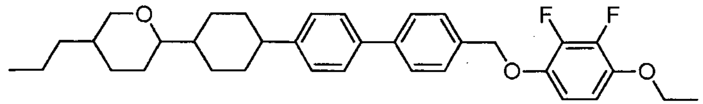 |
| 640 | 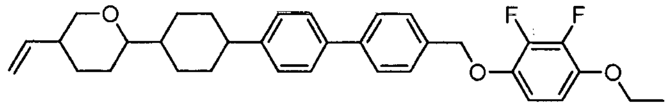 |
| 641 | 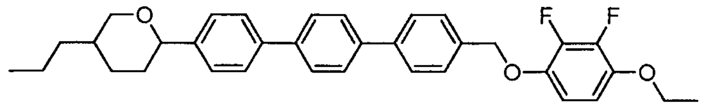 |
| 642 | 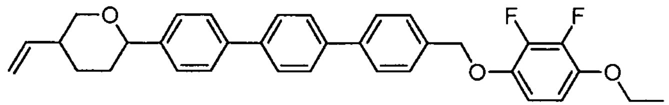 |
| 643 | 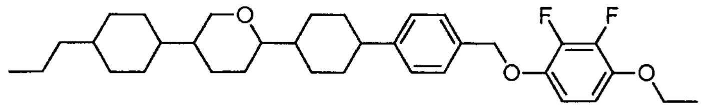 |

(continued)

| No. | |
|---|---|
| 644 | 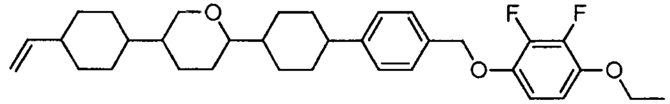 |
| 645 | 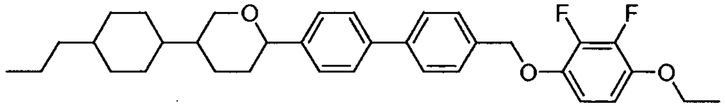 |
| 646 | 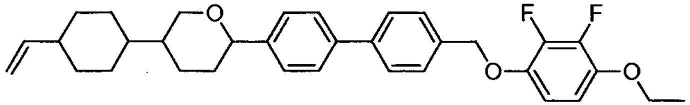 |
| 647 | 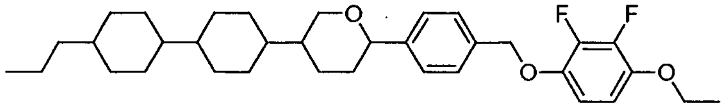 |
| 648 | 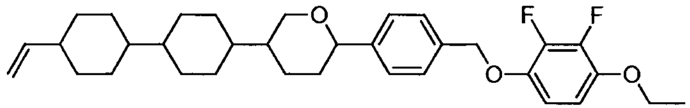 |

**Comparative Example 1**

The liquid crystal composition B consisting of the compound (b) (15% by weight) that was prepared according to the patent document, JP 2000-008040 A (applicant: Chisso Corporation), and the mother liquid crystals A (85% by weight) was prepared. Physical properties of the resultant liquid crystal composition B were measured, and extrapolated values on physical properties of the liquid crystal compound (b) were calculated from measured values, according to the extrapolation. The extrapolated values were as follows.

Maximum temperature (NI) = 121.3 °C; Dielectric anisotropy (Δε) = -7.3; Refractive index anisotropy (Δn) = 0.107; Viscosity (η) = 61.4 mPa·s.

**Example 4**

The liquid crystal composition C consisting of the compound No. 9 (15% by weight) and the mother liquid crystals A (85% by weight) was prepared. Physical properties of the resultant liquid crystal composition C were measured, and extrapolated values on physical properties of the compound No. 9 were calculated from the measured values, according to the extrapolation. The extrapolated values were as follows.

Maximum temperature (NI) = 116.6 °C; Dielectric anisotropy (Δε) = -8.5; Refractive index anisotropy (Δn) = 0.094; Viscosity (η) = 59.8 mPa·s.

It was found that the compound No. 9 of the invention in Example 1 was excellent in view of a negatively large dielectric anisotropy (Δε) and a low viscosity (η) in comparison with the compound (b) in Comparative Example 1.

**Example of liquid crystal compositions**

Typical compositions of the invention are summarized in Examples 5 to 10. First, compounds that are the component of a composition and their amounts (weight %) are shown. The compounds are expressed as symbols of left-terminal groups, bonding groups, ring structures and right-terminal groups according to notation described in Table 1.

84

Table. Method of Description of Compound using Symbols

| R-($A_1$)-$Z_1$-.....-$Z_n$-($A_n$) -R' | | | |
|---|---|---|---|
| 1) Left Terminal Group R- | Symbol | 4) Ring Structure -$A_n$- | Symbol |
| $C_nH_{2n+1}$- | n- | | |
| $C_nH_{2n+1}O$- | nO- | 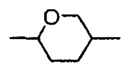 | Dh |
| $C_mH_{2m+1}OC_nH_{2n}$- | mOn- | | |
| $CH_2$=CH- | V- | 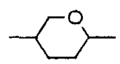 | dh |
| $C_nH_{2n+1}$-CH=CH- | nV- | | |
| $CH_2$=CH-$C_nH_{2n}$- | Vn- | 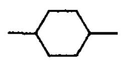 | H |
| $C_mH_{2m+1}$-CH=CH-$C_nH_{2n}$- | mVn- | | |
| $CF_2$=CH- | VFF- | 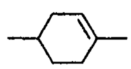 | Ch |
| $CF_2$=CH-$C_nH_{2n}$- | VFFn- | | |
| 2) Right Terminal Group -R' | Symbol | 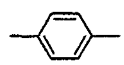 | B |
| -$C_nH_{2n+1}$ | -n | | |
| -$OC_nH_{2n+1}$ | -On | 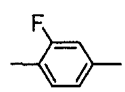 | B(2F) |
| -CH=$CH_2$ | -V | | |
| -CH=CH-$C_nH_{2n+1}$ | -Vn | 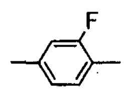 | B(3F) |
| -$C_nH_{2n}$-CH=$CH_2$ | -nV | | |

(continued)

| R-($A_1$)-$Z_1$-.....-$Z_n$-($A_n$) -R’ | | | |
|---|---|---|---|
| 1) Left Terminal Group R- | Symbol | 4) Ring Structure -$A_n$- | Symbol |
| -$CH=CF_2$ | -VFF | 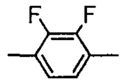 | B(2F,3F) |
| -$COOCH_3$ | -EMe | 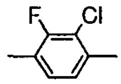 | B(2F,3Cl) |
| 3) Bonding Group -$Z_n$- | Symbol | | |
| -$C_nH_{2n}$- | n | | |
| -COO- | E | | |
| -CH=CH- | V | | |
| -$CH_2O$- | 1O | | |
| -$OCH_2$- | O1 | | |
| -$SiH_2$- | Si | | |
| -$CF_2O$- | X | | |
| 5) Compound of Description | | | |
| 1. 3-DhH1OB(2F,3F)-O2 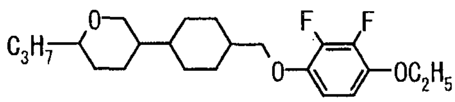 | | 2,3-BB(3F)B-4 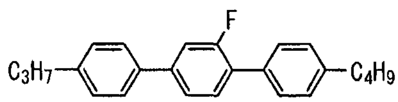 | |
| 3. 5-HBB(3F)B-3 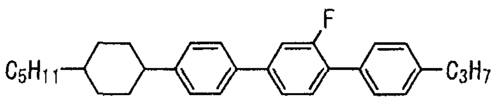 | | 4,3-HBB(2F,3F)-O2 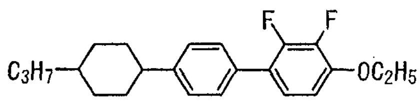 | |

**Example 5**

| | |
|---|---|
| 3-DhH1OB(2F,3F)-O2 | 15% |
| 5-DhH1OB(2F,3F)-O2 | 15% |
| 3-BB(3F)B-3 | 5% |
| V2-BB(3F)B-1 | 7% |
| 3-HB-O2 | 10° |
| 5-HB-O2 | 9% |
| 3-HB(2F,3F)-O2 | 5% |
| V-HB(2F,3F)-O2 | 10% |
| 3-DhB(2F,3F)-O2 | 8% |
| 3-HDhB(2F,3F)-O2 | 8% |
| 5-HDhB(2F,3F)-O2 | 8% |
| NI = 76.0 °C; Δn = 0.109, η = 39.7 mPa·s; Δε = -5.4. | |

**Example 6**

| | |
|---|---|
| 3-DhH1OB(2F,3F)-O2 | 12% |
| 5-DhH1OB(2F,3F)-O2 | 12% |
| | |
| 2-BB(3F)B-3 | 5% |
| 3-HB-O2 | 5% |
| 3-HHB-1 | 5% |
| V-HB(2F,3F)-O2 | 10% |
| 3-H2B(2F,3F)-O2 | 15% |
| 5-H2B(2F,3F)-O2 | 11% |
| 5-HHB(2F,3Cl)-O2 | 5% |
| 2-HBB(2F,3F)-O2 | 10% |
| 5-HBB(2F,3F)-O2 | 10% |
| NI = 81.2 °C; Δn = 0.111; η = 37.9 mPa·s; Δε = -5.4. | |

**Example 7**

| | |
|---|---|
| 3-DhH1OB(2F,3F)-O2 | 15% |
| 5-DhH1OB(2F,3F)-O2 | 15% |
| | |
| V2-BB-1 | 5% |
| V-HHB-1 | 3% |
| V2-HHB-1 | 3% |
| 3-HBB-2 | 5% |
| 3-HB(2F,3F)-O2 | 10% |
| V-HB(2F,3F)-O2 | 10% |
| 3-H2B(2F,3F)-O2 | 8% |
| 5-HHB(2F,3F)-O2 | 3% |
| V-HHB(2F,3F)-O2 | 3% |
| 2-HBB(2F,3F)-O2 | 3% |
| 1V2-HBB(2F,3F)-O2 | 7% |
| 5-DhB(2F,3F)-O2 | 10% |
| NI = 77.2 °C; Δn = 0.106; η = 37.3 mPa·s; Δε = -5.4. | |

**Example 8**

| | |
|---|---|
| 3-DhHlOB(2F,3F)-O2 | 15% |

(continued)

| | |
|---|---|
| 5-DhHIOB(2F,3F)-O2 | 15% |
| | |
| 2-BB(3F)B-3 | 4% |
| 5-HBB(3F)B-2 | 8% |
| 5-HBB(3F)B-3 | 7% |
| V2-BB-1 | 5% |
| 3-HHB-1 | 5% |
| V-HB(2F,3F)-O2 | 10% |
| 3-DhB(2F,3F)-O2 | 10% |
| 5-DhB(2F,3F)-O2 | 10% |
| 3-Dh2B(2F,3F)-O4 | 11% |
| NI = 84.7 °C; Δn = 0.117; η = 42.2 mPa·s ; Δε = -5.4. | |

**Example 9**

| | |
|---|---|
| 3-DhHIOB(2F,3F)-O2 | 15% |
| 5-DhHIOB(2F,3F)-O2 | 15% |
| | |
| 2-BB(3F)B-3 | 5% |
| 2-BB(3F)B-5 | 5% |
| 3-HB-02 | 7% |
| 3-HHEH-3 | 3% |
| 3-HHEBH-3 | 5% |
| 1O1-HBBH-4 | 3% |
| 3-HB(2F,3F)-O2 | 8% |
| V-HB(2F,3F)-O2 | 5% |
| 3-H2B(2F, 3F)-O2 | 10% |
| 3-Dh2B(2F,3F)-O4 | 10% |
| 5-Dh2B(2F,3F)-O2 | 4% |
| 5-BDhB(2F,3F)-O2 | 5% |
| NI = 83.1 °C; Δn = 0.106; η = 37.3 mPa·s; Δε = -5.4. | |

When 0.25 part of the optically active compound (Op-5) was added to 100 parts of the composition, the pitch was 61.1 μm.

**Comparative Example 2**

| | |
|---|---|
| 3-HDhIOB(2F,3F)-O2 | 10% |
| 3-HdhIOB(2F,3F)-O2 | 10% |
| 3-dhHIOB(2F,3F)-O2 | 10% |
| 3-DhIOHB(2F,3F)-O2 | 8% |
| 3-dhIOHB(2F,3F)-O2 | 8% |
| | |
| 2-BB(3F)B-5 | 3% |
| 5-HBB(3F)B-3 | 3% |
| V-HHB-1 | 3% |
| 101-HBBH-4 | 5% |
| 3-HB(2F,3F)-O2 | 10% |
| V-HB(2F, 3F)-O2 | 10% |
| 5-H2B(2F,3F)-O2 | 10% |
| 5-HHB(2F,3Cl)-O2 | 5% |
| 3-HHB(2F, 3F)-1 | 5% |

**Example 10**

| | |
|---|---|
| 3-DhHIOB(2F,3F)-O3 | 10% |
| 3-dhIOHB(2F,3F)-O2 | 10% |
| 3-HIODhB(2F,3F)-O2 | 10% |
| 3-DhBIOB(2F,3F)-O2 | 10% |
| 3-DhIOBB(2F,3F)-O2 | 8% |
| V2-BB(3F)B-I | 5% |
| 3-HB-O2 | 10% |
| 3-HHB-O1 | 5% |
| 3-HHEBH-5 | 4% |
| 3-HB(2F,3F)-O2 | 8% |
| V-HB(2F,3F)-O2 | 8% |
| 3-H2B(2F,3F)-O2 | 5% |
| 5-HHB(2F, 3F)-O2 | 7% |

**Applicability in Industry**

The invention provides a liquid crystal compound that is excellent in compatibility with other liquid crystal materials and has a negatively large dielectric anisotropy ($\Delta\varepsilon$).

The invention also provides a novel liquid crystal composition that contains the liquid crystal compound as a component and has desired characteristics, by means of suitable selection of rings, substituents and so forth that constitute the compound, and further provides a liquid crystal display device that is constituted by using the liquid crystal composition.

## Claims

1. A compound represented by formula (1-1)):

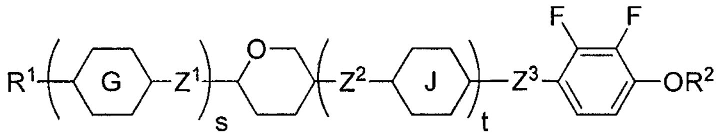

(1-1)

wherein
$R^1$ and $R^2$ are each independently alkyl having 1 to 10 carbons; and in the alkyl arbitrary $-CH_2-$ may be replaced by -O-, where a plurality of $-CH_2-$ adjacent to each other are not replaced, and arbitrary - $(CH_2)_2-$ may be replaced by -CH=CH-;
the ring G and the ring J are each independently 1,4-cyclohexylene or 1,4-phenylene; and in the 1,4-cyclohexylene arbitrary $-CH_2-$ may be replaced by -O-, and arbitrary - $(CH_2)_2-$ may be replaced by -CH=CH-, in the 1,4-phenylene arbitrary -CH= may be replaced by -N=;
$Z^1$ and $Z^2$ are each a single bond, $Z^3$ is $-CH_2O-$; and
s and t are each independently 0, 1, 2 or 3, where the sum of s and t is 1, 2 or 3.

2. The compound according to claim 1, wherein in formula (1-1), $R^1$ is alkyl having 1 to 10 carbons or alkenyl having 2 to 10 carbons; $R^2$ is alkyl having 1 to 10 carbons; and the ring G and the ring J are each independently 1,4-cyclohexylene or 1,4-phenylene.

3. The compound according to any one of claims 1 to 3, wherein the sum of s and t is 1 or 2.

4. A compound according to claim 1 represented by formula (1-1-1) :

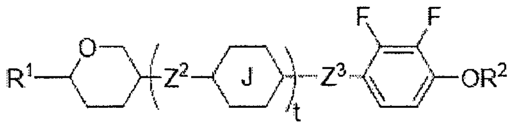

(1-1-1)

wherein $R^1$ is alkyl having 1 to 10 carbons or alkenyl having 2 to 10 carbons; $R^2$ is alkyl having 1 to 10 carbons; the ring J is independently 1,4-cyclohexylene or 1,4-phenylene; $Z^2$ is a single bond, $Z^3$ is $-CH_2O-$; and t is 1, 2 or 3.

5. A compound according to claim 1 represented by formula (1-1-1-1) :

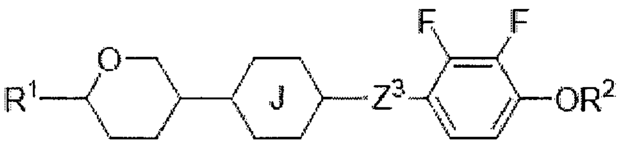

(1-1-1-1)

wherein $R^1$ and $R^2$ are each independently alkyl having 1 to 8 carbons; the ring J is 1,4-cyclohexylene or 1,4-phenylene; and $Z^3$ is $-CH_2O-$.

6. A liquid crystal composition comprising at least one of compounds according to any one of claims 1 to 5, as a component A.

7. The liquid crystal composition according to claim 6, further comprising at least one compound selected from the group of compounds represented by formulae (2), (3) and (4), as a component B:

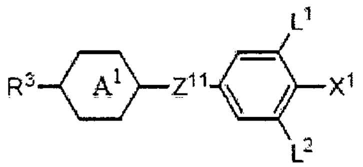

(2)

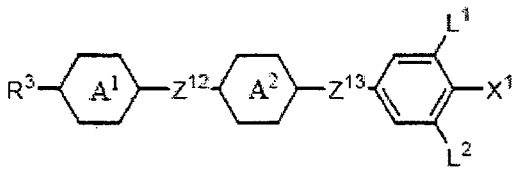

(3)

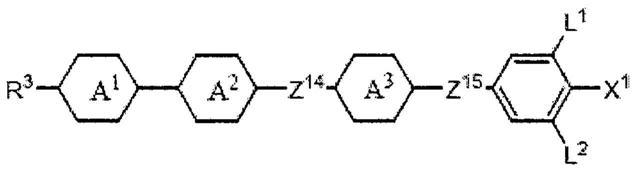

(4)

wherein
$R^3$ is alkyl having 1 to 10 carbons or alkenyl having 2 to 10 carbons, and in the alkyl and the alkenyl, arbitrary hydrogen may be replaced by fluorine and arbitrary $-CH_2-$ may be replaced by -O-;
$X^1$ is fluorine, chlorine, $-OCF_3$, $-OCHF_2$, $-CF_3$, $-CHF_2$, $-CH_2F$, $-OCF_2CHF_2$ or $-OCF_2CHFCF_3$;
the ring $A^1$, the ring $A^2$ and the ring $A^3$ are each independently 1,4-cyclohexylene, 1,3-dioxane-2,5-diyl, pyrimidine-2,5-diyl, 1-tetrahydropyran-2,5-diyl or 1,4-phenylene in which arbitrary hydrogen may be replaced by fluorine;
$Z^{11}$, $Z^{12}$, $Z^{13}$, $Z^{14}$ and $Z^{15}$ are each independently $-(CH_2)_2-$, $-(CH_2)_4-$, -COO-, -CF20-, $-OCF_2-$, -CH=CH-, -C≡C-, $-CH_2O-$ or a single bond; and
$L^1$ and $L^2$ are each independently hydrogen or fluorine.

**8.** The liquid crystal composition according to claim 6, further comprising at least one compound selected from the group of compounds represented by formula (5), as a component C:

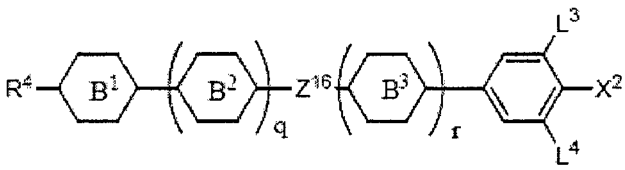

(5)

wherein
$R^4$ is alkyl having 1 to 10 carbons or alkenyl having 2 to 10 carbons, and in the alkyl and the alkenyl, arbitrary hydrogen may be replaced by fluorine and arbitrary
$-CH_2-$ may be replaced by -O-;
$X^2$ is -C=N or -C=C-C=N;
the ring $B^1$, the ring $B^2$ and the ring $B^3$ are each independently 1,4- cyclohexylene, 1,4-phenylene in which arbitrary hydrogen may be replaced by fluorine, 1,3-dioxane-2,5-diyl, 1-tetrahydropyran-2,5-diyl or pyrimidine-2,5-diyl;
$Z^{16}$ is $-(CH_2)_2-$, -COO-, $-CF_2O-$, $-OCF_2-$, -C≡C-, $-CH_2O-$ or a single bond;
$L^3$ and $L^4$ are each independently hydrogen or fluorine; and q is 0, 1 or 2 and r is 0 or 1.

**9.** The liquid crystal composition according to claim 6, further comprising at least one compound selected from the group of compounds represented by formulae (6), (7), (8), (9) and (10), as a component D:

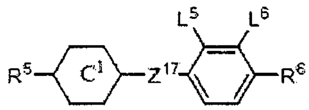

(6)

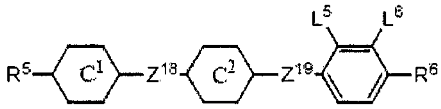

(7)

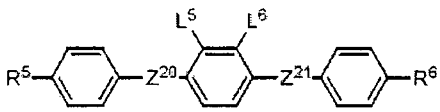

(8)

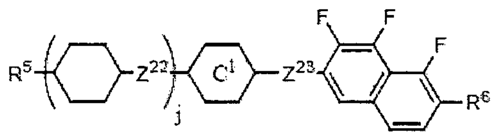

(9)

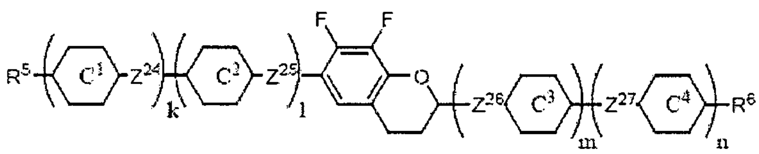

(10)

wherein
$R^5$ and $R^6$ are each independently alkyl having 1 to 10 carbons or alkenyl having 2 to 10 carbons, and in the alkyl and the alkenyl, arbitrary hydrogen may be replaced by fluorine and arbitrary $-CH_2-$may be replaced by -O-;
the ring $C^1$, the ring $C^2$, the ring $C^3$ and the ring $C^4$ are each independently 1,4-cyclohexylene, 1,4-cyclohexenylene, 1,4-phenylene in which arbitrary hydrogen may be replaced by fluorine or decahydro-2,6-naphthalene;
$Z^{17}$, $Z^{18}$, $Z^{19}$, $Z^{20}$, $Z^{21}$, $Z^{22}$, $Z^{23}$, $Z^{24}$, $Z^{25}$, $Z^{26}$ and $Z^{27}$ are each independently $-(CH_2)_2-$, -COO-, $-CH_2O-$, $-OCF_2-$, $-OCF_2(CH_2)_2-$ or a single bond;
$L^5$ and $L^6$ are each independently chlorine or fluorine; and
j, k, l, m and n are each independently 0 or 1, and the sum of k, l, m and n is 1 or 2.

**10.** The liquid crystal composition according to claim 6, further comprising at least one compound selected from the group of compounds represented by formulae (11), (12) and (13), as a component E:

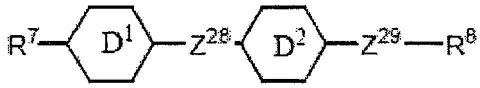

(11)

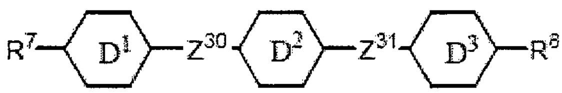

(12)

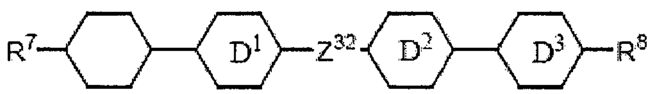

(13)

wherein

$R^7$ and $R^8$ are each independently alkyl having 1 to 10 carbons or alkenyl having 2 to 10 carbons, and in the alkyl and the alkenyl, arbitrary hydrogen may be replaced by fluorine and arbitrary $-CH_2-$ may be replaced by -O-; the ring $D^1$, the ring $D^2$ and the ring $D^3$ are each independently 1,4-cyclohexylene, pyrimidine-2,5-diyl, 1,4-phenylene, 2-fluoro-1,4-phenylene, 3-fluoro-1,4-phenylene or 2,5-difluoro-1,4-phenylene; and $Z^{28}$, $Z^{29}$, $Z^{30}$, $Z^{31}$ and $Z^{32}$ are each independently -C≡C-, -COO-, $-(CH_2)_2-$, -CH=CH- or a single bond.

**11.** The liquid crystal composition according to claim 7, further comprising at least one compound selected from the group of compounds represented by formula (5) according to claim 8.

**12.** The liquid crystal composition according to claim 7, further comprising at least one compound selected from the group of compounds represented by formulae (11), (12) and (13) according to claim 10.

**13.** The liquid crystal composition according to claim 8, further comprising at least one compound selected from the group of compounds represented by formulae (11), (12) and (13) according to claim 10.

**14.** The liquid crystal composition according to claim 9, further comprising at least one compound selected from the group of compounds represented by formulae (11), (12) and (13) according to claim 10.

**15.** The liquid crystal composition according to any one of claims 6 to 14, further comprising at least one optically active compound.

**16.** The liquid crystal composition according to any one of claims 6 to 15, further comprising at least one antioxidant, or at least one ultraviolet absorbent, or at least each of an antioxidant and an ultraviolet absorbent.

**17.** A liquid crystal display device comprising at least one of the liquid crystal compositions according to any one of claims 6 to 16.

**Patentansprüche**

**1.** Eine Verbindung dargestellt durch Formel (1-1):

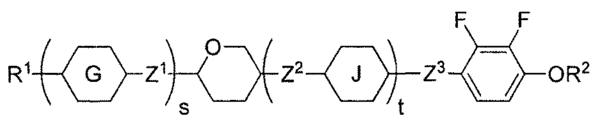

(1-1)

wobei
$R^1$ und $R^2$ jeweils unabhängig voneinander Akyl mit 1 bis 10 Kohlenstoffen sind; und im Alkyl beliebige $-CH_2-$ durch -O- ersetzt sein können, wo mehrere $-CH_2-$, die benachbart zueinander sind, nicht ersetzt werden, und beliebige $-(CH_2)_2-$ durch -CH=CH- ersetzt sein können;
der Ring G und der Ring J jeweils unabhängig voneinander 1,4-Cyclohexylen oder 1,4-Phenylen sind; und im 1,4-Cyclohexylen beliebige $-CH_2-$ durch -O- ersetzt sein können, und beliebige $-(CH_2)_2-$ durch -CH=CH- ersetzt sein können, im 1,4-Phenylen beliebige -CH= durch -N= ersetzt sein können;
$Z^1$ und $Z^2$ jeweils eine Einfachbindung sind, $Z^3$ $-CH_2O-$ ist; und s und t jeweils unabhängig voneinander 0, 1, 2 oder 3 sind, wobei die Summe von s und t 1, 2 oder 3 ist.

**2.** Die Verbindung gemäß Anspruch 1, wobei in Formel (1-1) $R_1$ Alkyl mit 1 bis 10 Kohlenstoffen oder Alkenyl mit 2 bis 10 Kohlenstoffen ist; $R^2$ Alkyl mit 1 bis 10 Kohlenstoffen ist; und der Ring G und der Ring J jeweils unabhängig voneinander 1,4-Cyclohexylen oder 1,4-Phenylen sind.

**3.** Die Verbindung gemäß einem der Ansprüche 1 bis 3, wobei die Summe von s und t 1 oder 2 ist.

**4.** Eine Verbindung gemäß Anspruch 1 der Formel (1-1-1):

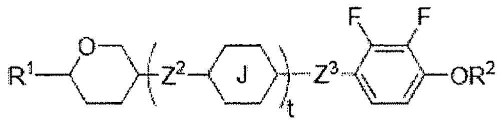

(1-1-1)

wobei $R^1$ Alkyl mit 1 bis 10 Kohlenstoffen oder Alkenyl mit 2 bis 10 Kohlenstoffen ist; $R^2$ Alkyl mit 1 bis 10 Kohlenstoffen ist; der Ring J unabhängig 1,4-Cyclohexylen oder 1,4-Phenylen ist; $Z^2$ eine Einfachbindung ist, $Z^3$ $-CH_2O-$ ist; und t 1, 2 oder 3 ist.

**5.** Eine Verbindung gemäß Anspruch 1 dargestellt durch Formel (1-1-1-1):

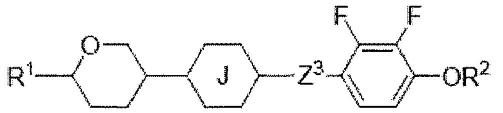

(1-1-1-1)

wobei $R^1$ und $R^2$ jeweils unabhängig voneinander Alkyl mit 1 bis 8 Kohlenstoffen sind; der Ring J 1,4-Cyclohexylen oder 1,4-Phenylen ist; und $Z^3$ $-CH_2O-$ ist.

**6.** Eine Flüssigkristallzusammensetzung umfassend mindestens eine Verbindung gemäß einem der Ansprüche 1 bis 5 als eine Komponente A.

**7.** Die Flüssigkristallzusammensetzung gemäß Anspruch 6, des Weiteren umfassend mindestens eine Verbindung ausgewählt aus der Gruppe von Verbindungen dargestellt durch Formeln (2), (3) und (4) als eine Komponente B:

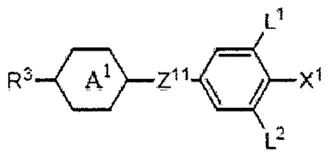

(2)

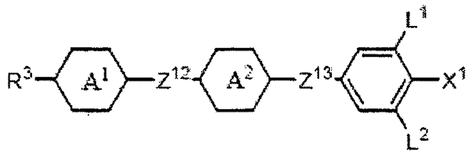

(3)

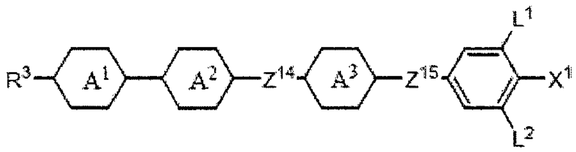

(4)

wobei

$R^3$ Alkyl mit 1 bis 10 Kohlenstoffen oder Alkenyl mit 2 bis 10 Kohlenstoffen ist, und im Alkyl und im Alkenyl beliebige Wasserstoffe durch Fluor ersetzt sein können und beliebige $-CH_2-$ durch -O- ersetzt sein können.

$X^1$ Fluor, Chlor, $-OCF_3$, $-OCHF_2$, $-CF_3$, $-CHF_2$, $-CH_2F-$, $-OCF_2CHF_2$ oder - $OCF_2CHFCF_3$ ist;

der Ring $A^1$, der Ring $A^2$ und der Ring $A^3$ jeweils unabhängig voneinander 1,4-Cyclohexylen, 1,3-Dioxan-2,5-diyl, Pyrimidin-2,5-diyl, 1-Tetrahydropyran-2,5-diyl oder 1,4-Phenylen, in welchem beliebige Wasserstoffe durch Fluor ersetzt sein können, sind;

$Z^{11}$, $Z^{12}$, $Z^{13}$, $Z^{14}$ und $Z^{15}$ jeweils unabhängig voneinander $-(CH_2)_2-$, $-(CH_2)_4-$, -COO- , $-CF_2O-$, $-OCF_2-$, -CH=CH-, $-C{\equiv}C-$, $-CH_2O-$ oder eine Einfachbindung sind; und

$L^1$ und $L^2$ jeweils unabhängig voneinander Wasserstoff oder Fluor sind.

**8.** Die Flüssigkristallzusammensetzung gemäß Anspruch 6, des Weiteren umfassend mindestens eine Verbindung ausgewählt aus der Gruppe von Verbindungen dargestellt durch Formel (5) als eine Komponente C:

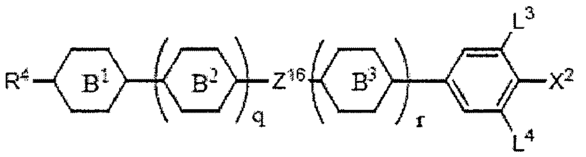
(5)

wobei
$R^4$ Alkyl mit 1 bis 10 Kohlenstoffen oder Alkenyl mit 2 bis 10 Kohlenstoffen ist, und im Alkyl oder im Alkenyl beliebige Wasserstoffe durch Fluor ersetz sein können und beliebige $-CH_2-$ durch -O- ersetzt sein können;
$X^2$ -C≡N oder -C≡C-C≡N ist;
der Ring $B^1$, der Ring $B^2$ und der Ring $B^3$ jeweils unabhängig voneinander 1,4-Cyclohexylen, 1,4-Phenylen, in welchem beliebige Wasserstoffe durch Fluor ersetzt sein können, 1,3-Dioxan-2,5-diyl, 1-Tetrahydropyran-2,5-diyl oder Pyrimidin-2,5-diyl sind;
$Z^{16}$ $-(CH_2)_2-$, -COO-, $-CF_2O-$, $-OCF_2-$, -C≡C-, $-CH_2O-$ oder eine Einfachbindung ist;
$L^3$ und $L^4$ jeweils unabhängig voneinander Wasserstoff oder Fluor sind; und q 0, 1 oder 2 ist und r 0 oder 1 ist.

**9.** Die Flüssigkristallzusammensetzung gemäß Anspruch 6, des Weiteren umfassend mindestens eine Verbindung ausgewählt aus der Gruppe von Verbindungen dargestellt durch Formeln (6), (7), (8), (9) und (10) als eine Komponente D:

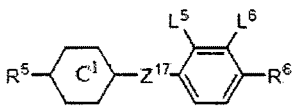
(6)

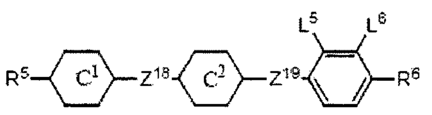
(7)

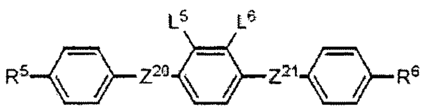
(8)

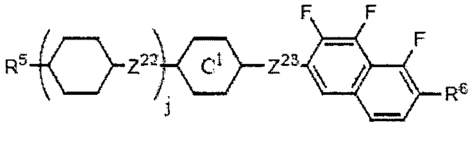
(9)

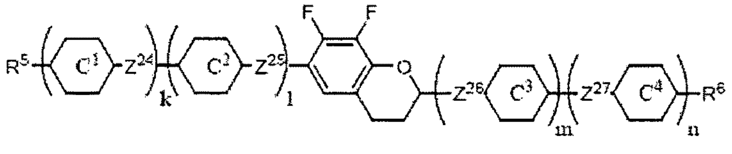
(10)

wobei
$R^5$ und $R^6$ jeweils unabhängig voneinander Alkyl mit 1 bis 10 Kohlenstoffen oder Alkenyl mit 2 bis 10 Kohlenstoffen sind, und im Alkyl oder im Alkenyl beliebige Wasserstoffe durch Fluor ersetzt sein können und beliebige $-CH_2-$ durch -O- ersetzt sein können;

der Ring $C^1$, der Ring $C^2$, der Ring $C^3$ und der Ring $C^4$ jeweils unabhängig voneinander 1,4-Cyclohexylen, 1,4-Cyclohexenylen, 1,4-Phenylen, in welchem beliebige Wasserstoffe durch Fluor ersetzt sein können, oder Decahydro-2,6-naphthalin sind;
$Z^{17}$, $Z^{18}$, $Z^{19}$, $Z^{20}$, $Z^{21}$, $Z^{22}$, $Z^{23}$, $Z^{24}$, $Z^{25}$, $Z^{26}$ und $Z^{27}$ jeweils unabhängig voneinander $-(CH_2)_2-$, -COO-, $-CH_2O-$, $-OCF_2-$, $-OCF_2(CH_2)_2-$ oder ein Einfachbindung sind;
$L^5$ und $L^6$ jeweils unabhängig voneinander Chlor oder Fluor sind; und j, k, l, m und n jeweils unabhängig voneinander 0 oder 1 sind, und die Summe von k, l, m und n 1 oder 2 ist.

**10.** Die Flüssigkristallzusammensetzung gemäß Anspruch 6, des Weiteren umfassend mindestens eine Verbindung ausgewählt aus der Gruppe von Verbindungen dargestellt durch Formeln (11), (12) und (13) als eine Komponente E:

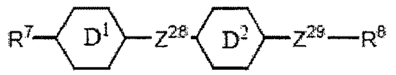
(11)

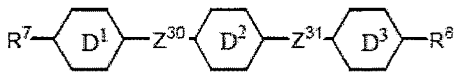
(12)

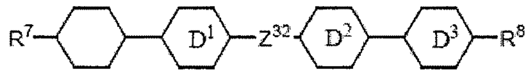
(13)

wobei
$R^7$ und $R^8$ jeweils unabhängig voneinander Alkyl mit 1 bis 10 Kohlenstoffen oder Alkenyl mit 2 bis 10 Kohlenstoffen sind, und im Alkyl oder im Alkenyl beliebige Wasserstoffe durch Fluor ersetzt sein können und beliebige $-CH_2-$ durch -O- ersetzt sein können;
der Ring $D^1$, der Ring $D^2$ und der Ring $D^3$ jeweils unabhängig voneinander 1,4-Cyclohexylen, Pyrimidin-2,5-diyl, 1,4-Phenylen, 2-Fluoro-1,4-phenylen, 3-Fluoro-1,4-phenylen oder 2,5-Difluoro-1,4-phenylen sind; und
$Z^{28}$, $Z^{29}$, $Z^{30}$, $Z^{31}$ und $Z^{32}$ jeweils unabhängig voneinander -C≡C-, -COO-, $-(CH_2)_2-$ , -CH=CH- oder eine Einfachbindung sind.

**11.** Die Flüssigkristallzusammensetzung gemäß Anspruch 7, des Weiteren umfassend mindestens eine Verbindung ausgewählt aus der Gruppe von Verbindungen dargestellt durch Formel (5) gemäß Anspruch 8.

**12.** Die Flüssigkristallzusammensetzung gemäß Anspruch 7, des Weiteren umfassend mindestens eine Verbindung ausgewählt aus der Gruppe von Verbindungen dargestellt durch Formeln (11), (12) und (13) gemäß Anspruch 10.

**13.** Die Flüssigkristallzusammensetzung gemäß Anspruch 8, des Weiteren umfassend mindestens eine Verbindung ausgewählt aus der Gruppe von Verbindungen dargestellt durch Formeln (11), (12) und (13) gemäß Anspruch 10.

**14.** Die Flüssigkristallzusammensetzung gemäß Anspruch 9, des Weiteren umfassend mindestens eine Verbindung ausgewählt aus der Gruppe von Verbindungen dargestellt durch Formeln (11), (12) und (13) gemäß Anspruch 10.

**15.** Die Flüssigkristallzusammensetzung gemäß einem der Ansprüche 6 bis 14, des Weiteren umfassend mindestens eine optisch aktive Verbindung.

**16.** Die Flüssigkristallzusammensetzung gemäß einem der Ansprüche 6 bis 15, des Weiteren umfassend mindestens ein Antioxidans oder mindestens ein Ultraviolett-Absorptionsmittel oder mindestens eines von jeweils einem Antioxidans und einem Ultraviolett-Absorptionsmittel.

**17.** Eine Flüssigkristallanzeige-Vorrichtung umfassend mindestens eine der Flüssigkristallzusammensetzungen gemäß einem der Ansprüche 6 bis 16.

## Revendications

**1.** Composé représenté par la formule (1-1) :

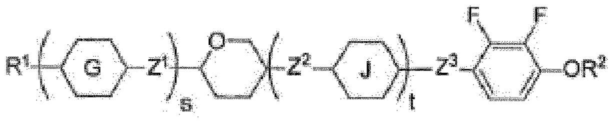

(1-1)

dans lequel
$R^1$ et $R^2$ sont chacun de façon indépendante un alkyle ayant de 1 à 10 carbones ; et dans l'alkyle, le $-CH_2-$ arbitraire peut être remplacé par -O-, dans lequel une pluralité de $-CH_2-$ adjacents les uns avec les autres ne sont pas remplacés, et le $-(CH_2)_2-$ arbitraire peut être remplacé par -CH=CH- ;
L'anneau G et l'anneau J sont chacun de façon indépendante le 1,4-cyclohexylène ou le 1,4-phénylène ; et dans le 1,4-cyclohexylène, le $-CH_2-$ arbitraire peut être remplacé par -O-, et le $-(CH_2)_2-$ arbitraire peut être remplacé par -CH=CH- et dans le 1,4-phénylène, le -CH= arbitraire peut être remplacé par -N= ;
$Z^1$ et $Z^2$ sont chacun une liaison unique, $Z^3$ est $-CH_20-$ ; et s et t sont chacun de façon indépendante 0, 1, 2 ou 3, dans lequel la somme de s et t est 1, 2 ou 3.

2. Composé selon la revendication 1, dans lequel dans la formule (1-1), $R^1$ est un alkyle ayant de 1 à 10 carbones ou un alkényle ayant de 2 à 10 carbones ; $R^2$ est un alkyle ayant de 1 à 10 carbones ; et l'anneau G et l'anneau J sont chacun de façon indépendante le 1,4-cyclohexylène ou le 1,4-phénylène.

3. Composé selon l'une des revendications 1 à 3 dans lequel la somme de s et t est 1 ou 2.

4. Composé selon la revendication 1, représenté par la formule (1-1-1) :

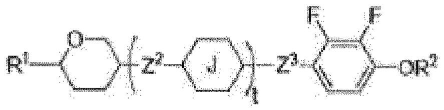

(1-1-1)

dans lequel $R^1$ est un alkyle ayant de 1 à 10 carbones ou un alkényle ayant de 2 à 10 carbones ; $R^2$ est un alkyle ayant de 1 à 10 carbones ; l'anneau J est de façon indépendante le 1,4-cyclohexylène ou le 1,4-phénylène ; $Z^2$ est une liaison unique, $Z^3$ est $-CH_2O-$ ; et t est 1, 2 ou 3.

5. Composé selon la revendication 1 représenté par la formule (1-1-1-1) :

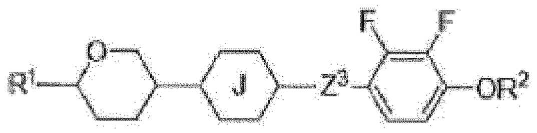

(1-1-1-1)

dans lequel $R^1$ et $R^2$ sont chacun de façon indépendante un alkyle ayant de 1 à 8 carbones ; l'anneau J est le 1,4-cyclohexylène ou le 1,4-phénylène ; et $Z^3$ est -CH=O-.

6. Composition de cristaux liquides comprenant au moins un des composés selon l'une quelconque des revendications 1 à 5, comme composant A.

7. Composition de cristaux liquides selon la revendication 6, comprenant en outre au moins un composé sélectionné parmi le groupe de composés représentés par les formules (2), (3) et (4), comme composant B :

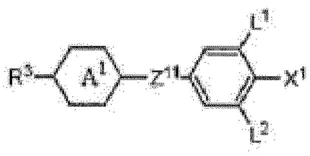

(2)

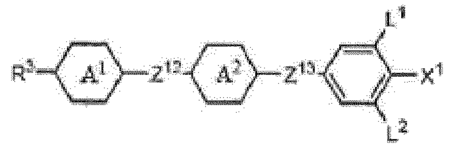 (3)

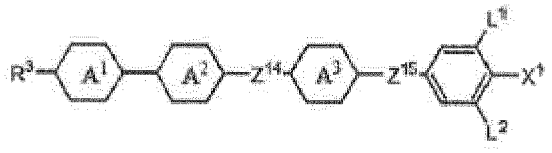 (4)

dans laquelle
$R^3$ est un alkyle ayant de 1 à 10 carbones ou un alkényle ayant de 2 à 10 carbones, et dans l'alkyle et l'alkényle, l'hydrogène arbitraire peut être remplacé par le fluor et le $-CH_2-$ arbitraire peut être remplacé par -0- ;
$X^1$ est le fluor, le chlore, $-OCF_3$, $-OCHF_2$, $-CF_3$, $-CHF_2$, $-CH_2F$, - $OCF_2CHF_2$ ou $-OCF_2CHFCF_3$ ;
l'anneau $A^1$, l'anneau $A^2$ et l'anneau $A^3$ sont chacun de façon indépendante le 1,4-cyclohexylène, le 1,3-dioxane-2,5-diyl, la pyrimidine-2,5-diyl, le 1-tétrahydropyrane-2,5-diyl ou le 1,4-phénylène dans lequel l'hydrogène arbitraire peut être remplacé par le fluor ;
$Z^{11}$, $Z^{12}$, $Z^{13}$, $Z^{14}$ et $Z^{15}$ sont chacun de façon indépendante - $(CH_2)_2$-, $-(CH_2)_4-$, -COO-, $-CF_2O-$, $-OCF_2-$, -CH=CH-, -C≡C-, $-CH_2O-$ ou une liaison unique ; et
$L^1$ et $L^2$ sont chacun de façon indépendante l'hydrogène ou le fluor.

8. Composition de cristaux liquides selon la revendication 6, comprenant en outre au moins un composé sélectionné parmi le groupe de composés représentés par la formule (5), comme composant C ;

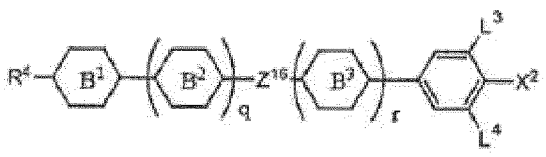 (5)

dans lequel
$R^4$ est un alkyle ayant de 1 à 10 carbones ou un alkényle ayant de 2 à 10 carbones, et dans l'alkyle et l'alkényle, l'hydrogène arbitraire peut être remplacé par le fluor et le $-CH_2-$ arbitraire peut être remplacé par -O- ;
$X^2$ est -C=N ou -C≡C-C≡N ;
l'anneau $B^1$, l'anneau $B^2$ et l'anneau $B^3$ sont chacun de façon indépendante le 1,4-cyclohexylène, le 1,4-phénylène dans lequel l'hydrogène arbitraire peut être remplacé par le fluor, le 1,3-dioxane-2,5-diyl, le 1-tétrahydropyrane-2,5-diyl ou la pyrimidine-2,5-diyl ;
$Z^{16}$ est $-(CH_2)_2-$, -COO-, $-CF_2O-$, $-OCF_2-$, -C≡C-, $-CH_2O-$ ou une liaison unique ;
$L^3$ et $L^4$ sont chacun de façon indépendante l'hydrogène ou le fluor ; et q est 0, 1 ou 2 et r est 0 ou 1.

9. Composition de cristaux liquides selon la revendication 6, comprenant en outre au moins un composé sélectionné parmi le groupe de composés représentés par les formules (6), (7), (8), (9) et (10), comme composant D :

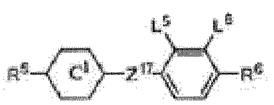 (6)

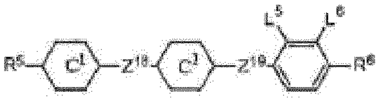 (7)

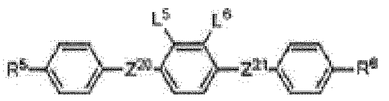

(8)

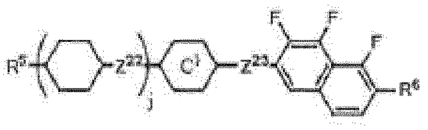

(9)

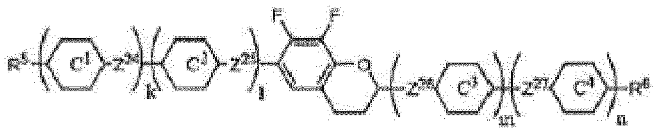

(10)

dans lequel

$R^5$ et $R^6$ sont chacun de façon indépendante un alkyle ayant de 1 à 10 carbones ou un alkényle ayant de 2 à 10 carbones, et dans l'alkyle et l'alkényle, l'hydrogène arbitraire peut être remplacé par le fluor et le $-CH_2-$ arbitraire peut être remplacé par -O- ;

l'anneau Cl, l'anneau $C^2$, l'anneau $C^3$ et l'anneau $C^4$ sont chacun de façon indépendante le 1,4-cyclohexylène, le 1,4-cyclohexenylène, le 1,4-phénylène dans lequel l'hydrogène arbitraire peut être remplacé par le fluor ou la décahydro-2,6-naphtalène ;

$Z^{17}$, $Z^{18}$, $Z^{19}$, $Z^{20}$, $Z^{21}$, $Z^{22}$, $Z^{23}$, $Z^{24}$, $Z^{25}$, $Z^{26}$ et $Z^{27}$ sont chacun de façon indépendante $-(CH_2)_2-$, -COO-, $-CH_2O-$, $-OCF_2-$, $-OCF_2(CH_2)_2-$ ou une liaison unique ;

$L^5$ et $L^6$ sont chacun de façon indépendante le chlore ou le fluor ; et

j, k, l, m et n sont chacun de façon indépendante 0 ou 1, et la somme de k, l, m et n est 1 ou 2.

**10.** Composition de cristaux liquides selon la revendication 6, comprenant en outre au moins un composé sélectionné parmi le groupe de composés représentés par les formules (11), (12) et (13), comme composant E :

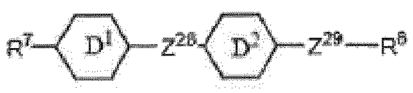

(11)

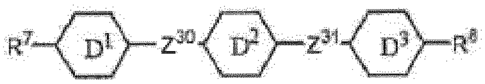

(12)

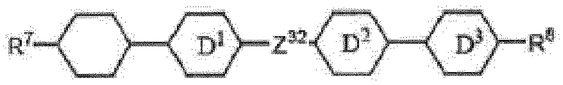

(13)

dans laquelle

$R^7$ et $R^8$ sont chacun de façon indépendante un alkyle ayant de 1 à 10 carbones ou un alkényle ayant de 2 à 10 carbones, et dans l'alkyle et l'alkényle, l'hydrogène arbitraire peut être remplacé par le fluor et le $-CH_2-$ arbitraire peut être remplacé par -O- ;

L'anneau $D^1$, l'anneau $D^2$ et l'anneau $D^3$ sont chacun de façon indépendante le 1,4-cyclohexylène, la pyrimidine-2,5-diyl, le 1,4-phénylène, le 2-fluoro-1,4-phénylène, le 3-fluoro-1,4-phénylène ou le 2,5-difluoro-1,4-phénylène ; et $Z^{28}$, $Z^{29}$, $Z^{30}$, $Z^{31}$ et $Z^{32}$ sont chacun de façon indépendante -C≡C-, -COO-, $-(CH_2)_2-$, -CH=CH- ou une liaison unique.

**11.** Composition de cristaux liquides selon la revendication 7, comprenant en outre au moins un composé sélectionné parmi le groupe de composés représentés par la formule (5) selon la revendication 8.

**12.** Composition de cristaux liquides selon la revendication 7, comprenant en outre au moins un composé sélectionné parmi le groupe de composés représentés par les formules (11), (12) et (13) selon la revendication 10.

**13.** Composition de cristaux liquides selon la revendication 8, comprenant en outre au moins un composé sélectionné parmi le groupe de composés représentés par les formules (11), (12) et (13) selon la revendication 10.

**14.** Composition de cristaux liquides selon la revendication 9, comprenant en outre au moins un composé sélectionné parmi le groupe de composés représentés par les formules (11), (12) et (13) selon la revendication 10.

**15.** Composition de cristaux liquides selon l'une quelconque des revendications 6 à 14, comprenant en outre au moins un composant optiquement actif.

**16.** Composition de cristaux liquides selon l'une quelconque des revendications 6 à 15, comprenant en outre au moins un antioxydant, ou au moins un absorbant des ultraviolets, ou au moins à la fois un antioxydant et un absorbant des ultraviolets.

**17.** Dispositif d'affichage à cristaux liquides comprenant au moins une des compositions de cristaux liquides selon l'une quelconque des revendications 6 à 16.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2811342 B
- JP H02004725 A
- JP 2000008040 A
- EP 0967261 A1
- JP 2006037053 A
- EP 2206695 A1
- EP 2128225 A1


### Non-patent literature cited in the description


- *Mol. Cryst. Liq. Cryst.,* 1970, vol. 12, 57
- *Liquid Crystals: An International Journal of Science and Technology,* 1989, vol. 5, 159-170
- Synthesis and Reaction of Organic Compounds. New Experimental Chemistry Course. Maruzen Co., Ltd, 1978, vol. 14
- Organic Synthesis I to VIII. Experimental Chemistry Course. Maruzen Co., Ltd, 1991, vol. 19-26
- **M. IMAI et al.** *Molecular Crystals and Liquid Crystals,* 1995, vol. 259, 37